(19) Europäisches Patentamt
European Patent Office
Office européen des brevets

(11) EP 1 350 793 A1

(12) **EUROPEAN PATENT APPLICATION**
published in accordance with Art. 158(3) EPC

(43) Date of publication:
**08.10.2003 Bulletin 2003/41**

(21) Application number: **01270221.3**

(22) Date of filing: **11.12.2001**

(51) Int Cl.[7]: **C07D 413/12**, A61K 31/422, A61K 9/10, A61K 9/14, A61K 47/20, A61K 47/26, A61K 47/28, A61K 47/32, A61K 47/38, A61K 47/40, A61P 35/00

(86) International application number:
**PCT/JP01/10827**

(87) International publication number:
**WO 02/048142 (20.06.2002 Gazette 2002/25)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.12.2000 JP 2000375608**

(71) Applicant: **Takeda Chemical Industries, Ltd.**
**Osaka-shi, Osaka 541-0045 (JP)**

(72) Inventor: **NARA, Eiji**
**Kawanishi-shi, Hyogo 666-0145 (JP)**

(74) Representative:
**Rickard, Timothy Mark Adrian et al**
**Takeda Euro IP Department,**
**11-12 Charles II Street**
**London SW1Y 4QU (GB)**

(54) **MEDICINAL COMPOSITIONS HAVING IMPROVED ABSORBABILITY**

(57) An HER2 inhibitor having an average particle size of about 3 µm or less or a composition containing the same which has improved HER2 inhibitor-absorbability.

EP 1 350 793 A1

## Description

TECHNICAL FIELD

The present invention relates to a HER2 inhibitor having an average particle size of about 3 μm or less, which has improved absorbability, a composition comprising the same, and a process for producing them.

BACKGROUND ART

JP 11-60571 A describes a compound having a HER2 inhibitory activity which is represented by the formula:

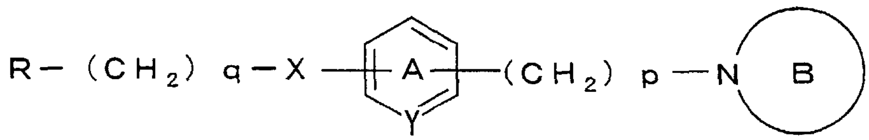

R represents an optionally substituted aromatic heterocyclic group, X represents an oxygen atom, an optionally oxidized sulfur atom, -C(=O)- or -CH(OH)-, Y represents CH or N, p represents an integer of 0 to 10, q represents an integer of 1 to 5, a group represented by the formula:

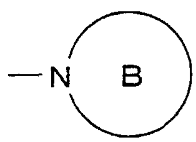

represents an optionally substituted aromatic azole group, and ring A may be further substituted, or a salt thereof.

OBJECTS OF THE INVENTION

A problem to be solved by the present invention is to provide a HER2 inhibitor having improved absorbability of the HER2 inhibitor, a composition comprising the same, and a process for producing the same.

SUMMARY OF THE INVENTION

The present inventor has intensively studied to solve the problem described above and found that, when a slightly slightly water-soluble to water-insoluble HER2 inhibitor is comminuted into particles sized about 3 μm or less, which can not be achieved by a conventional technique such as jet-mill pulverization, etc., in an aqueous solution to form fine particles, the absorbability of the HER2 inhibitor can be remarkably and unexpectedly improved. The present inventor has further studied based on this finding, and thus the present invention has been completed.

Namely, the present invention provides:

(1) A HER2 inhibitor having an average particle size of about 3 μm or less;
(2) A HER2 inhibitor which has an average particle size of about 3 μm or less when dispersed in water or an aqueous solution;
(3) The HER2 inhibitor according to the above (1) or (2), which is in the form of a crystalline particulate;
(4) The HER2 inhibitor according to the above (1) or (2), which is a compound represented by the formula:

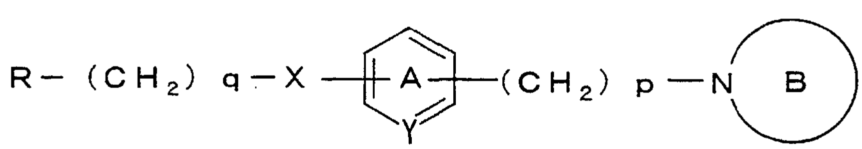

wherein R represents an optionally substituted aromatic heterocyclic group, X represents an oxygen atom, an optionally oxidized sulfur atom, -C(=O)- or -CH(OH)-, Y represents CH or N, p represents an integer of 0 to 10, q represents an integer of 1 to 5, a group represented by the formula:

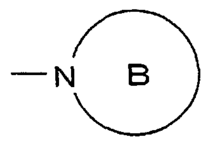

represents an optionally substituted aromatic azole group, and ring A may be further substituted, or a salt thereof or a prodrug thereof;

(5) The HER2 inhibitor according to the above (1) or (2), which is a compound represented by the formula:

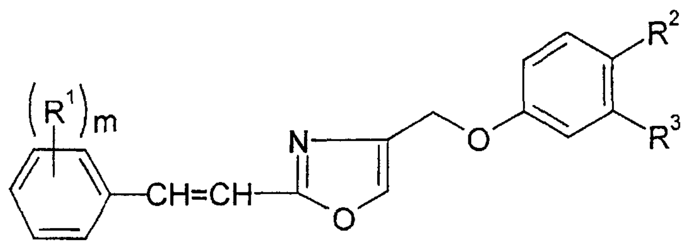

wherein m represents 1 or 2, $R^1$ represents halogen or an optionally halogenated $C_{1-2}$ alkyl, and one of $R^2$ and $R^3$ represents a hydrogen atom and the other represents a group represented by the formula:

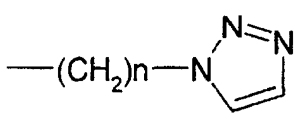

or

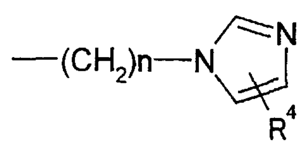

wherein n represents 3 or 4, and $R^4$ represents a $C_{1-4}$ alkyl group substituted with 1 to 2 hydroxy groups, or a salt thereof or a prodrug thereof;

(6) The HER2 inhibitor according to the above (1) or (2), which is (i) 1-(4-{4-[(2-{ (E)-2-[4-(trifluoromethyl)phenyl] ethenyl}-1,3-oxazol-4-yl)methoxy]phenyl}butyl)-1H-1,2,3-triazole, (ii) 1-(3-{3-[(2-{(E)-2-[4-(trifluoromethyl)phenyl] ethenyl}-1,3-oxazol-4-yl)methoxy]phenyl}propyl)-1H-1,2,3-triazole or (iii) 3-(1-{4-[4-({2-[(E)-2-(2,4-difluorophenyl) ethenyl] -1, 3-oxazol-4-yl}methoxy)phenyl]butyl}-1H-imidazol-2-yl)-1,2-propanediol, or a salt thereof or a prodrug thereof;

(7) A composition comprising the HER2 inhibitor of the above (1) or (2);

(8) The composition according to the above (7), which comprises a stabilizer;

(9) The composition according to the above (7), wherein the stabilizer is at least one member selected from (a) a surfactant, (b) a hydrophilic polymer and (c) an easily water-soluble cyclodextrin derivative;

(10) The composition according to the above (7), wherein the stabilizer is at least one member selected from sodium deoxycholate, hydroxypropylcellulose and polyvinyl pyrrolidone;

(11) The composition according to the above (8), which comprises both a surfactant and a hydrophilic polymer as the stabilizer;

(12) The composition according to the above (11), wherein the surfactant is an anionic surfactant or a nonionic surfactant;

(13) The composition according to the above (11), wherein the surfactant is an alkyl sulfate salt or a sucrose fatty acid ester;

(14) The composition according to the above (11), wherein the surfactant is sodium lauryl sulfate or sucrose stearate ester;

(15) The composition according to the above (11), wherein the hydrophilic polymer is hydroxypropylcellulose;

(16) The composition according to the above (11), wherein the surfactant is sodium lauryl sulfate or sucrose fatty

acid ester, and the hydrophilic polymer is hydroxypropylcellulose;
(17) The composition according to the above (7), which is used for oral administration;
(18) The composition according to the above (7), which is an anticancer agent;
(19) The composition according to the above (7), which is an agent for preventing or treating breast cancer or prostatic cancer;
(20) A process for producing the HER2 inhibitor according to the above (1) or (2), which comprises comminuting the HER2 inhibitor in water or an aqueous solution;
(21) A process for producing the composition according to the above (7), which comprises comminuting a HER2 inhibitor in water or an aqueous solution;
(22) A process for producing the composition according to the above (8), which comprises comminuting a HER2 inhibitor in an aqueous solution containing a stabilizer;
(23) The process according to the above (20), (21) or (22), wherein the comminution is carried out by using a compaction shearing mill;
(24) A composition obtainable by comminuting a HER2 inhibitor in an aqueous solution containing a stabilizer and removing a solvent;
(25) A method for preventing or treating cancer, which comprises orally administering a HER2 inhibitor having an average particle size of about 3 µm or less to a mammal; and
(26) Use of a HER2 inhibitor having an average particle size of about 3 µm or less for the manufacture of an agent for oral administration for preventing or treating cancer.

Further, the present invention provides:

(27) The HER2 inhibitor according to the above (4), wherein the cyclic group represented by the formula:

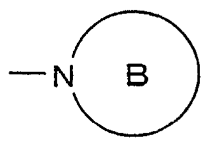

is a pyrrolyl group, an imidazolyl group, a pyrazolyl group, a triazolyl group, a tetrazolyl group or a benzimidazolyl group, each of which may be substituted with one or two substituents selected from (i) an alkyl group, (ii) an aryl group, (iii) a hydroxyalkyl group, (iv) a carboxyl group, (v) an alkoxycarbonyl group and (vi) a carbamoyl group;
(28) The HER2 inhibitor according to the above (4), wherein p is an integer of 3 to 5;
(29) The HER2 inhibitor according to the above (4), wherein q is 1;
(30) The HER2 inhibitor according to the above (4), wherein X is oxygen atom;
(31) The HER2 inhibitor according to the above (4), wherein R is an optionally substituted oxazolyl group or an optionally substituted thiazolyl group;
(32) The HER2 inhibitor according to the above (4), wherein R is an oxazolyl group, a benzoxazolyl group or a thiazolyl group, each of which may be substituted with one or two substituents selected from (i) an aryl group which may be substituted with one or two substituents selected from a hydroxyl group, an alkoxy group, an arylalkoxy group, an alkyl group, a cyano group, a halogen atom and a tetrazolyl group, (ii) an alkyl group, (iii) a hydroxyalkyl group, (iv) an alkoxycarbonylalkyl group, (v) an alkyl group substituted with one or two aryl groups, (vi) an alkenyl group substituted with one or two aryl groups, (vii) a cycloalkyl group, (viii) a partially saturated naphthyl group, (ix) a thienyl or furyl group which may be substituted with one or two substituents selected from a hydroxy group, an alkoxy group, an arylalkoxy group, an alkyl group, a cyano group, an allyl group and a halogen atom, (x) a benzofuranyl group and (xi) a benzothienyl group;
(33) The HER2 inhibitor according to the above (4), wherein R is an oxazolyl group, a benzoxazolyl group or a thiazolyl group, each of which may be substituted with one or two substituents selected from (i) an aryl group which may be substituted with one or two substituents selected from a hydroxyl group, an alkoxy group, an arylalkoxy group, an alkyl group, a cyano group, a halogen atom and a tetrazolyl group, (ii) an alkyl group, (iii) a hydroxyalkyl group, (iv) an alkoxycarbonylalkyl group, (v) an alkyl group substituted with one or two aryl groups, (vi) an alkenyl group substituted with one or two aryl groups, (vii) a cycloalkyl group, (viii) a partially saturated naphthyl group, (ix) a thienyl or furyl group which may be substituted with one or two substituents selected from a hydroxy group, an alkoxy group, an arylalkoxy group, an alkyl group, a cyano group, an allyl group and a halogen atom, (x) a benzofuranyl group and (xi) a benzothienyl group,

X is an oxygen atom,
p is an integer of 0 to 6,
q is 1,

the cyclic group represented by the formula:

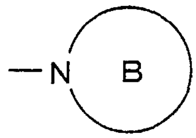

is a pyrrolyl group, an imidazolyl group, a pyrazolyl group, a triazolyl group, a tetrazolyl group or a benzimidazolyl group, each of which may be substituted with one or two substituents selected from (i) an alkyl group, (ii) an aryl group; (iii) a hydroxyalkyl group, (iv) a carboxyl group, (v) an alkoxycarbonyl group and (vi) a carbamoyl group;
(34) The HER2 inhibitor according to the above (4), wherein R is an oxazolyl group which is substituted with an arylalkenyl or arylalkoxy-aryl group,

X is an oxygen atom,
p is 3 or 4,
q is 1,

the cyclic group represented by the formula:

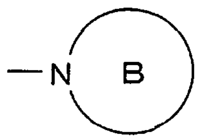

is an imidazolyl group or a triazolyl group, and
the group represented by the formula:

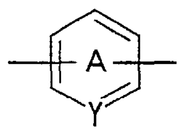

is a 1,3-phenylene group or a 1,4-phenylene group;
(35) The HER2 inhibitor according to the above (4), wherein R is an oxazolyl or thiazolyl group substituted with a thienyl group,

X is an oxygen atom,
p is 3 or 4,
q is 1,

the cyclic group represented by the formula:

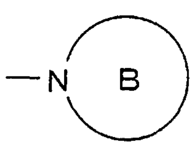

is an imidazolyl group or a triazolyl group, and

the group represented by the formula:

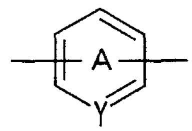

is a 1,3-phenylene group or a 1,4-phenylene group;

(36) The HER2 inhibitor according to the above (4), wherein R is a benzoxazolyl group substituted with a thienyl group,

X is an oxygen atom,
p is 3 or 4,
q is 1,

the cyclic group represented by the formula:

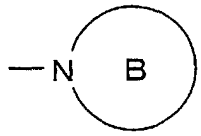

is an imidazolyl group or a triazolyl group, and

the group represented by the formula:

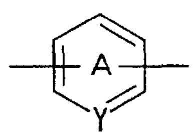

is a 1,3-phenylene group or a 1,4-phenylene group;

(37) The HER2 inhibitor according to the above (1) or (2), wherein HER2 inhibitor is (i) 1-[ 4-[4-[2-[ (E)-2-phenylethenyl]-4-oxazolylmethoxy]phenyl]butyl]-1,2,4-triazole, (ii) 4-[4-[4-(1-imidazolyl)butyl]phenoxymethyl]-2-[(E)-2-phenylethenyl]oxazole, (iii) 4-[4-[3-(1-imidazolyl)propyl]phenoxymethyl]-2-[(E)-2-phenylethenyl]oxazole, (iv) 4-[3-[3-(1-imidazolyl)propyl]phenoxymethyl]-2-[(E)-2-phenylethenyl]oxazole, (v) 2-(4-benzyloxyphenyl)-4-[4-[4-[3-(1-imidazolyl)propyl]phenoxymethyl]oxazole, (vi) 4-[4-[3-(1-imidazolyl)propyl]phenoxymethyl]-2-(2-thienyl)oxazole, (vii) 4-[4-[3-(1-imidazolyl)propyl]phenoxymethyl]-2-(5-methyl-2-thienyl)oxazole, (viii) 2-(5-chloro-2-thienyl)-4-[4-[3-(1-imidazolyl)-propyl]phenoxymethyl]oxazole, (ix) 4-[4-[3-(1-imidazolyl)propyl]phenoxymethyl]-2-(2-thienyl)thiazole, or (x) 5-[4-[3-(1-imidazolyl)propyl]phenoxymethyl]-2-(2-thienyl)benzoxazole, or a salt thereof or a prodrug thereof;

(38) The HER2 inhibitor according to the above (5), wherein $R^1$ is fluoro or trifluoromethyl;

(39) The HER2 inhibitor according to the above (5), wherein $R^2$ is a group represented by the formula:

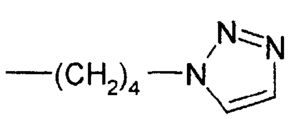

and $R^3$ is a hydrogen atom, or

$R^2$ is a hydrogen atom and $R^3$ is a group represented by the formula:

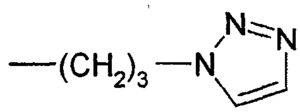

;

(40) The HER2 inhibitor according to the above (5), wherein $R^2$ is a group represented by the formula:

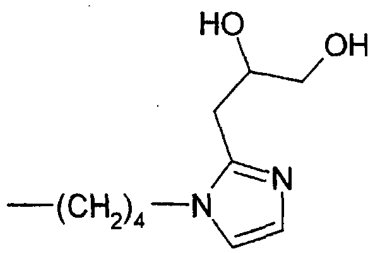

and $R^3$ is a hydrogen atom;

(41) The HER2 inhibitor according to the above (5), wherein m is 1,

$R^1$ is 4-trifluoromethyl,
$R^2$ is a group represented by the formula:

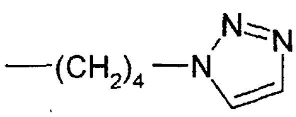

,

and
$R^3$ is a hydrogen atom; and

(42) The composition according to the above (7), which is an injectable preparation.

BRIEF DESCRIPTION OF THE DRAWINGS

Fig. 1 is a graph showing a change in a plasma level of a drug after the administration of a crystalline particulate of the drug prepared in Example 1a, in which the abscissa denotes time lapsed after the administration of the drug and the ordinate denotes the plasma level of the drug.
Fig. 2 is a graph showing a change in a plasma level of a drug after the administration of a crystalline particulate of the drugs prepared in Examples 4a and 8a, in which the abscissa denotes time lapsed after the administration of the drug and the ordinate denotes the plasma level of the drug.

EXPLANATION OF PREFERRED EMBODIMENTS OF THE INVENTION

As used herein, the term "HER2" refers to a "growth factor receptor tyrosine kinase".
As used herein, the term "HER2 inhibitor" is not specifically limited as long as it is a substance capable of inhibiting a function of HER2. Examples thereof include a substance capable of inhibiting the binding of HER2 with a ligand and a substance capable of inhibiting the production of HER2 (for example, DNA synthesis inhibitor, RNA synthesis inhibitor, mRNA transfer inhibitor or the like). As used herein, the term "substance" includes, for example, synthetic compounds, fermentation products, peptides, proteins, cell and tissue extracts, etc.
Although the HER2 inhibitor of the present invention may be soluble, slightly soluble or insoluble in water, the present invention is particularly suited for a slightly water-soluble or water-insoluble substance.
The term "slightly water-soluble or water-insoluble" means a solubility of less than 10,000 ppm, and preferably

less than 100 ppm in water at 25°C, or a solubility in water at 25°C is less than 10 mg/mL, and preferably less than 0.1 mg/mL. The solubility can be measured according to a conventional method.

The average particle size of the HER2 inhibitor of the present invention is about 3 μm or less and, more specifically, the average particle size is about 3 μm or less when dispersed in an aqueous solvent (for example, water, an aqueous methyl cellulose solution or the like). The average particle size of the HER2 inhibitor of the present invention is preferably about 2 μm or less, more preferably about 1 μm or less, and particularly preferably about 0.5 μm or less. The lower limit of the average particle size is not specifically limited, but is usually about 0.05 μm or more, preferably 0.1 μm or more, more preferably about 0.2 μm or more, and particularly preferably 0.4 μm or more. The average particle size is preferably from about 0.2 to 1.0 μm, and more preferably from 0.4 to 1 μm.

The average particle size can be measured by using a per se known method and a measuring equipment. The term "average particle size" refers to a volume-average particle size as measured, for example, by a laser diffraction/scattering particle size distribution measuring apparatus (for example, SALD-2000A manufactured by Shimadzu Corporation). The particle size is usually measured by uniformly dispersing a HER2 inhibitor suspension at a concentration of about 1 to 100 μg/ml at room temperature while being subjected to ultra-sonication for a short period of time such as within 1 minute, or while stirring using a vortex mixer. The aqueous solvent used in the measurement is usually water, but buffer solutions having various pH' s, a pseudo-body fluid and 0.5% methyl cellulose solution may be used.

In the HER2 inhibitor having an average particle size of about 3 μm or less of the present invention, for example, water solubility and absorbability (for example, oral absorbability, absorbability into blood) of the HER2 inhibitor are improved.

When the HER2 inhibitor having an average particle size of about 3 μm or less of the present invention is analyzed by a powder X-ray diffraction apparatus, a clear crystal peak appears and, therefore, it is considered that the HER2 inhibitor mainly exists in a crystalline state. Thus, it is assumed that the solubility in water does not change drastically.

Specifically, the wording "water solubility is improved" means that "dissolution rate is accelerated", and that the dissolution rate at 37°C is accelerated by 2 times, preferably 5 times, more preferably 10 times, and further preferably 100 or more times. The dissolution rate can be measured by a per se known method, but can also be measured in a simple manner by a dissolution test method. It is considered that "improvement in water solubility" is mainly caused by an increase in surface area of particles as a result of comminution into fine particles. In case that the dispersion state is stabilized by a polymer or a surfactant, it is considered that enhancement in wettability of drug particles by water also contributes to the improvement in water solubility.

Specifically, the wording "oral absorbability is improved" means that, in comparison with a HER2 inhibitor having a particle size of about 3 μm or more, at least either of "increase in absorption rate" and "improvement in absorption ratio and bioavailability" is achieved. In the preparation in the form of a crystalline particulate of the present invention, unexpectedly, the absorbability of the HER2 inhibitor is improved. Although its mechanism is not elucidated and the present inventor would not like to be restricted to the following theoretical consideration, for example, it can also be considered that the dissolution rate of the HER2 inhibitor increases and the amount of the drug dissolved increases until the HER2 inhibitor passes through the absorption portion of the alimentary canal, thereby improving the absorbability. It is assumed that an improvement in adhesion and retention in the alimentary canal due to comminution into fine particles, an improvement in transfer of Peyer's patches to the lymph duct via M cells, etc., also contribute to the improvement in absorbability. The index of "acceleration of drug absorption rate" includes $T_{max}$ (time required to reach maximum blood level), MRT (mean retention time), etc., while the index of "improvement in absorption ratio, bioavailability" includes AUC (area under blood level versus time curve), $C_{max}$ (time requited to reach maximum blood level), etc. Specifically, it means that the absorbability is improved by about 2 times, preferably about 5 times, more preferably 10 times, and further preferably 100 times, 1,000 times, 10,000 times, 100,000 time, 1000,000 times or more, compared with a HER2 inhibitor having a particle size of about 3 μm or more.

Specifically, the HER2 inhibitor used in the present invention is a slightly water-soluble or water-insoluble compound (I) represented by the formula:

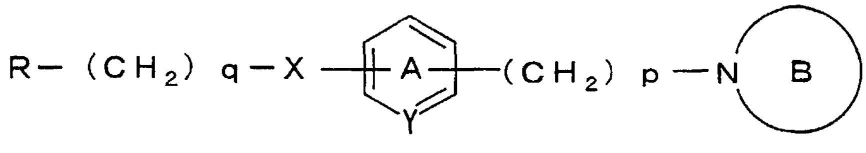

wherein R represents an optionally substituted aromatic heterocyclic group, X represents an oxygen atom, an optionally oxidized sulfur atom, -C(=O)- or -CH(OH)-, Y represents CH or N, p represents an integer of 0 to 10, q represents an

integer of 1 to 5, a group represented by the formula:

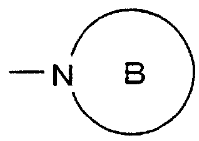

represents an optionally substituted aromatic azole group, and ring A may be further substituted, or a salt thereof or a prodrug thereof.

In the above compound (I), examples of the heterocyclic group in the optionally substituted aromatic heterocyclic group represented by R include (1) a 5- or 6-membered aromatic monocyclic heterocyclic group having as the ring-constituent atoms, in addition to carbon atoms, 1 to 4 atoms selected from a nitrogen atom, an oxygen atom and a sulfur atom, and (2) an aromatic condensed heterocyclic group formed by condensation of (i) a 5- or 6-membered aromatic monocyclic heterocyclic group having, as the ring-constituent atoms, in addition to carbon atoms, 1 to 4 atoms selected from a nitrogen atom, an oxygen atom and a sulfur atom with (ii) a 5- or 6-membered aromatic or non-aromatic heterocyclic group having, as the ring-constituent atoms, in addition to carbon atoms, 1 to 2 nitrogen atoms, a benzene ring, or a 5-membered aromatic or non-aromatic heterocyclic group having, as the ring-constituent atoms, in addition to carbon atoms, one sulfur atom, and the like.

Specific examples of these aromatic heterocyclic groups include pyridyl (for example, 2-pyridyl, 3-pyridyl, 4-pyridyl), pyrimidinyl (2-pyrimidinyl, 5-pyrimidinyl, 6-pyrimidinyl), pyridazinyl (for example, 3-pyridazinyl, 4-pyridazinyl), pyrazinyl (for example, 2-pyrazinyl), pyrrolyl (for example, 1-pyrrolyl, 2-pyrrolyl), imidazolyl (for example, 1-imidazolyl, 2-imidazolyl, 4-imidazolyl, 5-imidazolyl), pyrazolyl (for example, 1-pyrazolyl, 3-pyrazolyl, 4-pyrazolyl), isoxazolyl, isothiazolyl, thiazolyl (for example, 2-thiazolyl, 4-thiazolyl, 5-thiazolyl), oxazolyl (for example, 2-oxazolyl, 4-oxazolyl, 5-oxazolyl), oxadiazolyl (for example, 1,2,4-oxadiazolyl such as 1,2,4-oxadiazol-5-yl, etc., 1,2,3-oxadiazolyl, 1,3,4-oxadiazolyl), thiadiazolyl (for example, 1,2,3-thiadiazolyl, 1,2,4-thiadiazolyl, 1,3,4-thiadiazolyl), triazolyl (for example, 1,2,4-triazolyl such as 1,2,4-triazol-1-yl, 1,2,4-triazol-5-yl, etc., 1,2,3-triazolyl such as 1,2,3-triazol-1-yl, 1,2,3-triazol-2-yl, 1,2,3-triazol-4-yl, etc.), tetrazolyl (for example, tetrazol-1-yl, tetrazol-5-yl), benzimidazolyl (for example, benzimidazol-1-yl, benzimidazol-2-yl), indolyl (for example, indol-1-yl, indol-3-yl), indazolyl (for example, 1H-indazol-1-yl, 1H-indazol-3-yl), pyrrolopyrazinyl (for example, 1H-pyrrolo[2,3-b]pyrazinyl), pyrrolopyridyl (for example, 1H-pyrrolo[2,3-b]pyridyl), imidazopyridyl (for example, 1H-imidazo[4,5-b]pyridyl, 1H-imidazo[4,5-c]pyridyl), imidazopyrazinyl (for example, 1H-imidazo[4,5-b]pyrazinyl), pyrrolopyridazinyl (for example, pyrrolo[1,2-b]pyridazinyl), pyrazolopyridyl (for example, pyrazolo[1,5-a]pyridyl), imidazopyridyl (for example, imidazo[1,2-a]pyridyl, imidazo[1,5-a]pyridyl), imidazopyridazinyl (for example, imidazo[1,2-b]pyridazinyl), imidazopyrimidinyl (for example, imidazo[1,2-a]pyrimidinyl), furyl, thienyl, benzofuranyl, benzothienyl (for example, benzo[b]thienyl), benzoxazolyl, benzothiazolyl, quinolyl, isoquinolyl, quinazolinyl, and the like. Preferred examples include a 5-membered cyclic aromatic azole group such as oxazolyl, thiazolyl, isoxazolyl, isothiazolyl, imidazolyl, triazolyl, oxadiazolyl, thiadiazolyl, etc., an aromatic condensed azole group formed by condensation with a benzene ring such as benzoxazolyl, benzothiazolyl, etc., and a 6-membered monocyclic aromatic heterocyclic group such as pyridyl, pyrimidyl, etc. Further preferred examples of the aromatic heterocyclic group include a 5-membered monocyclic aromatic azole group such as oxazolyl group, thiazolyl group, etc.

The aromatic heterocyclic group represented by R and the aromatic azole group represented by the formula:

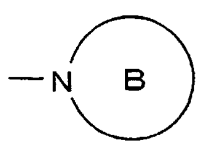

include, for example, (1) a 5-membered aromatic monocyclic heterocyclic group having, as the ring-constituent atoms, in addition to carbon atoms, 1 to 4 nitrogen atoms and optionally having one oxygen atom or one sulfur atom, and (2) an aromatic condensed heterocyclic group formed by condensation of (i) a 5-membered aromatic monocyclic heterocyclic group having, as the ring-constituent atoms, in addition to carbon atoms, 1 to 4 nitrogen atoms and optionally having one nitrogen atom or one sulfur atom, with (ii) a 5- or 6-membered aromatic or non-aromatic heterocyclic group having, as the ring-constituent atoms, in addition to carbon atoms, one or two nitrogen atoms, a benzene ring or a 5-membered aromatic or non-aromatic heterocyclic group containing, as the ring-constituent atoms, in addition to carbon atoms, one sulfur atom, and the like.

Specific examples of the aromatic azole group include aromatic heterocyclic groups such as pyrrolyl (for example, 1-pyrrolyl), imidazolyl (for example, 1-imidazolyl), pyrazolyl (for example, 1-pyrazolyl), triazolyl (for example, 1,2,4-triazol-1-yl, 1,2,3-triazol-1-yl), tetrazolyl (for example, tetrazol-1-yl), benzimidazolyl (for example, benzimidazol-1-yl), indolyl (for example, indol-1-yl), indazolyl (for example, 1H-indazol-1-yl), pyrrolopyrazinyl (for example, 1H-pyrrolo[2,3-b]pyrazin-1-yl), pyrrolopyridyl (for example, 1H-pyrrolo[2,3-b]pyridin-1-yl), imidazopyridyl (for example, 1H-imidazo[4,5-b]pyridin-1-yl), imidazopyrazinyl (for example, 1H-imidazo[4,5-b]pyrazin-1-yl), and the like. These groups are bonded to $-(CH_2)_m-$ through the nitrogen atom contained as one of the ring-constituent atoms. Preferred examples of the aromatic azole group include imidazolyl group and triazolyl group.

The aromatic heterocyclic group represented by R and the aromatic azole group represented by the formula:

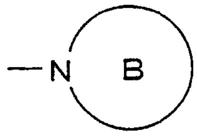

may have 1 to 3 (preferably one or two) substituents at any substitutable positions. Examples of the substituent include an aliphatic hydrocarbon group, an alicyclic hydrocarbon group, an aromatic hydrocarbon group, an aliphatic hydrocarbon group substituted with an aromatic hydrocarbon group, an aliphatic hydrocarbon group substituted with an alicyclic hydrocarbon group, an aromatic heterocyclic group, a non-aromatic heterocyclic group, an aliphatic hydrocarbon group substituted with an aromatic heterocyclic group, a halogen atom, a nitro group, a cyano group, an optionally substituted amino group, an optionally substituted acyl group, an optionally substituted hydroxyl group, an optionally substituted thiol group, and an optionally esterified or amidated carboxyl group. The aliphatic hydrocarbon group, alicyclic hydrocarbon group, aromatic hydrocarbon group, aliphatic hydrocarbon group substituted with an aromatic hydrocarbon group, aliphatic hydrocarbon group substituted with an aliphatic hydrocarbon group, aromatic heterocyclic group, non-aromatic heterocyclic group and aliphatic hydrocarbon group substituted with an aromatic heterocyclic group as the substituent may further be substituted, respectively.

The ring A may further have, in addition to X and $(CH_2)_p$, 1 to 4 (preferably 1 or 2) substituents at any substitutable positions. The substituents include those exemplified as the substituent of the substituent which the aromatic heterocyclic group represented by R may have, for example, an aliphatic hydrocarbon group, an alicyclic hydrocarbon group, an aromatic hydrocarbon group, an aliphatic hydrocarbon group substituted with an aromatic hydrocarbon group, an aliphatic hydrocarbon group substituted with an alicyclic hydrocarbon group, an aromatic heterocyclic group, an aliphatic hydrocarbon group substituted with an aromatic heterocyclic group, a halogen atom, a nitro group, a cyano group, an optionally substituted amino group, an optionally substituted acyl group, an optionally substituted hydroxyl group, an optionally substituted thiol group, an optionally esterified or amidated carboxyl group, and the like. The aliphatic hydrocarbon group, alicyclic hydrocarbon group, aromatic hydrocarbon group, aliphatic hydrocarbon group substituted with an aromatic hydrocarbon group, aliphatic hydrocarbon group substituted with an alicyclic hydrocarbon group, aromatic heterocyclic group, non-aromatic heterocyclic group and aliphatic hydrocarbon group substituted with an aromatic heterocyclic group mentioned above as the substituent may be further substituted, respectively.

Examples of the aliphatic hydrocarbon group include a straight or branched chain aliphatic hydrocarbon group having 1 to 15 carbon atoms, for example, an alkyl group, an alkenyl group, an alkynyl group, and the like.

Preferred examples of the alkyl group include a $C_{1-10}$ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl, decyl, 1,1-dimethylbutyl, 2,2-dimethylbutyl, 3,3-dimethylbutyl, 2-ethylbutyl, etc., more preferably a $C_{1-6}$ alkyl group.

Preferred examples of the alkenyl group include a $C_{2-10}$ alkenyl group such as vinyl (ethenyl), allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc., more preferably a $C_{2-6}$ alkenyl group.

Preferred examples of the alkynyl group include a $C_{2-10}$ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc., more preferably a $C_{2-6}$ alkynyl group.

Examples of the alicyclic hydrocarbon group include a $C_{3-12}$ saturated or unsaturated alicyclic hydrocarbon group such as a cycloalkyl group, a cycloalkenyl group, a cycloalkadienyl group, a partially unsaturated condensed dicyclic hydrocarbon group, and the like.

Preferred examples of the cycloalkyl group include a $C_{3-10}$ cycloalkyl group such as cyclopropyl, cyclobutyl, cyclopentyl, cyclohexyl, cycloheptyl, cyclooctyl, etc.; and a $C_{6-10}$ bicycloalkyl group such as bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, etc.

Preferred examples of the cycloalkenyl group include a $C_{5-10}$ cycloalkenyl group such as 2-cyclopenten-1-yl, 3-cyclopenten-1-yl, 2-cyclohexen-1-yl, 3-cyclohexen-1-yl, etc.

Preferred examples of the cycloalkadienyl group include a $C_{5-10}$ cycloalkadienyl group such as 2,4-cyclopentadien-1-yl, 2, 4-cyclohexadien-1-yl, 2,5-cyclohexadien-1-yl, etc.

Preferred examples of the partially unsaturated condensed dicyclic hydrocarbon group include a $C_{9-12}$ group formed by condensation of a benzene ring with an alicyclic hydrocarbon such as indanyl group or partially unsaturated naphthyl group (for example, dihydronaphthyl group such as 3,4-dihydro-2-naphthyl, etc.; tetrahydronaphthyl such as 1,2,3,4-tetrahydronaphthyl, etc.; and the like), and the like.

As the aromatic hydrocarbon group, there is, for example, a monocyclic or condensed polycyclic aromatic hydrocarbon group, and preferred examples thereof include a $C_{6-14}$ aryl group such as phenyl, naphthyl, anthryl, phenanthryl, acenaphthylenyl, 9-fluorenone-2-yl, etc. Among them, a monocyclic or condensed dicyclic aromatic hydrocarbon group such as phenyl, 1-naphthyl, 2-naphthyl, etc., is preferable.

The aliphatic hydrocarbon group substituted with an aromatic hydrocarbon group includes, for example, an aliphatic hydrocarbon group substituted with 1 to 3 (preferably 1 or 2) $C_{7-20}$ aromatic hydrocarbon groups. Preferred examples of such an aliphatic hydrocarbon group substituted with an aromatic hydrocarbon group include a $C_{1-6}$ alkyl group substituted with 1 to 3 $C_{6-14}$ aryl groups (for example, a $C_{1-6}$ alkyl group substituted with 1 to 3 phenyl groups such as benzyl, 2-phenylethyl, 1,2-diphenylethyl, 2,2-diphenylethyl, etc., a $C_{1-6}$ alkyl group substituted with 1 to 3 naphthyl groups, a 9-fluorenyl-$C_{1-6}$ alkyl group, etc.) and a $C_{2-6}$ alkenyl group substituted with 1 to 3 $C_{6-14}$ aryl groups (for example, a $C_{2-6}$ alkenyl group substituted 1 to 3 phenyl groups, such as (E)-2-phenylethenyl, (Z)-2-phenylethenyl, 2,2-diphenylethenyl, 2-(2-naphthyl)ethenyl, 4-phenyl-1,3-butadienyl, etc., a $C_{2-6}$ alkenyl group substituted with 1 to 3 naphthyl groups, or 9-fluorenylidenealkyl group) and the like.

As the aliphatic hydrocarbon group substituted with an alicyclic hydrocarbon group, there is, for example, the above-mentioned aliphatic hydrocarbon group substituted with 1 to 3 (preferably 1 or 2) the above-mentioned alicyclic hydrocarbon groups. Preferred examples of such aliphatic hydrocarbon group substituted with an alicyclic hydrocarbon group include a $C_{1-6}$ alkyl group substituted with 1 to 3 $C_{3-10}$ cycloalkyl groups such as cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl, etc.; a $C_{2-6}$ alkenyl group substituted with 1 to 3 $C_{3-10}$ cycloalkyl groups; a $C_{1-6}$ alkyl groups substituted with 1 to 3 $C_{5-10}$ cycloalkenyl groups; $C_{2-6}$ alkenyl groups substituted with 1 to 3 $C_{5-10}$ cycloalkenyl groups; and the like.

Preferred examples of the aromatic heterocyclic group include a 5- or 6-membered aromatic monocyclic heterocyclic group containing, as ring-constituent atoms, in addition to carbon atoms, 1 to 4 atoms selected from a nitrogen atom, an oxygen atom and a sulfur atom, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.; an aromatic condensed heterocyclic group formed by condensation of (i) a 5- or 6-membered aromatic heterocyclic group containing, as ring-constituent atoms, in addition to carbon atoms, 1 to 4 atoms selected from a nitrogen atom, an oxygen atom and a sulfur atom with (ii) a 5- or 6-membered aromatic or a non-aromatic heterocyclic group containing, as ring-constituent atoms, in addition to carbon atoms, 1 or 2 nitrogen atoms, a benzene ring, or a 5-membered aromatic or a non-aromatic heterocyclic group containing, as ring-constituent atoms, in addition to carbon atoms, one sulfur atom, such as benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, $\alpha$-carbolinyl, $\beta$-carbolinyl, y-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo [1,2-b]pyridazinyl, imidazo[1,2-a]pyrimidinyl, 1,2,4-triazolo[4,3-a]pyridyl, 1,2,4-triazolo[4,3-b]pyridazinyl, etc.; and the like.

Preferred examples of the non-aromatic heterocyclic group include a 3- to 7-membered non-aromatic heterocyclic group containing, as ring-constituent atoms, in addition to carbon atoms, 1 or 2 atoms selected from a nitrogen atom, an oxygen atom and a sulfur atom, such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, etc.

As the aliphatic hydrocarbon group substituted with an aromatic heterocyclic group, there is, for example, a $C_{1-6}$ aliphatic hydrocarbon group substituted with 1 to 3 (preferably 1 or 2) the above-mentioned aromatic heterocyclic groups (for example, a $C_{1-6}$ alkyl group, a $C_{2-6}$ alkenyl group, etc.). Preferred examples of the aliphatic hydrocarbon group substituted with an aromatic heterocyclic group include a $C_{1-6}$ alkyl group substituted with, for example, 1 to 3 groups such as furyl group, thienyl group, imidazolyl group or pyridyl group (for example, (2-furyl)methyl, thienylmethyl, 2-(1-imidazolyl)ethyl, etc.), a $C_{2-6}$ alkenyl group substituted with 1 to 3 groups such as furyl group, thienyl group, imidazolyl group or pyridyl group (for example, 2-(2-furyl)ethenyl, 2-thienylethenyl, etc.), and the like.

As the halogen atom, there are, for example, fluorine, chlorine, bromine and iodine, especially fluorine and chlorine being preferable.

Examples of the optionally substituted amino group include an amino group optionally mono- or di-substituted with, for example, a $C_{1-10}$ alkyl group, a $C_{3-10}$ cycloalkyl, a $C_{2-10}$ alkenyl group, a $C_{5-10}$ cycloalkenyl group, a $C_{1-10}$ acyl group or a $C_{6-12}$ aromatic hydrocarbon group (for example, methylamino, dimethylamino, ethylamino, diethylamino, dibutylamino, diallylamino, cyclohexylamino, acetylamino, propionylamino, benzoylamino, phenylamino, N-methyl-N-phenylamino, etc.) and a 4- to 6-membered cyclic amino group (for example, 1-azetidinyl, 1-pyrrolidinyl, piperidino, morpholino, 1-piperazinyl, etc.).

The 4- to 6-membered cyclic amino group may be further substituted with (1) a $C_{1-6}$ alkyl group, (2) a $C_{6-14}$ aryl group optionally substituted with halogen, a $C_{1-6}$ alkoxy group or trifluoromethyl (for example, phenyl, naphthyl, etc.), (3) a 5- or 6-membered heterocyclic group containing, as ring-constituent atoms, in addition to carbon atoms, 1 to 2 nitrogen atoms (for example, 2-pyridyl and pyrimidinyl) or (4) a 6-membered cyclic amino group (for example, piperidino, 1-piperazinyl, etc.), and the like.

The acyl group of optionally substituted acyl group includes, for example, a $C_{1-13}$ acyl group, more specifically, in addition to formyl, those formed by linkage of, for example, a $C_{1-6}$ alkyl group, a $C_{3-10}$ cycloalkyl group, a $C_{2-6}$ alkenyl group, a $C_{5-10}$ cycloalkenyl group, a $C_{6-12}$ aromatic hydrocarbon group (for example, phenyl, naphthyl, etc.) or a aromatic heterocyclic ring (for example, pyridyl) with carbonyl group, for example, a $C_{2-7}$ alkanoyl group (for example, acetyl, propionyl, butyryl, isobutyryl, valeryl, isovaleryl, pivaloyl, hexanoyl, heptanoyl, octanoyl, etc.), a $C_{3-10}$ cycloalkyl-carbonyl group (for example, cyclobutanecarbonyl, cyclopentanecarbonyl, cyclohexanecarbonyl, cycloheptanecarbonyl, etc.), a $C_{3-7}$ alkenoyl group (for example, crotonoyl group, etc.), a $C_{5-10}$ cycloalkenyl-carbonyl group (for example, 2-cyclohexenecarbonyl, etc.), benzoyl group, nicotinoyl group, and the like.

The substituent in the optionally substituted acyl group includes, for example, a $C_{1-3}$ alkyl group, a $C_{1-3}$ alkoxy groups, halogen (for example, chlorine, fluorine and bromine), a nitro group, a hydroxyl group, amino group, and the like. The number of substituents is, for example, 1 to 3.

Examples of the optionally substituted hydroxyl group include a hydroxyl group, an alkoxy group, a cycloalkyloxy group, an alkenyloxy group, a cycloalkenyloxy group, an aralkyloxy group, an aryloxy group, an acyloxy group, and the like.

Preferred examples of the alkoxy group include a $C_{1-10}$ alkoxy group such as methoxy, ethoxy, propoxy, isopropoxy, butoxy, isobutoxy, sec-butoxy, tert-butoxy, pentyloxy, isopentyloxy, neopentyl, hexyloxy, heptyloxy, nonyloxy, and the like.

Preferred examples of the cycloalkyloxy group include a $C_{3-10}$ cycloalkyloxy group such as cyclobutoxy, cyclopentyloxy, cyclohexyloxy, and the like.

Preferred examples of the alkenyloxy group include a $C_{2-10}$ alkenyloxy group such as allyloxy, crotyloxy, 2-pentenyloxy, 3-hexenyloxy, and the like.

Preferred examples of the cycloalkenyloxy group include a $C_{5-10}$ cycloalkenyloxy group such as 2-cyclopentenyloxy, 2-cyclohexenyloxy, and the like.

Preferred examples of the aralkyloxy group include a $C_{7-20}$ aralkyloxy group such as a $C_{6-14}$ aryl-$C_{1-6}$ alkoxy group, more specifically, a phenyl-$C_{1-6}$ alkoxy group (for example, benzyloxy, phenethyloxy, etc.), a naphthyl-$C_{1-6}$ alkoxy group, and the like.

Preferred examples of the aryloxy group include a $C_{6-14}$ aryloxy group optionally substituted with a $C_{1-3}$ alkyl group, a $C_{1-3}$ alkoxy group, halogen, a nitro group, a hydroxyl group or an amino group, for example, phenoxy, 4-chlorophenoxy, and the like.

Preferred examples of the acyloxy group include a $C_{2-15}$ acyloxy group such as a $C_{2-7}$ alkanoyloxy group (for example, acetyloxy, propionyloxy, butyryloxy, isobutyryloxy, etc.), $C_{6-14}$ aryl-carbonyloxy (for example, benzoyloxy and naphthoyloxy, etc.), and the like.

Examples of the optionally substituted thiol group include a mercapto group, an alkylthio group, a cycloalkylthio group, an alkenylthio group, an aralkylthio group, an arylthio group, a heteroarylthio group, a heteroarylalkylthio group, an acylthio group, and the like.

Preferred examples of the alkylthio group include a $C_{1-10}$ alkylthio group such as methylthio, ethylthio, propylthio, isopropylthio, butylthio, isobutylthio, sec-butylthio, tert-butylthio, pentylthio, isopentylthio, neopentylthio, hexylthio, heptylthio, nonylthio, and the like.

Preferred examples of the cycloalkylthio group include a $C_{3-10}$ cycloalkylthio group such as cyclobutylthio, cyclopentylthio, cyclohexylthio, and the like.

Preferred examples of the alkenylthio group include a $C_{2-10}$ alkenylthio group such as allylthio, crotylthio, 2-pentenylthio, 3-hexenylthio, and the like.

Preferred examples of the aralkylthio group include a $C_{7-20}$ aralkylthio group such as a $C_{6-14}$ arylthio group, more specifically, phenyl-$C_{1-6}$ alkylthio (for example, benzylthio, phenethylthio, etc.), naphthyl-$C_{1-6}$ alkylthio, and the like.

Preferred examples of the arylthio group include a $C_{6-14}$ arylthio group optionally substituted with a $C_{1-3}$ alkyl group, a $C_{1-3}$ alkoxy group, halogen, a nitro group, a hydroxyl group or an amino group, such as phenylthio, naphthylthio, 4-chlorophenylthio, and the like.

The heteroarylthio group includes, for example, a mercapto group substituted with an aromatic heterocyclic group as mentioned above, especially preferred one being pyridylthio (for example, 2-pyridylthio, 3-pyridylthio, etc.), imidazolylthio (for example, 2-imidazolylthio, etc.), triazoylthio (for example, 1,2,4-triazol-5-ylthio, etc.), and the like.

The heteroarylalkylthio group includes, for example, the above-mentioned alkyl thio group substituted with the above-mentioned aromatic heterocyclic group. Preferred examples of the heteroarylthio group include a pyridyl-$C_{1-6}$ alkylthio group (for example, 2-pyridylmethylthio, 3-pyridylmethylthio, etc.).

Preferable examples of the acylthio group include a $C_{2-15}$ acylthio group such as a $C_{2-7}$ alkanoylthio group (for example, acetylthio, propionylthio, butyrylthio isobutyrylthio, etc.), $C_{6-14}$ aryl-carbonylthio (for example, benzoylthio, naphthoylthio, etc.), and the like.

The optionally esterified or amidated carboxyl groups includes, for example, a carboxyl group, an esterified carboxyl group and an amidated carboxyl group.

Examples of the esterified carboxyl group include an alkoxy carbonyl group, an aralkyloxy carbonyl group, an aryloxycarbonyl group, a heteroarylalkyloxycarbonyl group, and the like.

Preferred examples of the alkoxycarbonyl group include a $C_{2-7}$ alkoxycarbonyl group such as methoxycarbonyl, ethoxycarbonyl, propoxycarbonyl, butoxycarbonyl, and the like.

Preferred examples of the aralkyloxycarbonyl group include a $C_{8-21}$ aralkyloxycarbonyl group such as phenyl-$C_{2-7}$ alkoxycarbonyl (for example, benzyloxycarbonyl, etc.), naphthyl-$C_{2-7}$ alkoxycarbonyl, and the like.

Preferable examples of the aryloxycarbonyl group include a $C_{7-15}$ aryloxycarbonyl group optionally substituted with a $C_{1-3}$ alkyl group, a $C_{1-3}$ alkoxy group, halogen, a nitro group, a hydroxyl group or an amino group, such as phenoxycarbonyl, p-tolyloxycarbonyl, and the like.

The heteroarylalkyloxycarbonyl includes, for example, the above-mentioned alkoxycarbonyl group substituted with the above-mentioned aromatic heterocyclic group. Preferred examples of the heteroarylalkyloxycarbonyl group include a pyridyl-$C_{2-7}$ alkoxycarbonyl group (for example, 2-pyridylmethoxycarbonyl, 3-pyridylmethoxycarbonyl, etc.), and the like.

The amidated carboxyl group includes, for example, a group represented by the formula: -CON ($R^1$) ($R^2$) [wherein $R^1$ and $R^2$ may be the same or different and represent a hydrogen atom, an optionally substituted hydrocarbon group or an optionally substituted heterocyclic group]. The hydrocarbon group in the optionally substituted hydrocarbon group represented by $R^1$ or $R^2$ includes, for example, the aliphatic hydrocarbon group, the alicyclic hydrocarbon group and the aromatic hydrocarbon group exemplified as the substituent on the aromatic heterocyclic group represented by R. The heterocyclic group in the optionally substituted heterocyclic group represented by $R^1$ or $R^2$ includes, for example, the aromatic heterocyclic group exemplified as the substituent on the aromatic heterocyclic groups represented by R. The substituent on the hydrocarbon group or heterocyclic group in $R^1$ or $R^2$ includes, for example, 1 to 3 substituents selected from a halogen atom (for example, chlorine, fluorine, bromine, iodine, etc.), a $C_{1-6}$ alkyl group and a $C_{1-6}$ alkoxy group.

In the general formula (I), when the substituent on the aromatic heterocyclic group represented by R, the aromatic azole group represented by the formula:

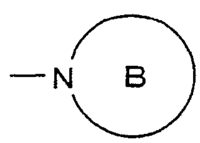

or the ring A is an alicyclic hydrocarbon group, an aromatic hydrocarbon group, an aliphatic hydrocarbon group substituted with an aromatic hydrocarbon group, an aromatic heterocyclic group, a non-aromatic heterocyclic group or an aliphatic hydrocarbon group substituted with an aromatic heterocyclic group, the alicyclic hydrocarbon group, the aromatic hydrocarbon group, the aromatic hydrocarbon group in the aliphatic hydrocarbon group substituted with an aromatic hydrocarbon group, the aromatic heterocyclic group, the non-aromatic hydrocarbon group or the aromatic heterocyclic group in the aliphatic hydrocarbon group substituted with an aromatic heterocyclic group may further have 1 to 3 (preferably 1 or 2) substituents at substitutable positions, respectively. Examples of such substituent include an optionally substituted $C_{1-6}$ alkyl group, a $C_{2-6}$ alkenyl group, a $C_{2-6}$ alkynyl group, a $C_{3-10}$ cycloalkyl group, a $C_{5-10}$ cycloalkenyl group, a $C_{6-14}$ aryl groups (for example, phenyl, naphthyl, etc.), an aromatic heterocyclic group (for example, thienyl, furyl, pyridyl, oxazolyl, thiazolyl, tetrazolyl, etc.), a non-aromatic heterocyclic group (for example, tetrahydrofuryl, morpholinyl, pyrrolidyl, piperazinyl, etc.), a $C_{7-20}$ aralkyl group (for example, a phenyl-$C_{1-6}$ alkyl group, a

naphthyl-$C_{1-6}$ alkyl group, etc.), an amino group, a N-mono($C_{1-6}$) alkylamino group, a N,N-di($C_{1-6}$)alkylamino group, a $C_{2-7}$ acylamino group (for example, a $C_{2-7}$ alkanoylamino group such as acetylamino, propionylamino, etc., a benzoylamino group, and the like), an amidino group, a $C_{2-7}$ acyl group (for example, a $C_{2-7}$ alkanoyl group, a benzoyl group, etc.), a carbamoyl group, a N-mono($C_{1-6}$) alkylcarbamoyl group, a N,N-di($C_{1-6}$) alkylcarbamoyl group, a sulfamoyl group, a N-mono ($C_{1-6}$) alkylsulfamoyl group, a N,N-di ($C_{1-6}$) alkylsulfamoyl group, a carboxyl group, a $C_{2-7}$ alkoxycarbonyl group, a $C_{8-21}$ aralkyloxycarbonyl group (for example, phenyl-$C_{2-7}$ alkoxycarbonyl, naphthyl-$C_{2-7}$ alkoxycarbonyl, etc.), a hydroxyl group, an optionally substituted $C_{1-6}$ alkoxy group, a $C_{2-6}$ alkenyloxy group, $C_{3-10}$ a cycloalkyloxy group, a $C_{5-10}$ cycloalkenyloxy group, a $C_{7-20}$ aralkyloxy group (for example, a phenyl-$C_{1-6}$ alkoxy group, a naphthyl-$C_{1-6}$ alkoxy group, etc.), a $C_{6-14}$ aryloxy group (for example, phenoxy, naphthyloxy, etc.), a mercapto group, a $C_{1-6}$ alkylthio group, a $C_{3-10}$ cycloalkylthio group, a $C_{7-20}$ aralkylthio group (for example, a phenyl-$C_{1-6}$ alkyl group, a naphthyl-$C_{1-6}$ alkylthio, etc.), a $C_{6-14}$ arylthio group (for example, phenylthio, naphthylthio, etc.), a sulfo group, a cyano group, an azide group, a nitro group, a nitroso group, a halogen atom (for example, fluorine, chlorine, bromine, iodine, etc.), and the like.

The substituent in the above-mentioned optionally substituted $C_{1-6}$ alkoxy group and optionally substituted $C_{1-6}$ alkyl group includes, for example, 1 to 3 substituents selected from a halogen atom (for example, fluorine, chlorine, bromine, iodine, etc.), a hydroxyl group, a $C_{1-6}$ alkoxy group and the like.

The substituted $C_{1-6}$ alkoxy group includes, for example, trifluoromethoxy, difluoromethoxy, 2,2,2-trifluoroethoxy, 1,1-difluoroethoxy, etc.

The substituted $C_{1-6}$ alkyl group includes, for example, trifluoromethyl, difluoromethyl, 2,2,2-trifluoroethyl, trichloromethyl, hydroxymethyl, methoxymethyl, 2-methoxyethyl, 2,2-dimethoxyethyl, etc.

In the general formula (I), when the substituent on the aromatic heterocyclic group represented by R, the aromatic azole group shown by the formula:

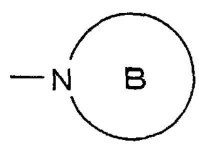

or the ring A is an aliphatic hydrocarbon group, an aliphatic hydrocarbon group substituted with an aromatic hydrocarbon group, or an aliphatic hydrocarbon group substituted with a aromatic heterocyclic group, the aliphatic hydrocarbon group, the aliphatic hydrocarbon group in the aliphatic hydrocarbon group substituted with an aromatic hydrocarbon group, or the aliphatic hydrocarbon group in the aliphatic hydrocarbon group substituted with an aromatic heterocyclic group may have further 1 to 3 (preferably 1 or 2) substituents at substitutable positions, respectively. Examples of such a substituent include a non-aromatic heterocyclic group (for example, tetrahydrofuryl, morpholinyl, piperidyl, pyrrolidyl, piperazinyl, etc.), an amino group, a N-mono ($C_{1-6}$) alkylamino group, a N,N-di ($C_{1-6}$) alkylamino group, a $C_{2-7}$ acylamino group (for example, a $C_{2-8}$ alkanoylamino group such as acetylamino, propionylamino, etc., a benzoylamino group, and the like), an amidino group, a $C_{2-7}$ acyl group (for example, a $C_{2-7}$ alkanoyl group, a benzoyl group, etc.), a carbamoyl group, a N-mono ($C_{1-6}$) alkylcarbamoyl group, a N,N-di($C_{1-6}$)alkylcarbamoyl group, a sulfamoyl group, a N-mono($C_{1-6}$)alkylsulfamoyl group, a N,N-di($C_{1-6}$)alkylsulfamoyl group, a carboxyl group, a $C_{2-7}$ alkoxycarbonyl group, a $C_{8-21}$ aralkyloxycarbonyl group (for example, a phenyl-$C_{2-7}$ alkoxycarbonyl group, a naphthyl-$C_{2-7}$ alkoxycarbonyl group, etc.), a hydroxyl group, an optionally substituted $C_{1-6}$ alkoxy group, a $C_{2-6}$ alkenyloxy group, a $C_{3-10}$ cycloalkyloxy group, a $C_{5-10}$ cycloalkenyloxy group, a $C_{7-20}$ aralkyloxy group (for example, a phenyl-$C_{1-6}$ alkoxy group, a naphthyl-$C_{1-6}$ alkoxy group, etc.), a $C_{6-14}$ aryloxy group (for example, phenoxy, naphthyloxy, etc.), a mercapto group, a $C_{1-6}$ alkylthio group, a $C_{3-10}$ cycloalkylthio group, a $C_{7-20}$ aralkylthio group (for example, a phenyl-$C_{1-6}$ alkyl group, a naphthyl-$C_{1-6}$ alkyl group, etc.), a $C_{6-14}$ arylthio group (for example, phenylthio, naphthylthio, etc.), a sulfo group, a cyano group, an azide group, a nitro group, a nitroso group, a halogen atom (for example, fluorine, chlorine, bromine, iodine, and the like).

The substituent in the above-mentioned optionally substituted $C_{1-6}$ alkoxy group includes, for example, 1 to 3 substituents selected from a halogen atom (for example, fluorine, chlorine, bromine, iodine, etc.), a hydroxyl group, a $C_{1-6}$ alkoxy group and the like. The above-mentioned substituted $C_{1-6}$ alkoxy group includes, for example, trifluoromethoxy, difluoromethoxy, 2,2,2-trifluoroethoxy, 1,1-difluoroethoxy, and the like.

Preferred examples of R are an oxazolyl group, a benzoxazolyl group or a thiazolyl group respectively substituted with 1 or 2 substituents selected from (i) an aryl group (for example, a phenyl group, a naphthyl group, etc.) optionally substituted with 1 or 2 substituents selected form a hydroxyl group, an alkoxy group (for example, a $C_{1-6}$ alkoxy group), an arylalkoxy group (for example, a phenyl-$C_{1-6}$ alkoxy group), an alkyl group (for example, a $C_{1-6}$ alkyl group), a cyano group, a halogen atom and a tetrazolyl group, (ii) an alkyl group (for example, a $C_{1-10}$ alkyl group), (iii) a hydroxyalkyl group (for example, a hydroxy-$C_{1-10}$ alkyl group), (iv) an alkoxycarbonylalkyl group (for example, a $C_{2-7}$

alkoxycarbonyl-$C_{1-10}$ alkyl group), (v) an alkyl group substituted with 1 or 2 aryl groups (for example, a $C_{1-6}$ alkyl group substituted with 1 or 2 phenyl groups), (vi) an alkenyl group substituted with 1 or 2 aryl groups (for example, a $C_{2-6}$ alkenyl group substituted with 1 or 2 phenyl groups), (vii) a cycloalkyl group (for example, a $C_{3-10}$ cycloalkyl group), (viii) a partially saturated naphthyl group (for example, a dihydronaphthyl group), (ix) a thienyl or furyl group optionally substituted with 1 or 2 substituents selected from a hydroxyl group, an alkoxy group, an arylalcohol group, an alkyl group, a cyano group, an allyl group and a halogen atom, (x) a benzofuranyl group and (xi) a benzothienyl, with an oxazolyl group substituted with an arylalkenyl group (for example, a phenyl-$C_{2-6}$ alkenyl group) and an oxazolyl group substituted with an arylalkoxy-aryl group (for example, a phenyl-$C_{1-6}$ alkoxyphenyl group) being more preferred.

Preferred examples of the aromatic azole group represented by the formula:

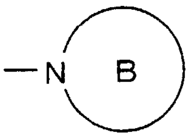

include a pyrrolyl group, an imidazolyl group, a pyrazolyl group, a triazolyl group, a tetrazolyl group or a benzimidazolyl group respectively substituted with 1 or 2 substituents selected from (i) an alkyl group (e.g. a $C_{1-10}$ alkyl group), (ii) an aryl group (e.g. a phenyl group), (iii) a hydroxylalkyl group (e.g. a hydroxy-$C_{1-10}$ alkyl group), (iv) a carboxyl group, (v) an alkoxycarbonyl group (e.g. a $C_{2-7}$ alkoxycarbonyl group) and (vi) a carbamoyl group, with an imidazolyl group and a triazolyl group being more preferred.

The ring A forms, according to the kind of Y (CH or N), an optionally substituted benzene ring or an optionally substituted pyridine ring. Preferred examples include an optionally substituted benzene ring. More preferred examples include a benzene ring or a pyridine ring optionally substituted with 1 or 2 $C_{1-6}$ alkoxy groups.

Preferred examples of the group represented by the formula:

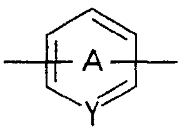

include a group represented by

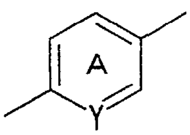

or

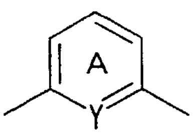

and most preferable one is a 1,3-phenylene group or a 1,4-phenylene group.

X represents an oxygen atom (O), an optionally oxidized sulfur atom [S(O)k (k represents an integer of 0 to 2)], -C(=O)- or -CH(OH)-, and the preferred examples include an oxygen atom, etc.

The symbol p represents an integer of 0 to 10, preferably 0 to 6, more preferably 3 to 5.

The symbol q represents an integer of 1 to 5, preferably 1.

As specific examples of the compound (I), the compounds prepared in the Examples of JP 11-60571 A are used. Among them, preferred examples include (i) 1-[4-[4-[2-[(E)-2-phenylethenyl]-4-oxazolylmethoxy]phenyl]butyl]-1,2,4-triazole, (ii) 4-[4-[4-(1-imidazolyl)butyl]phenoxymethyl]-2-[(E)-2-phenylethenyl]oxazole, (iii) 4-[4-[3-( 1-imidazolyl) propyl]phenoxymethyl]-2-[(E)-2-phenylethenyl]oxazole, (iv) 4-[3-[3-(1-imidazolyl)propyl]phenoxymethyl]-2-[(E)-2-phenylethenyl]oxazole, (v) 2-(4-benzyloxyphenyl)-4-[4-[4-[3-(1-imidazolyl)propyl]phenoxymethyl]oxazole, (vi) 4-[4-[3-(1-imidazolyl)propyl]phenoxymethyl]-2-(2-thienyl)oxazole, (vii) 4-[4-[3-(1-imidazolyl)propyl]phenoxymethyl]-2-(5-methyl-2-thienyl)oxazole, (viii) 2-(5-chloro-2-thienyl)-4-[4-[3-(1-imidazolyl)-propyl]phenoxymethyl]oxazole, (ix) 4-[4-[3-(1-imidazolyl)propyl]phenoxymethyl]-2-(2-thienyl)thiazole, (x) 5-[4-[3-(1-imidazolyl)propyl]phenoxymethyl]-2-(2-thienyl)benzoxazole, and the like.

The above-mentioned compound (I) is preferably, for example, a group represented by the formula:

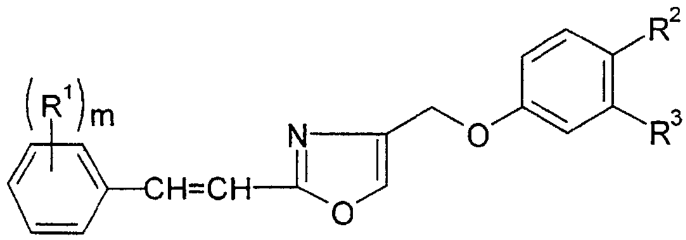

(I')

wherein m represents 1 or 2, $R^1$ represents halogen or optionally halogenated $C_{1-2}$ alkyl, one of $R^2$ and $R^3$ is a hydrogen atom and the other one is a group represented by the formula:

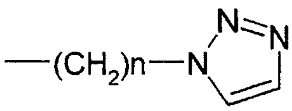

or

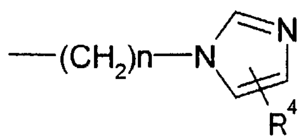

wherein n represents 3 or 4, and $R^4$ represents a $C_{1-4}$ alkyl group substituted with 1 to 2 hydroxy groups, and the like.

In the above-mentioned formula (I'), examples of the "halogen" represented by $R^1$ include fluoro, chloro, bromo, iodo, etc. Among them, fluoro is preferred.

Examples of the "halogen" of the "optionally halogenated $C_{1-2}$ alkyl" represented by $R^1$ include fluoro, chloro, bromo, iodo, etc. Among them, fluoro is preferred.

Examples of the "$C_{1-2}$ alkyl" of optionally halogenated $C_{1-2}$ alkyl" represented by $R^1$ include methyl and ethyl. Among them, methyl is preferred.

The "$C_{1-2}$ alkyl" may have 1 to 3, preferably 2 to 3, halogens at substitutable positions and, when the number of halogens is 2 or more, the respective halogens may be the same or different.

Specific examples of the "optionally halogenated $C_{1-2}$ alkyl" include methyl, ethyl, trifluoromethyl, and the like.

$R^1$ is preferably halogen or halogenated $C_{1-2}$ alkyl, more preferably fluoro and trifluoromethyl.

When m is 2, the respective $R^1$ may be different.

A group represented by the formula:

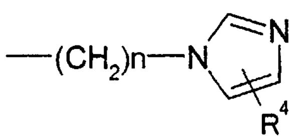

wherein $R^4$ is as defined above, which is represented by $R^2$ or $R^3$, is preferably a group represented by the formula:

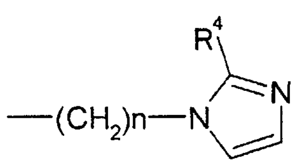

wherein $R^4$ is as defined above.

Examples of the "$C_{1-4}$ alkyl group" of the "$C_{1-4}$ alkyl group substituted with 1 to 2 hydroxy groups" represented by $R^4$ include methyl, ethyl, propyl, isopropyl, butyl, isobutyl, sec-butyl, tert-butyl, etc. Among them, ethyl, propyl, etc., are preferred.

Specific examples of the "$C_{1-4}$ alkyl group substituted with 1 to 2 hydroxy groups" include 2-hydroxyethyl, 2,3-dihydroxypropyl, 1,3-dihydroxypropyl, and the like. Among them, 2,3-dihydroxypropyl is preferred.

In the above-mentioned formula, $R^2$ is preferably a group represented by formula:

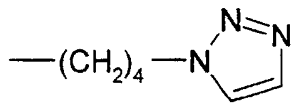

and $R^3$ is preferably a hydrogen atom.

It is also preferable that $R^2$ is a hydrogen atom and $R^3$ is a group represented by the formula:

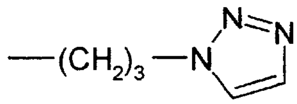

It is also preferable that $R^2$ is a group represented by the formula:

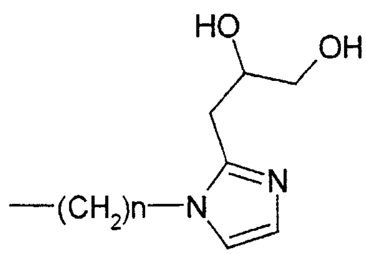

wherein n is as defined above, and $R^3$ is a hydrogen atom, more preferably n is 4.

Preferred examples of the compound (I') include a compound represented by the formula:

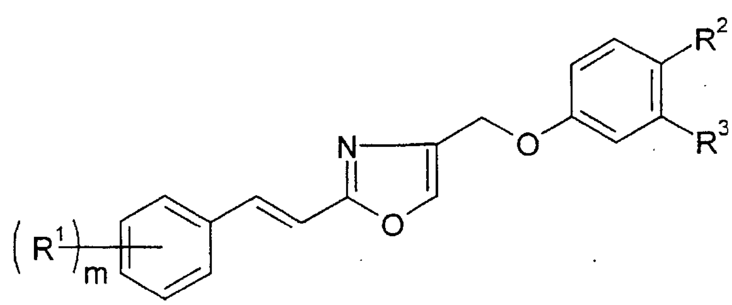

wherein the respective symbols are as defined above, or a salt thereof.

Among the compounds (I'), a compound wherein m is 1, $R^1$ is 4-trifluoromethyl, $R^2$ is a group represented by the formula:

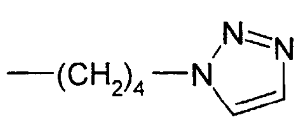

and $R^3$ is a hydrogen atom, or a salt thereof is preferred.

Specific examples of the compound (I') include:

(1) 1-(4-{4-[(2-{(E)-2-[4-(trifluoromethyl)phenyl]ethenyl}-1,3-oxazol-4-yl)methoxy]phenyl}butyl)-1H-1,2,3-triazole,
(2) 1-(3-{3-[(2-{(E)-2-[4-(trifluoromethyl)phenyl] ethenyl}-1,3-oxazol-4-yl)methoxy]phenyl}propyl)-1H-1,2,3-triazole,

(3) 3-(1-{4-[4-({2-[(E)-2-(2,4-difluorophenyl)ethenyl]-1,3-oxazol-4-yl }methoxy)phenyl]butyl}-1H-imidazol-2-yl)-1,2-propanediol, etc.

As a salt of the compound (I) of the present invention, a pharmaceutically acceptable salt is preferable. Examples thereof include salts with inorganic bases, salts with organic bases, salts with inorganic acids, salts with organic acids, salts with basic or acidic amino acids, etc. Preferred examples of the salts with inorganic bases include alkali metal salts such as a sodium salt, a potassium salt, etc.; alkaline earth metal salts such as a calcium salt, a magnesium salt, etc.; an aluminum salt; an ammonium salt; and the like. Preferred examples of the salts with organic bases include salts with trimethylamine, triethylamine, pyridine, picoline, ethanolamine, diethanolamine, triethanolamine, dicyclohexylamine, N,N-dibenzylethylenediamine, and the like. Preferred examples of the salts with inorganic acids include salts with hydrochloric acid, hydrobromic acid, nitric acid, sulfuric acid, phosphoric acid, and the like. Preferred examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like. Preferred examples of the salts with basic amino acids include salts with arginine, lysine, ornithine, and the like. Preferred examples of salts with acidic amino acids include salts with aspartic acid, glutamic acid, and the like.

In the compound (I), there are two kinds of compounds, i.e., the (Z)-ethenyl and (E)-ethenyl compounds and the present invention encompasses these isomers alone and a mixture thereof.

In case that the compound (I) contains an asymmetric carbon, an optical isomer is formed and the present invention encompasses these isomers alone and a mixture thereof.

The compound (I) or a salt thereof of the present invention can be produced by, for example, the process described in JP 11-60571 A or a modification thereof.

Particularly, the compound (I') or a salt thereof can be produced by, for example, the processes shown in the following reaction schemes A to H.

The respective symbols in the following reaction schemes are as defined above. The compounds in the reaction schemes also include salts thereof.

Reaction scheme A

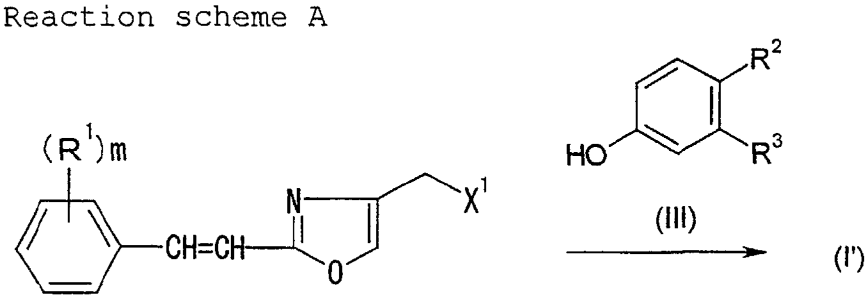

(III)

(I')

(II)

Examples of the "leaving group" represented by $X^1$ include a halogen (for example, chloro, bromo, etc.) or a group represented by the formula: $-OSO_2R^5$ [wherein $R^5$ represents an alkyl or an optionally substituted aryl].

Examples of the "alkyl" represented by $R^5$ include a $C_{1-6}$ alkyl such as methyl, ethyl, propyl, etc.

Examples of the "aryl" of the "optionally substituted aryl" represented by $R^5$ include a $C_{6-14}$ aryl such as phenyl, etc.

Examples of the "substituent" of the "optionally substituted aryl" represented by $R^5$ include a $C_{1-6}$ alkyl such as methyl, ethyl, propyl, etc.

Specific examples of the "optionally substituted aryl" include phenyl which may have a $C_{1-6}$ alkyl (for example, p-tolyl, etc.), and the like.

The compound (II) is reacted with the compound (iii) to obtain the compound (I').

According to this reaction, the compound (II) and the compound (III) are usually condensed in the presence of a base.

Examples of the "base" include hydroxides of alkali metals and alkaline earth metals (for example, sodium hydroxide, potassium hydroxide, etc.), carbonates of alkali metals and alkaline earth metals (for example, sodium hydrogencarbonate, potassium carbonate, etc.), amines (for example, pyridine, triethylamine, N,N-dimethylaniline, etc.), hydrides of alkali metals and alkaline earth metals (for example, sodium hydride, potassium hydride, calcium hydride, etc.), lower alkoxides of alkali metals and alkaline earth metals (for example, sodium methoxide, sodium

ethoxide, potassium tert-butoxide, etc.).

The amount of the "base" is preferably within a range from about 1 to 5 molar equivalents based on the compound (II).

The amount of the "compound (III)" is preferably within a range from about 0.5 to 5 molar based on the compound (II).

It is advantageous to carry out this reaction in the presence of a solvent which does not affect the reaction. The solvent is not specifically limited as long as the reaction proceeds and there can be used, for example, aromatic hydrocarbons, ethers, ketones, halogenated hydrocarbons, amides, sulfoxides, or a mixture of two or more kinds of them.

The reaction temperature is usually within a range from -50 to +150°C, preferably from about -10 to +100°C. The reaction time is usually within a range from 0.5 to 48 hours.

The compound (II) can be produced by a per se known process or a process analogous thereto and, for example, the compound (IIa) wherein X is chloro can be produced by a process shown in the following reaction scheme B.

Reaction scheme B

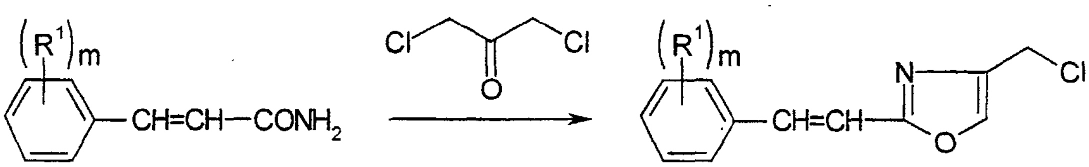

(IV) (IIa)

The compound (IV) and 1, 3-dichloroacetone are subjected to the condensation and dehydration reaction to obtain the compound (IIa).

In case that the compound (IV) is commercially available, a commercially available product may be used as it is, or may be produced according to a per se known process or a process analogous thereto.

The amount of "1,3-dichloroacetone" is within a range from about 1 molar equivalent to an excess amount (amount sufficient for a solvent) based on the compound (IV).

It is advantageous to carry out this reaction without any solvent or in a presence of a solvent which does not affect the reaction. The solvent is not specifically limited as long as the reaction proceeds and there can be used, for example, aromatic hydrocarbons, ethers, ketones, halogenated hydrocarbons, or a mixture of two or more kinds of them.

The reaction temperature is usually within a range from 50 to 150ºC, preferably about 60 to 120ºC. The reaction time is usually within a range from 0.5 to 48 hours.

The product can be used as it is in the form of a reaction mixture or a crude product in the subsequent reaction, or can be isolated from the reaction mixture according to a conventional method.

Among the compounds (III), the compound (IIIa) wherein $R^3$ is a hydrogen atom can be produced by a per se known process or a process analogous thereto, for example, it can be produced by the process shown in the following reaction scheme C.

Reaction scheme C

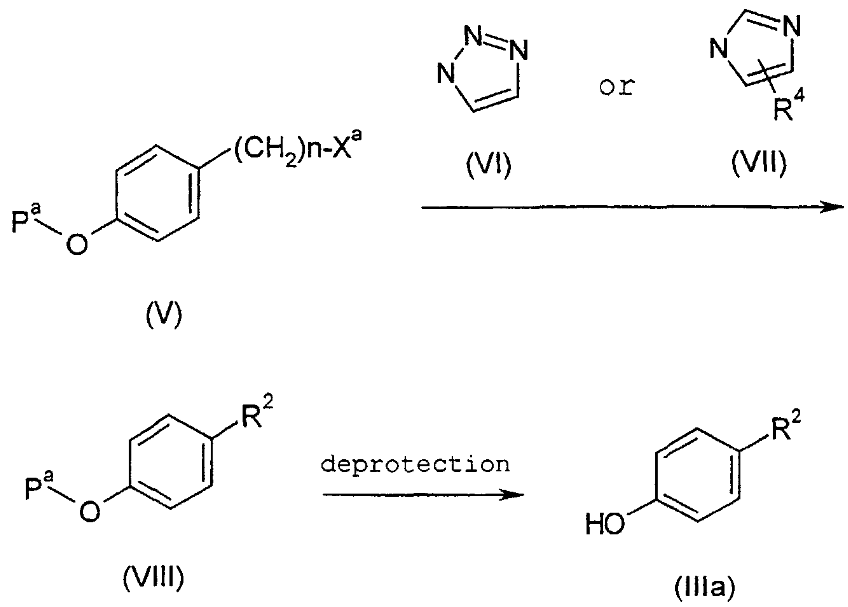


(V) (VI) or (VII) (VIII) (IIIa)

In the above-mentioned formula, $P^a$ represents a hydrogen atom or a protective group, and $X^a$ represents a leaving group.

Examples of the "protective group" represented by $P^a$ include an alkyl (for example, a $C_{1-6}$ alkyl such as methyl, ethyl, etc.), a phenyl-$C_{1-6}$ alkyl (for example, benzyl, etc.), a $C_{1-6}$ alkyl-carbonyl, an alkyl-substituted silyl (for example, trimethylsilyl, tert-butyldimethylsilyl, etc.), and the like.

Examples of the "leaving group" represented by $X^a$ include those exemplified as the "leaving group" represented by R.

The compound (V) is condensed with the compound (VI) or the compound (VII) to obtain the compound (VIII), if necessary, followed by deprotection to obtain a compound (IIIa).

In case that the compound (V), the compound (VI) and the compound (VII) are commercially available, commercially available products may be used as they are, or may be produced by a per se known process or a process analogous thereto.

The "condensation reaction" is usually conducted in a solvent which does not affect the reaction in the presence of a base.

As the "base", there can be used that described in detail with respect to the above-mentioned reaction scheme A.

The amount of the "base" is preferably within a range from about 1 to 5 molar equivalents based on the compound (V) .

The amount of the "compound (VI) or compound (VII)" is preferably within a range from about 0.5 to 5 molar equivalents based on the compound (V).

The solvent is not specifically limited as long as the reaction proceeds and there can be used, for example, aromatic hydrocarbons, ethers, ketones, halogenated hydrocarbons, amides, sulfoxides, or a mixture of two or more kinds of them.

The reaction temperature is usually within a range from -50 to +150°C, preferably from about -10 to +100°C. The reaction time is usually within a range from 0.5 to 48 hours.

The resulting compound (VIII) can be used as it is in the form of a reaction mixture or a crude product in the subsequent reaction, or can be isolated from the reaction mixture according to a conventional method.

The "deprotection reaction" can be appropriately selected from conventional methods.

For example, in case that $P^a$ is an alkyl, the compound (VIII) is subjected to treatment with an acid (for example, mineral acids such as hydrobromic acid, etc., and Lewis acids such as titanium tetrachloride, etc.).

In case that $P^a$ is a phenyl-$C_{1-6}$ alkyl, the compound (VIII) is subjected to a hydrogenation reaction.

In case that $P^a$ is an alkyl-substituted silyl, the compound (VIII) is reacted with a fluoride (for example, tetrabutylammonium fluoride, etc.).

The resulting compound (IIIa) can be used as it is in the form of a reaction mixture or a crude product in the

subsequent reaction, or can be isolated from the reaction mixture according to a conventional method.

Among the compounds (III), the compound (IIIb) wherein $R^2$ is a hydrogen atom can be produced by a per se known process or a process analogous thereto, and can be produced by the process shown in the following reaction scheme D.

Reaction scheme D

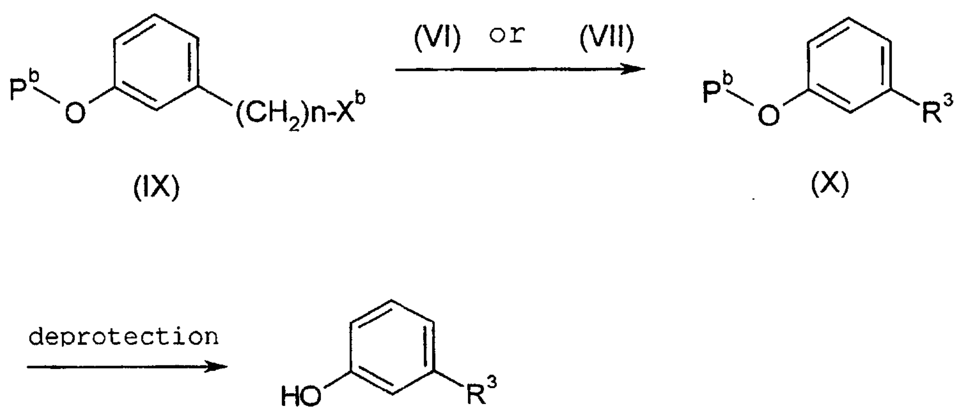


(IX) (X)

(IIIb)

In the above-mentioned formula, $P^b$ represents a hydrogen atom or a protective group, and $X^b$ represents a leaving group.

Examples of the "protective group" represented by $P^b$ include those exemplified as the "protective group" represented by $p^a$.

Examples of the "leaving group" represented by $X^b$ include those exemplified as the "leaving group" represented by $X^1$.

According to the same manner as in the reaction scheme C, the compound (IX) is condensed with the compound (VI) or the compound (VII) to obtain the compound (X), if necessary, followed by deprotection to obtain the compound (IIIb).

In case that the compound (IX) is commercially available, a commercially available product may be used as it is, or may be produced by a per se known process or a process analogous thereto.

Among the compounds (I'), the compound (I'a) wherein $R^3$ is a hydrogen atom can also be produced by the process shown in the following reaction scheme E.

Reaction scheme E

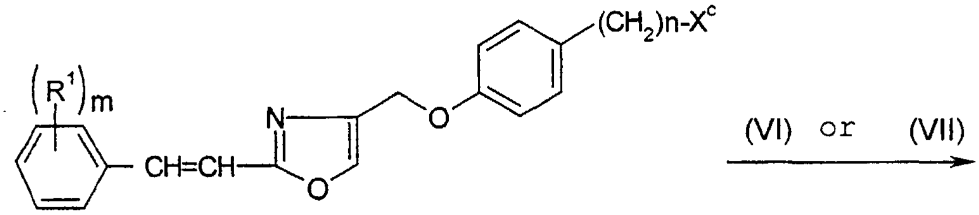


(XI)

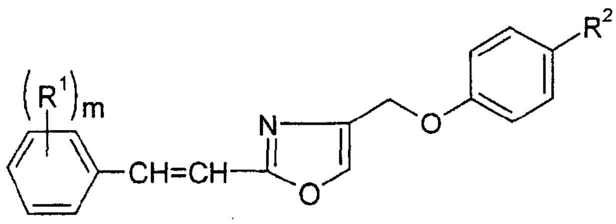

(I'a)

In the above-mentioned formula, $X^c$ represents a leaving group.
Examples of the "leaving group" represented by $X^c$ include those exemplified as the "leaving group" represented by $X^1$.
The compound (XI) is reacted with the compound (VI) or the compound (VII) to obtain the compound (Ia).
According to this reaction, the compound (XI) is usually condensed with the compound (VI) or the compound (VII) in the presence of a base.
As the "base", there can be used the base described in detail with respect to the above-mentioned reaction scheme A.
The amount of the "base" is within a range from about 1 to 5 molar equivalents based on the compound (XI).
The amount of the "compound (VI) or compound (VII)" is preferably within a range from about 0.5 to 5 molar equivalents based on the compound (XI).
The solvent is not specifically limited as long as the reaction proceeds and there can be used, for example, aromatic hydrocarbons, ethers, ketones, halogenated hydrocarbons, amides, sulfoxides, or a mixture of two or more kinds of them.
The reaction temperature is usually within a range from -20 to +150°C, preferably from about -10 to +100°C. The reaction time is usually within a range from 0.5 to 48 hours.
The compound (XI) can be produced by a per se known process or a process analogous thereto, and can be produced by the process shown in the following reaction scheme F.

Reaction scheme F

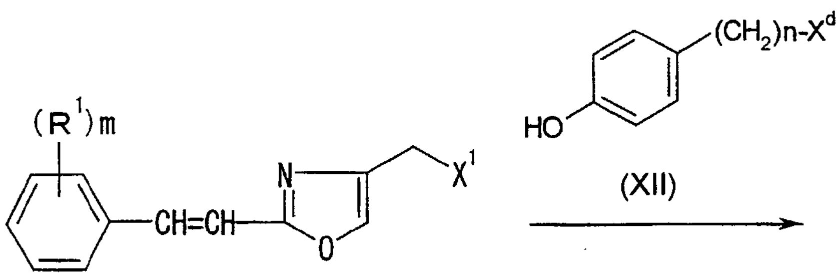


(XI)

(II)

In the above-mentioned formula, $X^d$ represents a leaving group.
The "leaving group" represented by $X^d$ include, for example, include those exemplified as the above "leaving group" represented by $X^1$, preferably leaving group having lower reactivity than that represented by $X^1$.
In the same manner as in the reaction scheme A, the compound (II) is reacted with the compound (XII) to obtain the compound (XI).
In case that the compound (XII) is commercially available, a commercially available product may be used as it is, or may be produced by a per se known process or a process analogous thereto.
Among the compounds (I'), the compound (I'b) wherein $R^2$ is a hydrogen atom can also be produced by the process shown in the following reaction scheme G.

Reaction scheme G

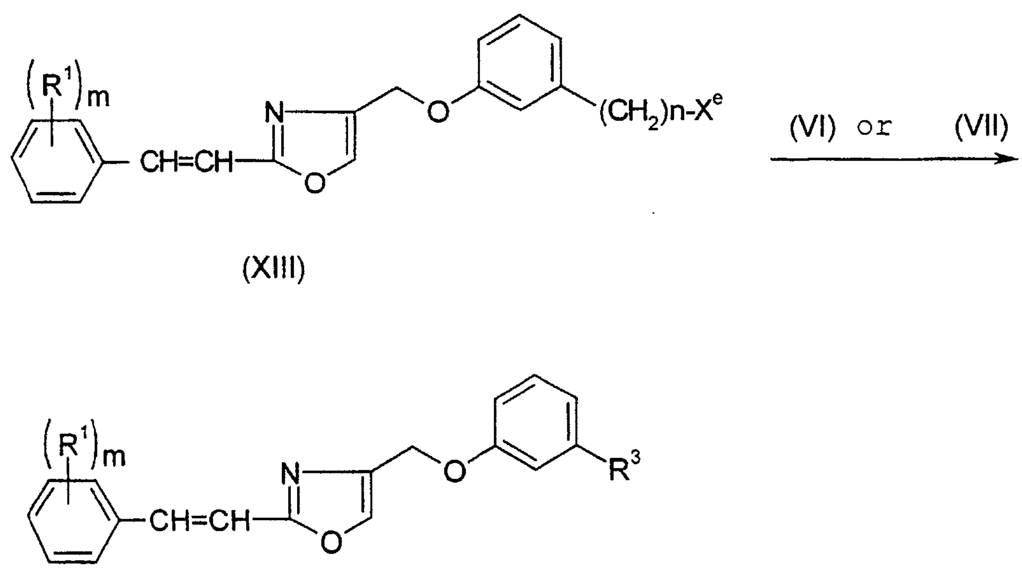


(XIII)

(I'b)

In the above-mentioned formula, $X^e$ represents a leaving group.
The "leaving group" represented by $X^e$ include, for example, include those exemplified as the "leaving group" represented by $X^1$.
In the same manner as in the reaction scheme E, the compound (XIII) is reacted with the compound (VI) or the compound (VII) to obtain the compound (I'b).
The compound (XIII) can be produced by a per se known method or a process analogous thereto and, for example, can be produced by the process shown in the following reaction scheme H.

Reaction scheme H

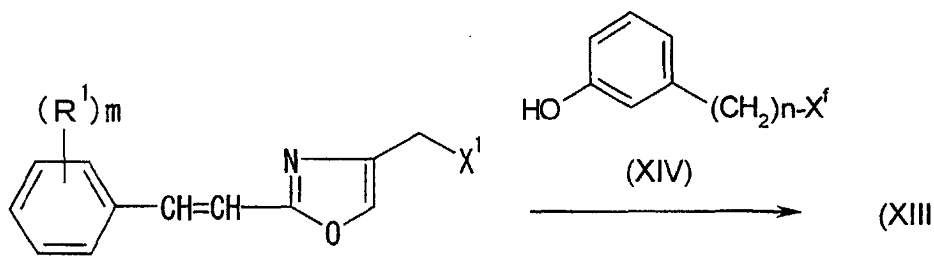

(XIV)

(XIII

In the above-mentioned formula, $X^f$ represents a leaving group.
The "leaving group" represented by $X^f$ include, for example, include those exemplified as the "leaving group" represented by $X^1$, preferably leaving group having lower reactivity than that represented by $X^1$.

In the same manner as in the reaction scheme A, the compound (II) is reacted with the compound (XIV) to obtain the compound (XIII).
In case that the compound (XIV) is commercially available, a commercially available product may be used as it is, or may be produced by a per se known process or a process analogous thereto.
As the above-mentioned "aromatic hydrocarbons", for example, there can be used benzene, toluene, xylene, etc.
As the above-mentioned "ethers", for example, there can be used tetrahydrofuran, dioxane, etc.
As the above-mentioned "ketones", for example, there can be used acetone, 2-butanone, etc.
As the above-mentioned "halogenated hydrocarbons", for example, there can be used chloroform, dichloromethane, etc.
As the above-mentioned "amides", for example, there can be used N,N-dimethylformamide, etc.
As the above-mentioned "sulfoxides", for example, there can be used dimethyl sulfoxide, etc.
In the above-mentioned reactions, when the product is obtained as a free compound, it can be converted into the corresponding salt, and, when the product is obtained as a salt, it can be converted into the free compound, by a per se known method or a method analogous thereto, respectively.
In the above-mentioned reactions, when the substituent contains amino ($NH_2$), hydroxy (OH), carboxyl (COOH), etc., the compound in which such group is protected may be used as a starting compound, and, after completion of the reaction, the protecting group is removed to produce the objective compound. The amino-protecting group includes, for example, an acyl group (e.g. a $C_{1-6}$ alkyl-carbonyl group such as acetyl; a $C_{1-6}$ alkoxycarbonyl such as benzyloxycarbonyl and tert-butoxycarbonyl; phthaloyl; formyl; etc.), and the like. The hydroxyl-protecting group includes, for example, a $C_{1-6}$ alkyl (for example, methyl, ethyl, etc.), a phenyl-$C_{1-6}$ alkyl (for example, benzyl, etc.), a $C_{1-6}$ alkyl-carbonyl (for example, acetyl, etc.), benzoyl, an alkyl-substituted silyl (for example, trimethylsilyl, tertbutylsilyl, etc.), and the like. The carboxyl-protecting group includes, for example, a $C_{1-6}$ alkyl (for example, methyl, ethyl, etc.), a phenyl-$C_{1-6}$ alkyl (for example, benzyl, etc.), and the like.
The compound (I') [including compounds (I'a) and (I'b)] thus obtained can be isolated and purified by a per se known isolating and purifying means such as concentration, concentration under reduced pressure, solvent extraction, crystallization, recrystallization, phasic transfer, chromatography, and the like.
When the compound (I') is obtained as a free compound, it can be converted into the objective salt and, when the compound is obtained as a salt, it can be converted into the free compound, by a per se known method or a method analogous thereto, respectively.
The compound (I) may be a hydrate or a non-hydrate.
In case that the compound (I) is obtained as a mixture of optically active substances, it can be separated into the objective (R)- and (S)-forms by a per se known optical resolution means.
The compound (I) may be labeled with an isotope (for example, $^3H$, $^{14}C$, etc.).
The prodrug of the compound (I) or a salt thereof (abbreviated to the compound (I)) may be a compound which is converted into the compound (I) through, for example, enzyme- or gastric acid-mediated reaction *in vivo* under physiological conditions, i.e., compounds which may undergo enzymatic oxidization, reduction, hydrolysis, etc., to be converted into the compound (I), and compounds which may undergo hydrolysis by gastric acid, etc., to be converted into the compound (I).
Examples of the prodrug of the compound (I) include compounds wherein the amino group of the compound (I) has been acylated, alkylated or phosphorylated (e.g., compounds wherein the amino group of the compound (I) has been eicosanoylated, alanylated, pentylaminocarbonylated, (5-methyl-2-oxo-1,3-dioxolene-4-yl) methoxycarbonylated, tetrahydrofuranylated, pyrrolidylmethylated, pivaloyloxymethylated, tert-butylated, etc.); compounds wherein the hydroxy group of the compound (I) has been acylated, alkylated, phosphorylated or borated (e.g., compounds wherein the hydroxy group of the compound (I) has been acetylated, palmitoylated, propanoylated, pivaloylated, succinylated, fumarylated, alanylated or dimethylamino methylcarbonylated, etc.); compounds wherein the carboxyl group of the compound (I) has been esterified or amidated (e.g., compounds wherein the carboxyl group of the compound (I) has been ethylesterified, phenylesterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolene-4-yl)methyl esterified, cyclohexyloxycarbonylethyl esterified, methylamidated, etc.), and the like. These compounds can be prepared from the compound (I) using a per se known method.
Further, prodrugs of the compound (I) may be compounds which may be converted into the compound (I) under physiological conditions as described in "Development of pharmaceuticals (Iyakuhin no Kaihatsu)", vol. 7, Molecular Design, pp. 163-198, Hirokawa Shoten (1990).
The composition containing the HER2 inhibitor having an average particle size of about 3 μm or less of the present invention may be the HER2 inhibitor having an average particle size of about 3 μm or less, the HER2 inhibitor having an average particle size of about 3 μm or less when dispersed in an aqueous solution, or a crystalline particulate thereof of the present invention as it is, or may contain suitable additives. Further, the form of the preparation is not

specifically limited to and may be any of solid preparations such as powders, granules, etc.; and liquid preparations such as suspensions, etc.

As the additives contained in the composition of the present invention, for example, stabilizers for improving the dispersibility in an aqueous solution can be used. Examples of the stabilizers include surfactants, hydrophilic polymers, easily water-soluble cyclodextrin derivatives or salts thereof, etc., and these stabilizers can be used alone or in combination. Among them, surfactants, high molecular weight polymers, and a combination thereof are preferred.

As the surfactants, there can be used nonionic surfactants, anionic surfactants, cationic surfactants, amphoteric surfactants and surfactants derived from natural products, etc.

As the nonionic surfactants, there can be used higher alcohol ethylene oxide adducts, alkylphenol ethylene oxide adducts, fatty acid ethylene oxide adducts, polyhydric alcohol fatty acid ester ethylene oxide adducts, higher alkylamine ethylene oxide adducts, fatty acid amide ethylene oxide adducts, ethylene oxide adducts of fats and oils, glycerin fatty acid esters, fatty acid esters of pentaerythritol, alkyl ethers of polyhydric alcohol, fatty acid amides of alkanolamines, etc.

Among these nonionic surfactants, for example, there can be preferably used fatty acid esters of sorbitol and sorbitan, polyoxyethylene sorbitan fatty acid esters, polyethylene glycol fatty acid esters, sucrose fatty acid esters, polyethoxylated castor oils, polyethoxylated hydrogenated castor oils, polyoxyethylene propylene glycol copolymers, glycerin fatty acid esters, polyglycerin fatty acid esters, etc. Preferred examples of the sorbitan fatty acid esters include sorbitan monostearate (trade name: SS-10, manufactured by NIKKO CHEMICALS CO., LTD.), sorbitan sesquioleate (trade name: SO-15, manufactured by NIKKO CHEMICALS CO., LTD.), sorbitan trioleate (trade name: SO-30, manufactured by NIKKO CHEMICALS CO., LTD.), etc. Preferred examples of the polyoxyethylenesorbitan fatty acid ester include Polysolvate 20 (trade name: TL-10, manufactured by NIKKO CHEMICALS CO., LTD.), Polysolvate 40 (trade name: TP-10, manufactured by NIKKO CHEMICALS CO., LTD.), Polysolvate 60 (trade name: TS-10, manufactured by NIKKO CHEMICALS CO., LTD.), Polysolvate 80 (trade name: TO-10, manufactured by NIKKO CHEMICALS CO., LTD.), etc. Preferred examples of the polyethylene glycol fatty acid esters include polyethylene glycol monolaurate (10 E.O.) (trade name: MYL-10, manufactured by NIKKO CHEMICALS CO., LTD.), etc. Preferred examples of the sucrose fatty acid esters include sucrose palmitates (for example, trade name: P-1670, manufactured by Mitsubishi-Kagaku Foods Corporation), sucrose stearates (for example, trade name: S-1670, manufactured by Mitsubishi-Kagaku Foods Corporation), etc. Preferred examples of the polyethoxylated castor oils include polyoxyethylene glycerol triricinoleate 35 (Polyoxy 35 Castor Oil, trade name: Cremophor EL or EL-P, manufactured by BASF, Japan), etc. Preferred examples of the polyethoxylated hydrogenated castor oils include polyoxyethylene hydrogenated castor oil 50, polyoxyethylene hydrogenated castor oil 60, etc. Preferable examples of the polyoxyethylene polyoxypropylene glycol copolymers include polyoxyethylene (160) polyoxypropylene (30) glycol (trade name: ADEKA PLURONIC F-68, manufactured by ASAHI DENKA KOGYO K.K.), etc. Preferred examples of the glycerin fatty acid esters include glyceryl monostearate (MGS series, manufactured by NIKKO CHEMICALS CO., LTD.), etc. Preferred examples of the polyglycerin fatty acid esters include tetraglycerinmonostearic acid (MS-310, manufactured by Sakamoto Yakuhin Kogyo Co., Ltd.), decaglycerinmonolauric acid (Decaglyn 1-L, manufactured by NIKKO CHEMICALS CO., LTD.), etc.

As the anionic surfactants, for example, there can be used sulfate salts (for example, higher alcohol sulfate salt, higher alkyl ether sulfate salt, sulfated oil, sulfated fatty acid ester, sulfated fatty acid and sulfated olefin), sulfonate salts (for example, sodium alkylbenzenesulfonate, oil soluble alkylbenzenesulfonte salt, $\alpha$-olefin sulfonate salt, Igepon T type and aerosol OT type), phosphate esters (for example, phosphate ester of higher alcohol ethylene oxide adduct), dithiophosphate salts, and the like.

Among these anionic surfactants, for example, bile acid salts such as sodium glycolate, sodium deoxycholate, etc.; fatty acids and salts thereof, such as stearic acid and sodium caprate; and alkyl sulfate salts such as sodium lauryl sulfate are preferred.

As the cationic surfactants, for example, there can be used amine salt type cationic surfactants (for example, amine salt type cationic surfactant made of higher alkylamine, amine salt type cationic surfactant made of lower alkylamine), quaternary ammonium salt type cationic surfactants (for example, quaternary ammonium salt type cationic surfactant made of higher alkylamine and quaternary ammonium salt type cationic surfactant made of lower alkylamine), etc.

As the amphoteric surfactants, for example, there can be used amino acid type amphoteric surfactants, betain type amphoteric surfactants, etc.

As the surfactants derived from natural products, for example, there can be used lecithin phospholipids such as yolk lecithin (trade name: PL-100H, manufactured by Q.P. Corporation), soybean lecithin (trade name: Lecinol S-10, manufactured by NIKKO CHEMICALS CO., LTD.), etc.

Among them, sodium deoxycholate, alkyl sulfates, Polysolvate 80, polyoxyethylene (160) polyoxypropylene (30), polyoxyethylene glycerol tricicynolate 35, lecithins, polyethoxylated hydrogenated castor oils, sucrose fatty acid esters and polyglycerin fatty acid esters are more preferable, and alkyl sulfate salts and sucrose fatty acid esters are still more preferable. Among the above-mentioned alkyl sulfate salts, sodium lauryl sulfate is preferred. Among sucrose

fatty acid esters, sucrose stearate is more preferred.

As the hydrophilic polymers, for example, water-soluble polymers are preferably used.

As the water-soluble polymers, there can be used cellulose derivatives, for example, hydroxyalkylcelluloses such as hydroxypropylcellulose, hydroxypropylmethylcellulose, etc., and alkylcelluloses such as methylcellulose, etc.; polyalkenyl pyrrolidones such as polyvinyl pyrrolidone, etc.; polyalkylene glycols such as polyethylene glycol, etc.; polyvinyl alcohol; and the like.

Among these polymers, hydroxyalkylcelluloses such as hydroxypropylcellulose, etc., polyalkenyl pyrrolidones such as polyvinyl pyrrolidone, etc., and the like are preferred, and hydroxypropylcellulose, polyvinyl pyrrolidone, and the like are more preferred.

As the easily water-soluble cyclodextrin derivatives, a commercially available product may be used. Alternatively, the easily water-soluble cyclodextrin derivatives can also be produced by a per se known method.

As the easily water-soluble cyclodextrin derivative, there can be preferably used a compound which is a cyclic oligosaccharide composed of 6 to 12 glucose units and whose hydrogens of a part or all of the hydroxyl groups at the 2-, 3- and 6-positions of the glucoses are substituted with other functional groups (for example, dihydroxyalkyl group, sugar residue, hydroxyalkyl group, sulfo alkyl group, etc.), and the like.

The easily water-soluble derivative has water solubility of about 100 mg/ml or more, preferably about 130 mg/ml or more.

Preferred specific examples of the easily water-soluble cyclodextrin derivative include a compound represented by the formula:

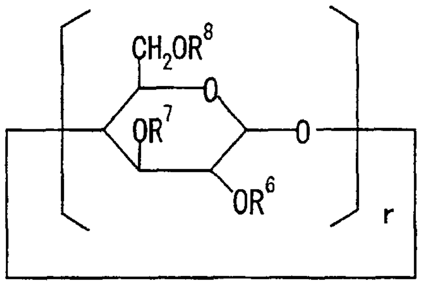

wherein r represents an integer of 6 to 12, and $R^6$, $R^7$ and $R^8$ may be the same or different in each repeating unit and each represents a dihydroxyalkyl group, a sugar residue, a hydroxyalkyl group or a sulfoalkyl group. Specific examples thereof include ether derivatives of hydroxyl groups such as α-CyD (r = 6), β-CyD (r = 7), γ-CyD (r = 8), δ-CyD (r = 9), etc. Among them, an ether derivative of hydroxyl group such as β-CyD is preferred.

As the dihydroxyalkyl group represented by $R^6$ to $R^8$, there can be used a dihydroxy-$C_{1-6}$ alkyl group (for example, dihydroxymethyl, 2,2-dihydroxyethyl, 2,2-dihydroxypropyl, 2,2-dihydroxypentyl, 2,2-dihydroxyhexyl, etc.), preferably a dihydroxy-$C_{1-4}$ alkyl group (for example, dihydroxymethyl, 2,2-dihydroxyethyl, 2,2-dihydroxypropyl, etc.).

As the sugar residue represented by $R^6$ to $R^8$, there can be used a $C_{3-24}$ sugar residue (for example, erythrosyl, threosyl, arabinosyl, ribosyl, glucosyl, galactosyl, glycero-gluco-heptosyl, maltosyl, lactosyl, maltotriosyl, dimaltosyl, etc.), preferably a $C_{6-24}$ sugar residue (for example, glucosyl, galactosyl, glycero-gluco-heptosyl, maltosyl, lactosyl, maltotriosyl, dimaltosyl, etc.), particularly preferably a $C_{6-12}$ sugar residue (for example, glucosyl, galactosyl, glycero-gluco-heptosyl, maltosyl, lactosyl, etc.).

As the hydroxyalkyl group represented by $R^6$ to $R^8$, there can be used a hydroxy-$C_{1-6}$ alkyl group (for example, hydroxymethyl, 2-hydroxyethyl, 2-hydroxypropyl, 2-hydroxypentyl, 2-hydroxyhexyl, etc.), preferably a hydroxy-$C_{1-4}$ alkyl group (for example, hydroxymethyl, 2-hydroxyethyl, 2-hydroxypropyl, etc.), particularly preferably 2-hydroxypropyl group.

As the sulfo alkyl group represented by $R^6$ to $R^8$, there can be used a sulfo-$C_{1-6}$ alkyl group (for example, sulfomethyl, sulfoethyl, sulfopropyl, sulfopentyl, sulfohexyl, etc.), preferably a sulfo-$C_{1-4}$ alkyl group (for example, sulfomethyl, sulfoethyl, sulfopropyl, etc.), particularly preferably sulfobutyl group.

More preferred specific examples of the easily water-soluble cyclodextrin derivative include a compound represented by the general formula (II) wherein at least one of $R^6$ to $R^8$ is a sugar residue, a hydroxyalkyl group or a sulfo alkyl group.

The compound (II) wherein at least one of $R^6$ to $R^8$ is a sugar residue includes, for example, glucosyl-α,β,γ,δ-CyD, maltosyl-α,β,γ,δ-CyD, maltotriosyl-α,β,γ,δ-CyD, dimaltosyl-α, β, γ, δ-CyD, etc. Among them, maltosyl-α, β, γ, δ -CyD and glucosyl-α, β, γ, δ-CyD are preferred. Further, maltosyl-β-CyD (hereinafter abbreviated to G2-β-CyD) and glucosyl-β-CyD are particularly preferred.

The compound (II) wherein at least one of $R^6$ to $R^8$ is a hydroxyalkyl group includes, for example, hydroxypropyl-α,β,γ,δ-CyD, etc. Among them, hydroxypropyl-β-CyD is particularly preferred.

As the compound (II) wherein at least one of $R^6$ to $R^8$ is a sulfo alkyl group includes, for example, sulfobutyl-α,β,γ,δ-CyD, etc. Among them, sulfobutyl-β-CyD is particularly preferred.

Further, as the easily water-soluble cyclodextrin derivative, a branched cyclodextrin-carboxylic acid can also be used. The branched cyclodextrin-carboxylic acid includes not only free carboxylic acid thereof, but also salts with alkali metals (for example, lithium, sodium, potassium, etc.), alkaline earth metals (for example, calcium, magnesium, etc.), and the like. These branched cyclodextrin-carboxylic acids may be used alone or in combination, and may be used in the state where free carboxylic acid is mixed with its salt.

The branched cyclodextrin-carboxylic acid is a cyclodextrin which has an organic group having at least one carboxyl group at the 6-O position of at least one glucose unit of the cyclodextrin ring.

The cyclodextrin ring of the branched cyclodextrin-carboxylic acid has, for example, 6, 7 or 8 glucose units. Preferably, the cyclodextrin ring has 7 glucose units. The cyclodextrin includes, for example, α-cyclodextrin, β-cyclodextrin, γ-cyclodextrin, etc.

It is preferable that the above organic group having at least one carboxyl group has 1 to 3 glucose units, and at least one hydroxymethyl group of the glucose unit in the organic group is oxidized into a carboxyl group.

Specific examples of the branched cyclodextrin-carboxylic acid include 6-O-cyclomaltohexaosyl-(6→1)-α-D-glucosyl-(4→1)-O-α-D-glucuronic acid (cyclomaltohexaosyl-(6→1)-α-D-glucopyranosyl-(4→1)-O-α-D-glucopyranosidouronic acid) (hereinafter abbreviated to α-CyD-$G_2$-COOH, sometimes, and, likewise, abbreviations of the following compounds are described in the parenthesis), 6-O-cyclomaltoheptaosyl-(6→1)-α-D-glucosyl-(4→1)-O-α-D-glucuronic acid (cyclomaltoheptaosyl-(6→1)-O-α-D-glucopyranosyl-(4→1)-O-α-D-glucopyranosidouronic acid) (β-CyD-$G_2$-COOH), 6-O-cyclomaltooctaosyl-(6→1)-α-D-glucosyl-(4→1)-O-α-D-glucuronic acid (cyclomaltooctaosyl-(6→1)-O-α-D-glucopyranosyl-(4→1)-O-α-D-glucopyranosidouronic acid) (Y-CyD-$G_2$-COOH), 6-O-cyclomaltohexaosyl-(6→1)-α-D-glucuronic acid (cyclomaltohexaosyl-(6→1)-O-α-D-glucopyranosidouronic acid) (α-CyD-$G_1$-COOH), 6-O-cyclomaltoheptaosyl-(6→1)-α-D-glucuronic acid (cyclomaltoheptaosyl-(6→1)-O-α-D-glucopyranosidouronic acid) (β-CyD-$G_1$-COOH), 6-O-cyclomaltooctaosyl-(6→1)-α-D-glucuronic acid (cyclomaltooctaosyl-(6→1)-0-α-D-glucopyranosidouronic acid) (γ-CyD-$G_1$-COOH), 2-O- (6-cyclomaltohexaosyl)-acetic acid (α-CyD-$CH_2COOH$), 2-O- (6-cyclomaltoheptaosyl)-acetic acid (β-CyD-$CH_2COOH$), 2-O- (6-cyclomaltooctaosyl)-acetic acid (γ-CyD-$CH_2COOH$), 3-O-(6-cyclomaltoheptaosyl)-propionic acid (β-CyD-$CH_2CH_2COOH$), 2-hydroxy-3-O- (6-cyclomaltoheptaosyl)-propionic acid (3-O-(6-cyclomaltoheptaosyl)-2-hydroxy-propionic acid) (β-CyD-$CH_2CH(OH)$-COOH), 7A,7C-di-0-[α-D-glucronyl-(1→4)-O-α-D-glucosyl]-(1→6)-maltoheptaose (β-CyD-$(G_2COOH)_2$), 6-O-cyclomaltoheptaosyl-O-α-D-maltosyl-(4→1)-O-α-D-glucuronic acid (cyclomaltoheptaosyl-(6→1)-O-α-D-glucopyranosyl-(4→1)-O-α-D-glucopyranosyl-(4→1)-O-α-D-glucopyranosidouronic acid) (β-CyD-$G_3$-COOH), and the above-mentioned salts thereof [for example, sodium salt of β-CyD-$G_2$-COOH (cyclomaltoheptaosyl-(6→)-O-α-D-glucopyranosyl-(4→1)-O-α-D-glucopyranosidouronic acid sodium (similarly abbreviated to β-CyD-$G_2$-COONa). Among them, β-CyD-$G_2$-COONa is preferred.

More specifically, 6-O-cyclomaltohexaosyl-(6→1)-α-D-glucosyl-(4→1)-O-α-D-glucuronic acid (α-CyD-$G_2$-COOH), 6-0-cyclomaltoheptaosyl-(6→1)-α-D-glucosyl-(4→1)-O-α-D-glucuronic acid (β-CyD-$G_2$-COOH) and 6-O-cyclomaltooctaosyl-α-D-glucosyl-(4→1) -O-α-D-glucuronic acid (γ-CyD-$G_2$-COOH) are branched cyclodextrin-carboxylic acids having α-cyclodextrin (number of glucose units: 6), β-cyclodextrin (number of glucose units: 7) and γ-cyclodextrin (number of glucose units: 8), respectively, in which maltose is α-(1→6)-linked to one glucose unit of the cyclodextrin ring and a hydroxymethyl group at the 6-position of glucose at the end of maltose is oxidized into a carboxyl group thereby to form glucuronic acid.

Further, 6-O-cyclomaltohexaosyl-(6→1)-α-D-glucuronic acid (α-CyD-$G_1$-COOH), 6-O-cyclomaltoheptaosyl-(6→1)-α-D-glucuronic acid (β-CyD-$G_1$-COOH) and 6-O-cyclomaltooctaosyl-(6→1)-α-D-glucuronic acid (γ-CyD-$G_1$-COOH) are branched cyclodextrin-carboxylic acids, in which glucose is α-(1→6)-linked to one glucose unit of the cyclodextrin ring and a hydroxymethyl group at the 6-position of the branched glucose is oxidized into a carboxyl group thereby to form glucuronic acid.

Furthermore, 2-O-(6-cyclomaltohexaosyl)-acetic acid (α-CyD-$CH_2COOH$), 2-O-(6-cyclomaltoheptaosyl)-acetic acid (β-CyD-$CH_2COOH$), and 2-O-(6-cyclomaltooctaosyl)-acetic acid (Y-CyD-$CH_2COOH$) are branched cyclodextrin-carboxylic acids, in which a carboxymethyl group is linked by branching at one glucose unit of the cyclodextrin ring.

These branched cyclodextrin-carboxylic acid or salts thereof are described in JP 7-76594 A and JP 7-215895 A, and can be produced by the processes described in the above-mentioned publications, JP 10-210996 A, JP 10-210996 A, etc., or a method analogous thereto.

The above-mentioned stabilizers can be used alone, or preferably used in combination with those selected from hydroxypropylcellulose, polyvinyl pyrrolidone, sulfobutyl-β-CyD, G2-β-CyD, sodium deoxycholate, sodium lauryl sulfate, Polysolvate 80, polyoxyethylene (160) polyoxypropylene (30), lecithins and sucrose fatty acid esters. Among them, combinations selected from sodium deoxycholate, hydroxypropyl cellulose and polyvinyl pyrrolidone are more preferred.

It is preferable to use surfactants (especially anionic or nonionic surfactants) as the stabilizer in combination with hydrophilic polymers. Among them, a combination of hydroxypropylcellulose as the hydrophilic polymer and sodium lauryl sulfate or sucrose fatty acid ester as the surfactant is preferable and a combination of hydroxypropylcellulose and sodium lauryl sulfate or sucrose stearate ester is more preferable.

In the present invention, the above-mentioned composition of the present invention is abbreviated to a "fine particle preparation", sometimes.

The composition of the present invention may contain excipients, lubricants, binders, disintegrators, coating agents, etc., for solid preparations; and solvents, isotonizers, buffering agents, soothing agents, etc., for liquid preparations. Further, if necessary, the composition can also be contains additives for preparations such as antiseptics, antioxidants, colorants, sweeteners, etc.

Also the composition of the present invention can be formed into solid preparations or liquid preparations using the above-mentioned fine particle preparations of the present invention. The composition of the present invention may contain excipients, lubricants, binders, disintegrators, coating agents, etc., for solid preparations; and solvents, isotonizers, buffering agents, soothing agents, etc., for liquid preparations. Specific examples of these additives will be described in detail hereinafter.

The content of the HER2 inhibitor having an average particle size of about 3 μm or less in the composition of the present invention is usually within a range from 10 to 100% (w/w) based on the total weight of the preparation, while varying with particular dosage forms, administration routes, carriers, etc.

Since the composition of the present invention is superior in physical and chemical stability under storage conditions at high temperature and high humidity, the composition can maintain its properties such as drug content, particle size, crystalline state, etc., even when stored under the conditions at 40°C and RH 75% for about 3 months. Therefore, additives for stabilization of the preparation are not required and high drug content in addition to improvement in absorption can be achieved. Specifically, the pharmaceutical preparation can contain the drug in the amount within a range from 30 to 100% (w/w), preferably from 45 to 100% (w/w), and more preferably from 70 to 100% (w/w), based on the total weight of the preparation.

The content of the stabilizer in the composition of the present invention is usually within a range from 0 to 90% (w/w) based on the total weight of the preparation, while varying with particular dosage forms, administration routes, etc. For the purpose of achieving high content of the drug, the crystalline particulate preparation can contain the stabilizer in the amount within a range from 0 to 70% (w/w), preferably from 0 to 55% (w/w), and more preferably from 0 to 20% (w/w), based on the total weight of the preparation.

The content of other additives in the composition of the present invention can be usually within a range from 0 to 90% (w/w) based on the total weight of the preparation, while varying with particular dosage forms, administration routes, etc.

In case of using the fine particle preparation as a main component in the composition of the present invention, the pharmaceutical preparation of the present invention can be produced, while varying with, for example, particular dosage forms, administration routes, carriers, etc., in accordance with a conventional process by incorporating the fine particle preparation of the present invention thereinto in the amount of 0.1 to 95% (w/w) based on the total weight of the preparation.

Since the fine particle preparation of the present invention is superior in physical and chemical stability under storage conditions at high temperature and high humidity, the composition can maintain its properties such as drug content, particle size, crystalline state, etc., even when stored under the conditions at 40°C and RH75% for about 3 months. Therefore, additives for stabilization of the preparation are not required and high drug content in addition to improvement in absorption can be achieved. Specifically, the pharmaceutical preparation can contain the drug in the amount, for example, within a range from 30 to 95% (w/w), and preferably from 45 to 95% (w/w), based on the total weight of the preparation.

The content of the additives in the composition of the present invention is usually within a range from 5 to 95% (w/w) based on the total weight of the preparation, while varying with particular dosage forms, administration routes, etc.

The HER2 inhibitor having an average particle size of about 3 μm or less and the HER2 inhibitor having an average particle size of about 3 μm or less when dispersed in an aqueous solution of the present invention (hereinafter, both substances are sometimes abbreviated to the HER2 inhibitor of the present invention) as well as the composition containing the same can be prepared, for example, by comminuting the HER2 inhibitor of the present invention, if necessary, in the presence of the stabilizer. Further, in the composition of the present invention, there can be added excipients, lubricants, binders, disintegrators, coating agents, etc., for solid preparations; and solvents, isotonizers, buffering agents, soothing agents, etc., for liquid preparations during the comminution process. Further, if necessary, additives for preparations such as antiseptics, antioxidants, colorants, sweeteners, etc., can be further present during the comminution. These additives will be described in detail hereinafter.

The comminuting operation can be conducted, for example, in a solution (for example, water or an aqueous

solution containing a salt) in the state where the HER2 inhibitor is dispersed therein. Further, the composition can be prepared by using a known method, for example, the method using a grind container and a grinding ball described in Chemical Pharmaceutical Bulletin), Vol.41, pp.737-740, 1993 and the method using a high-pressure homogenizer described in International Journal of Pharmaceutics, Vol. 196, pp.161-164, 2000.

In the comminution process, for example, a medium agitating mill using a grind container and grinding medium, a container-driving medium mill, and a high-pressure homogenizer such as Micron Lab 40 (APV Homogenizer), etc., are used alone or in combination. The comminution temperature is usually within a range from 4°C to room temperature and is controlled severely, when necessary.

The comminution time varies with particular comminution conditions, kinds and presence or absence of additives and properties of drug crystals. For example, when using a grind container and grinding medium (the grinding medium is usually selected from stainless steel, zirconium oxide, agate, polystyrene, etc., and the average particle size of the medium is usually 3 mm or less), the comminution time was usually within a range from about several hours to 10 days, preferably from about 15 hours to 5 days, and more preferably from about 24 hours to 3 days. In the process using a high-pressure homogenizer, treatment is conducted about 1 to 30 times at pressure of about 200 to 2000 bar, preferably about 5 to 20 times at pressure of about 500 to 1500 bar, and more preferably about 5 to 10 times at pressure of about 1000 to 1500 bar to obtain a composition of the HER2 inhibitor of the present invention in the form of a suspension.

The concentration of the drug during comminution in a solution varies with particular properties of the drug, kinds of additives, etc., but is generally within a range from about 0.01 to 60% (w/w), preferably from about 0.1 to 60% (w/w), and more preferably from about 0.5 to 30% (w/w). Although additives other than the drug are not essential, when added, for example, additives are added in an amount within a range from about 0.1 to 300% (w/w), preferably from about 0.1 to 80% (w/w), and more preferably from about 1 to 75% (w/w), based on the drug in case of wet comminution.

More specifically, the composition can be produced by the following processes.

(1) A mixture of a HER2 inhibitor and a stabilizer is comminuted at room temperature for about 10 minutes to 10 hours, preferably about 10 minutes to 5 hours using a container-driving medium mill. The comminuted mixture is suspended in a solution such as water and then subjected to ultrasonication in ice-cooled water to obtain a dispersion of the HER2 inhibitor. Furthermore, the dispersion is treated about 1 to 20 times (preferably about 5 to 10 times) at, usually, about 100 to 3000 bar (preferably about 1000 to 2000 bar) with a high-pressure homogenizer to obtain a composition of the HER2 inhibitor of the present invention in the form of a suspension.

(2) After adding water to a mixture of the HER2 inhibitor and a stabilizer, the resultant mixture is subjected to ultrasonication in ice-cooled water to obtain a dispersion of the HER2 inhibitor. Furthermore, the dispersion is treated about 1 to 20 times (preferably about 5 to 10 times) at, usually, about 100 to 3000 bar (preferably about 1000 to 2000 bar) with a high-pressure homogenizer to obtain a composition of the HER2 inhibitor of the present invention in the form of a suspension.

(3) After adding water to a mixture of the HER2 inhibitor and a stabilizer, the resultant mixture is subjected to ultrasonication in ice-cooled to obtain a dispersion of the HER2 inhibitor. A stainless steel grinding medium are added to the resultant dispersion, followed by shaking at room temperature for, usually, about one hour to 10 days to obtain a composition of the HER2 inhibitor of the present invention in the form of a suspension.

(4) After adding water to a mixture of the HER2 inhibitor and a stabilizer, the resultant mixture is subjected to ultrasonication in ice-cooled water to obtain a dispersion of the HER2 inhibitor. A stainless steel grinding medium are added to the resultant dispersion, followed by shaking at room temperature for, usually, about one hour to 10 days. After shaking, the dispersion is treated about 1 to 20 times (preferably about 5 to 10 times) at, usually, about 100 to 3000 bar (preferably about 1000 to 2000 bar) with a high-pressure homogenizer to obtain a composition of the HER2 inhibitor of the present invention in the form of a suspension.

The composition of the HER2 inhibitor of the present invention in the form of a suspension obtained by the above-mentioned method can be reduced to powder by a method generally used in the production of preparations. For reducing to powder, for example, there can be used a means such as a spray drier (for example, GS-31R, manufactured by Yamato Scientific Co. Ltd.), freeze-drying, spray granulation or spray coating on nonpareil particles. If necessary, the resulting particles can be further vacuum-dried. Further, if necessary, when the composition in the form of a suspension is reduced to powder, various pharmaceutically available additives for preventing agglomeration and improving stability can be added thereto. Examples of the additives to be added for preventing agglomeration and improving stability include sugars and sugar alcohols such as mannitol, trehalose, etc.; and coating agents such as hydroxypropylcellulose, etc. Surfactants such as sugar esters, sodium lauryl sulfate can be added as an anticoagulant.

The composition of the present invention can be prepared by a nano-precipitation method, and can also be obtained by a known method, for example, by dissolving the HER2 inhibitor of the present invention in a solvent, which can be removed by volatilization, such as methylene chloride, etc., and recrystallizing in a solution (for example, aqueous solution) while controlling a crystal deposition rate. Furthermore, it is possible to mix a base such as a polymer with the HER2 inhibitor (in this case, the HER2 inhibitor may be in either of crystalline and amorphous states, or a mixed state of crystals and an amorphous material), followed by treating the mixture with solvent vapor, etc., to obtain a dispersion comprising a base and a crystalline particulate dispersed in the base.

Furthermore, the composition of the present invention may contain excipients, lubricants, binders, disintegrators, coating agents, etc., for solid preparations; and solvents, isotonizers, buffering agents, soothing agents, etc., for liquid preparations during the crystallization process. If necessary, it is also possible to include additives for preparations such as antiseptics, antioxidants, colorants, sweeteners, etc., during the crystallization process. Specific examples of these additives will be described in detail hereinafter.

According to the nano-precipitation method, the composition of the present invention can be obtained by dispersing a solvent in which the HER2 inhibitor has been dissolved in water (or an aqueous solution), if necessary, in the presence of a stabilizer and removing the solvent. The solvent, which can be removed by volatilization, to be used in the preparation is not specifically limited as long as it can dissolve the HER2 inhibitor, and is preferably a water immiscible solvent such as methylene chloride, ethyl acetate, etc., or a water miscible solvent such as acetone, methanol, ethanol, tetrahydrofuran, etc. The solvent in which the HER2 inhibitor has been dissolved is added dropwise in water or an aqueous solution containing a salt, if necessary, in the presence of the stabilizer, followed by stirring with a stirrer such as magnetic stirrer, Ultra-Turrax T-25 (Ika), etc., an ultrasonic mill VP-25 (TITEC), a high-pressure homogenizer Micron Lab 40 (APV Homogenizer), etc. The solvent used as that for the drug can be removed at reduced pressure or by heating and, for example, a rotary evaporator, etc., can be used. Also a crystalline particulate can be obtained as a dried powder by removing the solvent using a spray drier or a freeze-dryer. Furthermore, the solvent can be removed by employing a carbon dioxide gas in a critical state.

The composition of the present invention is previously mixed with additives such as a polymer as a base, etc., and the drug and the resultant mixture is treated with solvent vapor, thereby making it possible to obtain a dispersion of a crystalline particulate. The HER2 inhibitor in the state of a mixture with additives may be in an either of crystalline and amorphous states, or a mixed state of crystals and an amorphous material.

The additives serving as the base may be stabilizers such as hydrophilic polymers, surfactants, easily water-soluble cyclodextrin derivatives, or may be the above-mentioned excipients, lubricants, binders, disintegrators, coating agents, isotonizers, buffering agents, soothing agents, antiseptics, antioxidants, colorants, sweeteners, etc.

The pharmaceutical composition of the present invention can be produced by a comminution operation using a general medium mill or a high-pressure homogenizer, or a nano-precipitation operation such as recrystallization with controlling a crystal deposition rate. More preferably, the pharmaceutical composition can be prepared by using a compaction shear mill such as Micros (manufactured by Nara Machinery Co., Ltd.). This kind of a pulverizer is a mill wherein particles of a sample substance are interposed between plural rollers (or rings for comminution) rotating at a high speed and an inner wall of the mill and then comminution is effected by compression and shear action, and also the comminution efficiency is enhanced by increasing the contact area between rollers (or rings for comminution) rotating at a high speed and the mill inner wall.

In the production of the crystalline particlulate by the nano-precipitation operation, a solvent, which is harmful to a human body, is generally used and such a harmful solvent is likely to be remained in the fine particles thus produced. Further, there are such problems that it is difficult to control crystal deposition and to store fine particles for a long period of time. Then, for simplification and convenience, the production is carried out by comminution with a medium mill or a high-pressure homogenizer.

In case of a general medium mill, particles are broken by impact or shear force when the medium comes into collision with particles to be comminuted. To enhance the comminution efficiency, it is required to move a smaller medium having higher density at a higher speed. However, it is assumed that isolation of the medium from a sample and an operation for washing the medium become more complicated by using a smaller medium. Further, a long comminution period is required sometimes (U.S. Patent No. 5,145,684, 1992) and attention must be paid on problems such as generation of microorganisms, etc. On the other hand, a comminution using a high-pressure homogenizer has such an advantage that contamination of foreign matters into a sample can be suppressed, while, in case of comminution of a hard crystalline particulate, satisfactory comminution can not be expected by using a high-pressure homogenizer alone and plural times of comminution treatment (number of passing) are required and this is not suitable for practical use. (U.S. Patent No. 6,177,103, 2001).

In case of a compaction shear mill, large compression and shear force is applied to particles to be comminuted by a centrifugal force produced by plural rollers (or rings for comminution) which revolves and rotates at a high speed. The particles to be comminuted are "ground" by large compression force, thereby making it possible to achieve submicron fine particles using a compaction shear mill with reduced treatment time such as 1/6 to 1/30 of that of a conventional mill.

In case of a wet comminution using a compaction shear mill, in comparison with a comminution using a medium mill capable of mechanically fluidizing a grinding medium such as balls and beads, or using a high-pressure

homogenizer, the particles can be comminuted into a high concentration suspension (slurry) within relatively short time and a crystalline particulate having an average particle size of 1 µm or less can be efficiently obtained. Furthermore, the disassembling and cleaning operation is easily conducted because of the structure of the mill and generation of microorganisms are less likely.

Since the HER2 inhibitor (for example, the compound (I), or a salt thereof or a prodrug thereof) has a tyrosine kinase inhibiting activity, the HER2 inhibitor of the present invention or the composition thereof can be used for the prevention or treatment of tyrosine kinase dependent diseases in mammals. These tyrosine kinase dependent diseases include diseases stimulating cell proliferation due to abnormal tyrosine kinase activity. Furthermore, since the HER2 inhibitor specifically inhibits HER2 tyrosine kinase, the HER2 inhibitor of the present invention or the composition thereof is useful as an agent for treating cancer expressing HER2 by inhibiting the growth thereof, or an agent for preventing transition of hormone dependent cancer to hormone independent cancer.

In other words, the HER2 inhibitor of the present invention or the composition thereof can be safely used as an agent for preventing or treating diseases caused by abnormal cell proliferation including various cancers (especially breast cancer, prostatic cancer, pancreactic cancer, stomach cancer), atherosclerosis, angiogenesis (for example, angiogenesis accompanying the growth of solid tumor or sarcoma, angiogenesis accompanying metastatis of tumors, angiogenesis accompanying diabetic retinopathy, etc.), viral diseases (for example, HIV infection, etc.), and the like.

The tyrosine kinase dependent diseases further include cardiovascular diseases relating to abnormal tyrosine kinase activity. Therefore, the crystalline particulate preparation of the present invention can also be used as an agent for preventing or treating cardiovascular diseases such as restenosis.

The HER2 inhibitor or the composition thereof of the present invention is useful as an anticancer agent for preventing or treating cancers, for example, breast cancer, prostatic cancer, pancreactic cancer, stomach cancer, etc.

The HER2 inhibitor or the composition thereof of the present invention has low toxicity and can be used as such or as a pharmaceutical composition prepared by mixing with a per se known pharmaceutically acceptable carrier or the like for mammals (for example, human, horse, cow, dog, cat, rat, mouse, rabbit, pig, monkey, etc.).

Further, any other active component, for example, the following hormone therapeutic agents, chemotherapeutic agents and immunotherapeutic agents may be included in the composition of the present invention together with the HER2 inhibitor of the present invention.

Upon administration of the HER2 inhibitor or the composition thereof of the present invention to a mammal such as a human, etc., it can be administered orally or parenterally in the form of solid preparations such as tablets, capsules (including soft capsules and microcapsules), powders, granules, suppositories, pellets, etc.; or in the form of liquid preparations such as suspensions, syrups, injectable preparations, etc.; by mixing with pharmaceutically acceptable carriers.

As the pharmaceutically acceptable carriers, there can be used conventional organic or inorganic carriers for preparations, and they can be formulated as excipients, lubricants, binders, disintegrators, coating agents for solid preparations; and solvents, isotonizers, buffering agents and local anesthetic agents for liquid preparations; and the like. Further, if necessary, there can be used additives for preparations as antiseptics, antioxidants, colorants, sweeteners, and the like.

Preferred examples of excipients include lactose, sucrose, D-mannitol, starch, crystalline cellulose, light silicon dioxide, etc.

Preferred examples of lubricants include magnesium stearate, calcium stearate, talc, colloid silica, etc.

Preferred examples of binders include crystalline cellulose, sucrose, D-mannitol, dextrin, hydroxypropylcellulose, hydroxypropylmethylcellulose, polyvinyl pyrrolidine, etc.

Preferred examples of disintegrators include starch, carboxymethylcellulose, carboxymethylcellulose calcium, croscarmellose sodium, carboxymethyl starch sodium, etc.

As preferable examples of coating agents, hydrophilic high molecular weight polymers, etc., are commonly used. Among these, water-soluble polymers such as cellulose derivatives, for example, the above-mentioned hydroxyalkyl cellulose, alkyl cellulose, etc., enteric polymers, stomach soluble polymers, etc., are preferably used. Further, these hydrophilic polymers may be used in combination of two or more kinds thereof.

Examples of the enteric polymers include hydroxyalkylcellulose phthalates such as hydroxypropylmethylcellulose phthalate, etc.; hydroxyalkylcellulose acetate succinates such as hydroxypropylmethylcellulose acetate succinate, etc.; carboxyalkylcelluloses such as carboxymethylethylcellulose, etc.; cellulose acetate phthalates; copolymers of ethyl acrylate and methacrylic acid such as methacrylate copolymer L-100-55, etc.; copolymers of methyl methacrylate and methacrylic acid such as methacrylate copolymer L, methacrylate copolymer S, etc.; and the like.

Examples of usable stomach soluble polymers include amino alkyl methacrylate copolymer E; polyvinyl acetal diethylaminoacetate; etc.

In addition, there can be used copolymers having a small amount of a quaternary ammonium group of ethyl acrylate and methyl methacrylate such as methacrylate copolymer RL, methacrylate copolymer RS, etc.; and hydrophilic polymers such as carboxymethylcellulose, carboxyvinyl polymer, polyvinyl alcohol, gum arabic, sodium alginate, propylene glycol alginate, agar, gelatin, chitosan, etc.

Preferred examples of solvents include ethyl alcohol, propylene glycol, macrogol, sesame oil, corn oil, etc.

Preferred examples of isotonizers include sodium chloride, glycerin, D-mannitol, etc.

Preferred examples of buffering agents include buffer solutions such as phosphates, acetates, carbonates, citrates, etc.

Preferred examples of soothing agents include benzyl alcohol, etc.

Preferred examples of antiseptics include para-hydroxybenzoic acid esters, chlorobutanol, benzyl alcohol, phenethyl alcohol, dehydroacetic acid, sorbic acid, etc.

Preferred examples of antioxidants include sulfites, ascorbic acid, etc.

The dosage of the HER2 inhibitor or the composition thereof of the present invention for a patient (40 to 80 kg body weight) with breast cancer or prostatic cancer ranges, while varying with particular administration routes, symptoms or the like, in the case of oral administration, preferably from 0.5 to 100 mg/kg, more preferably from 1 to 50 mg/kg per day, and most preferably from 1 to 25 mg/kg per day. This dosage can be administered once daily or dividing into two to three times a day.

Further, the composition of the present invention can be administered to the same subject simultaneously with any other agent for hormone therapy, chemotherapy, immunotherapy, a cell growth factor and a drug capable of inhibiting the action of its receptor, or the like, or it can be administered to the same subject with a time lag.

Examples of the hormone therapeutic agents include fosfestrol, diethylstilbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, antiestrogens (for example, tamoxifen citrate, toremifene citrate, etc.), pill preparation, mepitiostane, testolactone, aminoglutethimide, LH-RH agonist (for example, goserelin acetate, buserelin, leuprorelin, etc.), droloxifene, epitiostanol, thinylestradiol sulfonate, aromatase inhibitors (for example, fadrozole hydrochloride, anastrozole, letrozole, exemestane, vorozole, formestane, etc.), anti-androgens (for example, flutamide, bicalutamide, nilutamide, etc.), 5α-reductase inhibitors (for example, finasteride, epristeride, etc.), adrenocortical hormones (for example, dexamethasone, prednisolone, betamethasone, triamcinolone, etc.), androgen synthesis inhibitors (for example, abiraterone, etc.), retinoid and suppressing agents of retinoid metabolism (for example, liarozole, etc.), and the like.

Examples of the chemotherapeutic agents include alkylating agent, metabolic antagonists, antitumor antibiotics, antineoplastic agents derived from plants, and the like.

Examples of the alkylating agents include nitrogen mustard, nitrogen mustard N-oxide hydrochloride, chlorambucil, cyclophosphamide, ifosfamide, thiotepa, carboquone, improsulphan tosilate, busulfan, nimustine hydrochloride, mitobronitol, melphalan, dacarbazine, ranimustine, estramustine phosphate sodium, triethylenemelamine, carmustine, lomustine, streptozocin, pipobroman, ethoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulfan, trofosfamide, zinostatin stimalamer, carboquone, adozelesin, cystemustine, bizelesin, etc.

Examples of the antimetabolites include mercaptopurine, 6-mercaptopurine riboside, thioinosine, methotrexate, enocitabine, cytarabine, cytarabine ocfosfate, ancitabine hydrochloride, 5-FU analogues (for example, fluorouracil, tegafur, UFT, doxifluridine, carmofur, gallocitabine, emitefur, etc.), aminopterin, leucovorin calcium, tabloid, butocin, calcium folinate, calcium levofolinate, cladribine, emitefur, fludarabine, gemcitabine, hydroxycarbamide, pentostatin, piritorexim, idoxuridine, mitoguazone, tiazofurin, ambamustine, etc.

Example of antitumor antibiotics include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride, idarubicin hydrochloride, etc.

Examples of the antineoplastic agents derived from plants include etoposide, etoposide phosphate, vinblastine sulfate, vincristine sulfate, vindesine sulfate, teniposide, paclitaxel, docetaxel, vinorelbine, etc.

Examples of the immunotherapies (BRM) include picibanil, krestin, sizofiran, lentinan, ubenimex, interferons, interleukins, macrophage-colony stimulating factor, granules stimulating factor of spheroid colony, erythropoietin, lymphotoxin, BCG vaccine, corynebacterium parvum, levamisole, polysaccharide-K, procodazol, etc.

The "cell growth factor" in the "cell growth factor and the drug capable of inhibiting an action of its receptor" may be any substance as long as it is a substance capable of accelerating the growth of cells, and is usually a factor which is a peptide having a molecular weight of 20,000 or less and is capable of exerting an action at low concentration as a result of the binding to a receptor. Specific examples thereof include (1) EGF (epidermal growth factor) or substance having substantially the same activity [for example, EGF, heregulin (HER2 ligand), etc.], (2) insulin or substance having substantially the same activity [for example, insulin, IGF (insulin-like growth factor)-1, IGF-2, etc.], (3) FGF (fibroblast growth factor) or substance having substantially the same activity [for example, acidic FGF, basic FGF, KGF (keratinocyte growth factor), FGF-10, etc.], (4) other cell growth factors [for example, CSF (colony stimulating factor), EPO (erythropoietin), IL-2 (interleukin-2), NGF (nerve growth factor), PDGF (platelet-derived growth factor), TGF β (transforming growth factor β), HGF (hepatocyte growth factor) and VEGF (vascular endothelial growth factor), etc.], and the like.

The "receptor of cell growth factor" may be any one as long as it is a receptor capable of binding to the above cell growth factor, and specific examples thereof include EGF receptor, heregulin receptor (HER2), insulin receptor-1, insulin receptor-2, IGF receptor, FGF receptor-1, FGF receptor-2, etc.

Examples of the "drug capable of inhibiting the action of the cell growth factor" include herceptin (HER2 receptor antibody), etc.

Others include L-asparaginase, aceglatone, procarbazine hydrochloride, protoporphyrin cobalt complex, mercury hematoporphyrin sodium, topoisomerase 1I inhibitors (for example, irinotecan, topotecan, etc.), topoisomerase II inhibitors (for example, sobuzoxane, etc.), differentiated induction drugs (for example, retinoid, vitamin D, etc.), angiogenesis inhibitors, α-broker (for example, tamsulosin hydrochloride, etc.), and the like.

The amount of these other drugs contained in the pharmaceutical composition of the present invention varies according to particular dosage forms, administration routes, carriers, etc., but is usually from 0.1 to 95% (w/w) based on the total weight of the preparation.

The present invention will be illustrated in further detail by the following Reference Examples, Examples and Test Examples, which are not intended to limit the present invention.

In the column chromatography in Reference Examples, elution was carried out with monitoring by TLC (Thin Layer Chromatography) . In the TLC monitoring, $60F_{254}$ (70 to 230 mesh) plates manufactured by Merck & Co., Inc. were used as the TLC plate and the same solvent as that used for elution in the column chromatography was used as the developing solvent, and the detection was conducted with a UV detector. The silica gel for the column was Kieselguhr $60F_{254}$ (70 to 230 mesh) manufactured by Merck & Co. Inc. NMR spectra show proton NMR and were measured using tetramethylsilane as the internal standard with VARIAN Gemini-200 (270 MHz type spectrometer). All δ values were expressed in ppm.

The abbreviations used in Reference Examples have the following meanings:

| | |
|---|---|
| s: | singlet, |
| br: | broad, |
| d: | doublet, |
| t: | triplet, |
| q: | quartet, |
| dd: | double doublet, |
| dt: | double triplet, |
| m: | multiplet, |
| J: | coupling constant, |
| Hz: | Hertz, |
| DMF: | N,N-dimethylformamide, |
| THF: | tetrahydrofuran. |

Reference Example A1

4-Chloromethyl-2-[(E)-2-(4-methylphenyl)ethenyl]-1,3- 3-oxazole

(i) (E)-3-(4-Methylphenyl)-2-propenamide

To a THF (100 mL) solution of 4-methylcinnamic acid (15.19 g), DMF (5 drops) was added and oxalyl chloride (9.6 mL) was further added thereto with ice-cooling, followed by stirring at room temperature for 2 hours. Oxalyl chloride (4.0 mL) was further added and, after stirring at room temperature for 1 hour, the mixture was concentrated to dryness. The residue was dissolved in ethyl acetate (50 mL) and the solution was added dropwise in a mixture of 25% ammonia water (50 mL) and ethyl acetate (20 mL) with ice-cooling. The aqueous layer was salted out and the organic layer was extracted with ethyl acetate. The extract was dried over magnesium sulfate and then concentrated under reduced pressure. The deposit was washed with hexane and diethyl ether to obtain the title compound (11.63 g) as colorless crystals.

$^1$H-NMR ($CDCl_3$) δ: 2.37 (3H, s), 5.56 (2H, brs), 6.41 (1H, d, J = 15.8), 7.18 (2H, d, J = 8.0), 7.42 (2H, d, J = 8.0), 7.62 (1H, d, J = 15.8).

IR (KBr): 1671, 1601, 1518, 1397, 1254, 1123, 990, 816 $cm^{-1}$.

(ii) 4-Chloromethyl-2-[(E)-2-(4-methylphenyl)ethenyl]-1,3-oxazole

(E)-3-(4-Methylphenyl)-2-propenamide (8.06 g) and 1,3-dichloroacetone (6.98 g) were refluxed in toluene (50 mL) for 3 hours. After cooling, the reaction mixture was diluted with ethyl acetate, washed with water and brine, dried over magnesium sulfate and then concentrated under reduced pressure. The residue was purified by subjecting to silica gel chromatography (eluent; ethyl acetate:hexane = 1:4) to obtain the title compound (8.44 g) as a white crystalline powder.
$^1$H-NMR ($CDCl_3$) δ: 2.38 (3H, s), 4.54 (2H, s), 6.87 (1H, d, J = 16.2), 7.20 (2H, d, J = 8.2), 7.43 (2H, d, J = 8.2), 7.52 (1H, d, J = 16.2), 7.62 (1H, s).
IR (KBr): 1642, 1607, 1591, 1537, 1345, 1267, 976, 943, 810cm$^{-1}$.

Reference Example A2

4-Chloromethyl-2-[(E)-2-(4-fluorophenyl)ethenyl]-1,3-oxazole

4-Fluorocinnamic acid (25 g) was suspended in dichloromethane (300 mL), and DMF (0.5 mL) and then oxalyl chloride (15.36 mL) were added dropwise with ice-cooling and stirring, and the mixture was maintained at the same temperature for 3 hours and gradually returned to room temperature. Under reduced pressure, the solvent was distilled off and the residue was dissolved in ethyl acetate (100 mL). The resulting solution was added dropwise in a ice-cooled mixture of 25% ammonia water (250 mL) and ethyl acetate (52.5 mL). The reaction mixture was extracted with ethyl acetate (400 mL x 2), washed with saturated brine and then dried over anhydrous magnesium sulfate. Under reduced pressure, the solvent was distilled off and the deposited crystals were collected by filtration and then dried to obtain (E)-3-(4-fluorophenyl)-2-propenamide (24.4 g).

The resulting (E)-3-(4-fluorophenyl)-2-propenamide (17.55 g) and 1,3-dichloroacetone (12.85 g) were melted at 130°C and then stirred for 1.5 hours. After returning to room temperature, the solution was extracted with ethyl acetate and then washed in turn with ice-water, aqueous saturated sodium bicarbonate and saturated brine. After drying over anhydrous sodium sulfate, the solvent was distilled off and then purified by subjecting to column chromatography (eluent; diethyl ether:hexane = 1:9 → 3:17) to obtain the title compound (10.5 g) as colorless crystals.
$^1$H-NMR ($CDCl_3$) δ: 4.54 (2H, s), 6.84 (1H, d, J = 16.0Hz), 7.09 (2H, t, J = 8.8Hz), 7.47-7.55 (3H, m), 7.63 (1H, s).
IR (KBr): 3173, 3133, 3063, 3040, 1645, 1601, 1591, 1537, 1508, 1435, 1416, 1350, 1275, 1233, 1167, 1101, 999 cm$^{-1}$.

Reference Example A3

4-Chloromethyl-2-[(E)-2-(4-trifluoromethylphenyl)ethenyl]-1,3-oxazole

(i) (E)-3-(4-Trifluoromethylphenyl)-2-propenamide

To a THF (100 mL) suspension of 4-trifluoromethylcinnamic acid (19.4 g) and DMF (6 droplets), oxalyl chloride (11.7 mL) was added dropwise at 0°C, followed by stirring at room temperature for 2 hours. After the solvent was distilled off under reduced pressure, the residue was dissolved in ethyl acetate (60 mL) and the solution was poured into 25% ammonia water-ethyl acetate (5:1., 120 mL). After the aqueous layer was salted out, the organic layer was extracted with a mixture (650 mL) of ethyl acetate and THF (12:1), and ethyl acetate (100 mL x 2), and then dried over anhydrous magnesium sulfate. After the solvent was distilled off under reduced pressure, the residue was recrystallized from ethyl acetate-hexane to obtain the title compound (18.0 g) as colorless plates.
$^1$H-NMR ($CDCl_3$) δ: 5.58 (2H, br s), 6.53 (1H, d, J = 15.8 Hz), 7.63-7.72 (5H, m).
IR (KBr): 3326, 3167, 1686, 1636, 1617, 1404, 1190 cm$^{-1}$.

(ii) 4-Chloromethyl-2-[(E)-2-(4-trifluoromethylphenyl)ethenyl]-1,3-oxazole

A toluene solution (83 mL) of (E)-3-(4-trifluoromethylphenyl)-2-propenamide (17.9 g) and 1,3-dichloroacetone (14.8 g) was heated at reflux for 9 hours using a Dean-Stark apparatus. After cooling, water was added to the reaction mixture and the mixture was extracted with ethyl acetate, washed with saturated brine and then dried over anhydrous magnesium sulfate. After the solvent was distilled off under reduced pressure, the residue was purified by subjecting to silica gel column chromatography (eluent; hexane:methyl acetate = 6:1 → 5:1) to obtain the title compound (15.1 g) as colorless needles.
$^1$H-NMR ($CDCl_3$) δ: 4.55 (2H, d, J = 0.8 Hz), 7.00 (1H, d, J = 16.2 Hz), 7.56 (1H, d, J = 16.2 Hz), 7.64-7.68 (5H, m).
IR (KBr): 1350, 1325, 1170, 1136, 1113, 1071, 959, 826, 727, 708 cm$^{-1}$.

Reference Example A4

4-Chloromethyl-2-[(E)-2-(2,4-difluorophenyl)ethenyl]-1,3-oxazole

Using (E)-3-(2,4-difluorophenyl)-2-propenamide (9.16 g) and 1,3-dichloroacetone (7.62 g), the same reaction as in Reference Example A1-(ii) was conducted to obtain the title compound (6.31 g) as colorless crystals.
$^1$H-NMR ($CDCl_3$) δ: 4.55 (2H, s), 6.8-7.0 (2H, m), 6.96 (1H, d, J = 16.8), 7.45-7.7 (3H, m).

Reference Example A5

4-Chloromethyl-2-[(E)-2-(2,6-difluorophenyl)ethenyl]-1,3-oxazole

Using (E)-(2,6-difluorophenyl)-2-propenamide (9.0 g) and 1,3-dichloroacetone (7.49 g), the same reaction as in Reference Example A1-(ii) was conducted to obtain the title compound (7.18 g) as a pale yellow solid.
$^1$H-NMR ($CDCl_3$) δ: 4.55 (2H, s), 6.85-7.0 (2H, m), 7.2-7.35 (2H, m), 7.55-7.7 (1H, m), 7.66 (1H, s).

Reference Example A6

3- (1H-Imidazol-2-yl) -1, 2-propanediol

3,4-Dihydroxybutyronitrile (30.33 g) was dissolved in anhydrous methanol (12.2 mL) and then a 5.12 N hydrogen chloride-ether solution (62 mL) was added at 5°C or lower with ice-cooling and stirring. When the mixture was stirred at the same temperature for 35 hours, a two-layered solution was obtained. The upper layer was removed and the lower layer was dissolved in anhydrous methanol (45 mL). An anhydrous ethanol (45 mL) solution of aminoacetoaldehyde methylacetal (31.5 g) was added thereto at 20°C or lower with ice-cooling and stirring, followed by stirring for 27 hours. Under reduced pressure, the solvent was distilled off and water (57 mL) and concentrated hydrochloric acid (142 mL) were added to the residue, followed by stirring at room temperature for 2 hours. Under reduced pressure, the solvent was distilled off and aqueous potassium carbonate solution was added to the residue, thereby to adjust the pH to 10, and the solvent was distilled off again. The residue was extracted with ethanol (500 mL) and then concentrated to dryness. The residue was purified by subjecting to silica gel column chromatography and then to a desalting treatment using an ion-exchange resin (Amberlyst 15) to obtain the title compound (13.16 g) as pale brown crystals.
mp 98-100°C.
$^1$H-NMR (DMSO-$d_6$) δ: 2.60 (1H, dd, J= 7.6Hz, 14.8Hz), 2.80 (1H, dd, J = 5.0Hz, 14.8Hz), 3.28 (1H, dd, J = 5.6Hz, 10.2Hz), 3.35 (1H/ dd, J = 5.4Hz, 10.2Hz), 3.72-3.85 (1H, m), 6.88 (2H, s).
IR (KBr): 3167, 3094, 2928, 2656, 1559, 1456, 1416, 1379, 1327, 1291, 1275, 1242, 1202, 1152, 1111, 1092, 1044 $cm^{-1}$.

Reference Example A7

(2R)-3- (1H-Imidazol-2-yl)-1,2-propanediol

(i) (2R)-1-(Benzyloxy)-3-(1-trityl-1H-imidazol-2-yl)-2-propanol

To a THF solution (80 mL) of 1-tritylimidazol (3.10 g), n-butyllithium (1.6M hexane solution, 6.9 ml) was added dropwise in an argon atmosphere with ice-cooling. After stirring at the same temperature for 30 minutes, (R)-2-[(benzyloxy)methyl]oxirane (1.52 mL) was added thereto. After stirring for 1.5 hours with ice-cooling and stirring at room temperature for 1 hour, water was added to the reaction mixture and the mixture was extracted with ethyl acetate. The extract was washed with water and brine, dried over magnesium sulfate and then concentrated under reduced pressure. The residue was purified by subjecting to silica gel chromatography (eluent; ethyl acetate:hexane = 1:1) to obtain the title compound (1.402 g) as a pale yellow oily product.
$^1$H-NMR ($CDCl_3$) δ: 2.06 (2H, dd, J = 2.8Hz, 18.0Hz), 3.08 (1H, dd, J = 5.4Hz, 9.8Hz), 3.21 (1H, dd, J = 5.4Hz, 9.8Hz), 3.55-3.7 (1H, m), 4.36 (2H, s), 6.73 (1H, d, J = 1.4Hz), 6.93 (1H, d, J = 1.4Hz), 7.0-7.4 (20H, m).

(ii) (2R)-1-(Benzyloxy)-3-(1H-imidazol-2-yl)-2-propanol

To an acetone (8 mL) solution of (2R)-1-(benzyloxy)-3-(1-trityl-1H-imidazol-2-yl)-2-propanol (1.40 g), 1 N hydrochloric acid (8 mL) was added, followed by stirring at 50°C for 1 hour. 1 N Hydrochloric acid (8 mL) was added thereto, followed by further stirring at 50°C for 2 hours. The reaction mixture was concentrated, water was added thereto, and the mixture was washed twice with diethyl ether. After the aqueous layer was neutralized with aqueous

sodium bicarbonate, the organic layer was extracted with ethyl acetate, washed with brine, dried over magnesium sulfate and then concentrated under reduced pressure. The residue was purified by subjecting to silica gel column chromatography (eluent; ethyl acetate:methanol = 10:1) to obtain the title compound (424 mg) as a colorless oily product.
$^1$H-NMR ($CDCl_3$) δ: 2.85 (1H, dd, J = 7.8Hz, 15.6Hz), 2.99 (1H, dd, J = 3.6Hz, 15.6Hz), 3.39 (1H, dd, J = 7.0Hz, 9.5Hz), 3.52 (1H, dd, J = 4.4Hz, 9.5Hz), 4.1-4.3 (1H, m), 4.55 (2H, s), 6.94 (2H, s), 7.3-7.45 (5H, m).

(iii) (2R)-3-(1H-Imidazol-2-yl)-1,2-propanediol

To a methanol (10 mL) solution of (2R)-1-(benzyloxy)-3-(1H-imidazol-2-yl)-2-propanol(424 mg), 10% palladium-carbon (50% moisture, 85 mg) was added, followed by stirring in a hydrogen atmosphere at 50-60°C for 2 days. After the catalyst was removed by filtration, the filtrate was concentrated to obtain the title compound (254 mg) as a white solid.
$^1$H-NMR ($CDCl_3$) δ: 2.58 (1H, dd, J = 7.6Hz, 14.6Hz), 2.78 (1H, dd, J = 5.2Hz, 14.6Hz), 3.17 (1H, d, J = 5.2Hz), 3.2-3.3 (1H, m), 3.7-3.85 (1H, m), 4.6-4.7 (1H, m), 4.86 (1H, d, J = 4.8Hz), 6.76 (1H, brs), 6.95 (1H, brs).
$[a]_D^{22}$ = + 2.5°(C = 1.0, methanol)

Reference Example A8

(2S) -3-(1H-Imidazol-2-yl)-1,2-propanediol

(i) (3S)-4-(Benzyloxy)-3-(trimethylsilyloxy)butyronitrile

To a mixture of (2S)-2-[(benzyloxy)methyl]oxirane (6.57 g) and trimethylsilanecarbonitrile (5.0 g), potassium cyanate (26 mg) and 18-crown-6 (106 mg) were added and the mixture was refluxed in an argon atmosphere at 135°C for 75 minutes. After cooling, the reaction mixture was distilled under reduced pressure to obtain the title compound (7.42 g).
$^1$H-NMR ($CDCl_3$) δ: 0.15 (9H, s), 2.52 (1H, dd, J = 6.6Hz, 16.6Hz), 2.65 (1H, dd, J = 4.6Hz, 16.6Hz), 3.39 (1H, dd, J = 6.8Hz, 9.6Hz), 3.50 (1H, dd, J = 4.8Hz, 9.6Hz), 4.01-4.14 (1H, m), 4.52 (2H, s), 7.26-7.44 (5H, m).
IR (neat): 3065, 3032, 2957, 2903, 2865, 2251, 1607, 1588, 1497, 1454, 1416, 1366, 1254, 1209, 1117, 1001 $cm^{-1}$.

(ii) (3S)-4-(Benzyloxy)-3-hydroxybutyronitrile

(3S)-4-(Benzyloxy)-3-[(trimethylsilyl)oxy]butyronitrile (7.41 g) was dissolved in tetrahydrofuran (28.2 mL) and a 1 M tetrabutylammonium fluoride THF solution (28.2 mL) was added, followed by stirring for 1.5 hours. Under reduced pressure, the solvent was distilled off and the residue was dissolved in ether and then washed with water and saturated brine. Under reduced pressure, the solvent was distilled off and the residue was purified by subjecting to silica gel column chromatography to obtain the title compound (4.58 g) as a colorless oily product.
$^1$H-NMR (DMSO-$d_6$) δ: 2.56 (1H, dd, J = 6.4Hz, 16.8Hz), 2.70 (1H, dd, J = 4.6Hz, 16.8Hz), 3.34 (1H, dd, J = 6.2Hz, 9.8Hz), 3.44 (1H, dd, J = 5.4Hz, 9.8Hz), 3.85-3.95 (1H, m), 5.52 (2H, d, J = 5.2Hz), 7.25-7.40 (5H, m).
IR (neat): 3600-3200, 3065, 3032, 2867, 2253, 1605, 1586, 1497, 1454, 1416, 1364, 1308, 1254, 1208, 1101, 1078 $cm^{-1}$.

(iii) (2S)-1-(Benzyloxy)-3- (1H-imidazol-2-yl)-2-propanol

Using (3S)-4-(benzyloxy)-3-hydroxybutyronitrile (6.51 g), 5.12 N hydrogen chloride-ether solution (7.0 mL) and aminoacetoaldehyde dimethyl acetal (3.58 g), the same reaction as in Reference Example 6 was conducted to obtain the title compound (2.22 g) as a pale brown oily product.
$^1$H-NMR ($CDCl_3$) δ: 2.84 (1H, dd, J = 7.8Hz, 15.4Hz), 2.97 (1H, dd, J = 3.6Hz, 15.4Hz), 3.41 (1H, dd, J = 6.8Hz, 9.4Hz), 3.51 (1H, dd, J = 4.4Hz, 9.4Hz), 4.11-4.23 (1H, m), 4.54 (2H, s), 6.91 (2H, s), 7.27 (5H, m).
IR (neat): 3400-3140, 3065, 3032, 2903, 2865, 1601, 1557, 1495, 1454, 1427, 1366, 1312, 1206, 1101, 1028 $cm^{-1}$.
$[a]_D^{22}$ = - 2.3°(C = 1.04, methanol)

(iv) (2S)-3-(1H-Imidazol-2-yl)-1,2-propanediol

(2S)-1-(Benzyloxy)-3- (1H-imidazol-2-yl)-2-propanol (1.725 g) was dissolved in ethanol (30 mL) and 10% palladium-carbon (1.04 g) was added, followed by vigorous stirring in a hydrogen atmosphere at 60°C under 5 atom for 24 hours. After the catalyst was removed by filtration, the solvent was distilled off, and the residue was purified by

subjecting to silica gel flash column chromatography to obtain the title compound (0.945 g).

Spectral data ($^1$H-NMR, IR) of this product corresponded to those of the compound of Reference Example 6.

Reference Example A9

(i) 4-(4-Benzyloxyphenyl)-3-buten-1-ol

In an argon atmosphere, 3-hydroxypropyltriphenylphosphonium bromide (4.02 g) was suspended in dehydrated THF (30 mL), 60% oily sodium hydrate (0.4 g) was added, and then the mixture was refluxed for 3 hours. To the reaction mixture, a dehydrated THF solution (7 mL) of 4-benzyloxybenzaldehyde (2.12 g) was added dropwise and then the solution was refluxed for 67 hours. After cooling, insolubles were removed by filtration and the filtrate was concentrated under reduced pressure. The residue was purified by subjecting to column chromatography (eluent; hexane:ethyl acetate = 9:1 $\rightarrow$ 4:1) to obtain the title compound (1.76 g) as colorless crystals.

$^1$H-NMR ($CDCl_3$) δ: 2.46 (0.8H, dq, J = 1.4Hz, 6.2Hz), 2.61 (1.2H, dq, J = 1.6Hz, 6.4Hz), 3.71-3.78 (2H, m), 5.06 (1.2H, s), 5.07 (1.8H, s), 5.59 (0.6H, dt, J = 7.2Hz, 11.6Hz), 6.07 (0.4H, dt, J = 7.2Hz, 15.8Hz), 6.45 (0.4H, d, J = 15.8Hz), 6.52 (0.6H, d, J = 11.6Hz), 6.89-6.98 (2H, m), 7.22-7.46 (7H, m).

IR (KBr): 3279, 3063, 3036, 3011, 2911, 2867, 1607, 1574, 1510, 1470, 1454, 1383, 1302, 1250, 1177, 1117, 1053, 1017 $cm^{-1}$.

(ii) 4-(4-Hydroxybutyl)phenol

4-(4-Benzyloxyphenyl)-3-buten-1-ol (1.70 g) was dissolved in a mixture of methanol and THF (1:1, 20 mL) and 10% palladium-carbon (0.17 g) was added thereto, followed by vigorous stirring in a hydrogen atmosphere for 1.5 hours. The catalyst was removed by filtration and the filtrate was concentrated under reduced pressure to obtain the title compound (1.1 g) as a colorless crystalline powder.

$^1$H-NMR ($CDCl_3$) δ: 1.50-1.76 (4H, m), 2.57 (2H, t, J = 7.1Hz), 3.67 (2H, t, J = 6.2Hz), 6.74 (2H, d, J = 8.4Hz), 7.03 (2H, d, J = 8.4Hz).

IR (KBr): 3500-3100, 3025, 2940, 2859, 1615, 1597, 1514, 1456, 1362, 1240, 1173, 1107, 1055, 1024 $cm^{-1}$.

(iii) 4-[4-(Benzyloxy)phenyl]-1-butanol

In an argon atmosphere, dry DMF (115 mL) was added to 4-(4-hydroxybutyl)phenol(9.43 g) and 65% oily sodium hydride (2.4 g), followed by stirring for 15 minutes. A dry dimethylformamide (29.5 mL) solution of benzyl bromide (9.87 g) was added dropwise thereto with ice-cooling and stirring, followed by stirring at the same temperature for 2 hours. To the reaction mixture, ice-water and an aqueous 1 N potassium hydrogensulfate solution were added to the reaction mixture and the mixture was extracted with ethyl acetate. The organic layer was washed with saturated brine and then dried over anhydrous sodium sulfate. The solvent was distilled off under reduced pressure and the residue was purified by subjecting to silica gel column chromatography to obtain the title compound (10.67 g) as a colorless crystalline powder.

$^1$H-NMR (DMSO-$d_6$) δ: 1.34-1.64 (4H, m), 2.50 (2H, t, J = 7.0Hz), 3.39 (2H, dt, J = 5.2Hz, 6.4Hz), 4.34 (1H, t, J = 5.2Hz), 5.05 (2H, s), 6.90 (2H, d, J = 8.6Hz), 7.09 (2H, d, J = 8.6Hz), 7.28-7.47 (5H, m).

IR (KBr): 3500-3200, 3048, 3036, 2928, 2907, 2861, 2840, 1615, 1582, 1514, 1472, 1454, 1379, 1360, 1298, 1285, 1250, 1175, 1119, 1063, 1012 $cm^{-1}$.

(iv) 4-[4-(Benzyloxy)phenyl]butylmethanesulfonate

To an ethyl acetate (390 mL) solution of 4-(4-benzyloxyphenyl)butanol (10 g), triethylamine (8.16 mL) and methanesulfonyl chloride (4.53 mL) were added dropwise with ice-cooling. After stirring at the same temperature for 30 minutes and stirring at room temperature for an hour, the mixture was washed with ice-water and saturated brine. After drying over anhydrous sodium sulfate, the solvent was distilled off under reduced pressure to obtain the title compound (14 g) as an oily product. The resulting product was used in the following process without purification.

$^1$H-NMR ($CDCl_3$) δ: 1.64-1.86 (4H, m), 2.60 (2H, t, J = 7.1HZ), 2.98 (3H, s), 4.23 (2H, t, J = 6.1Hz), 5.05 (2H, s), 6.91 (2H, d, J = 8.8Hz), 7.09 (2H, d, J = 8.8Hz), 7.32-7.48 (5H, m).

IR (neat): 3063, 3031, 2940, 2865, 1611, 1584, 1512, 1456, 1354, 1337, 1240, 1175, 1115, 1015 $cm^{-1}$.

(v) Benzyl 4-(4-iodobutyl)phenyl ether

Sodium iodide (29.25 g) was dissolved in acetone (195 mL) and 4-[4-(benzyloxy)phenyl]butylmethanesulfonate (13 g) was added, and then the mixture was refluxed at 80°C for 1.5 hours. After cooling, the solvent was distilled off and ethyl acetate (750 mL) was added to the residue, followed by washing in turn with water, an aqueous sodium thiosulfate solution and saturated brine. The organic layer was dried over anhydrous magnesium sulfate and the solvent was distilled off under reduced pressure to obtain the title compound (14.29 g) as an oily product. The resulting product was used in the following process without purification.
$^1$H-NMR ($CDCl_3$) 5: 1.63-1.93 (4H, m), 2.57 (2H, t, J = 7.3Hz), 3.19 (2H, t, J = 6.8Hz), 5.04 (2H, s), 6.90 (2H, d, J = 8.8Hz), 7.09 (2H, d, J = 8.8Hz), 7.30-7.47 (5H, m).
IR (neat): 3063, 3031, 2932, 2857, 1611, 1582, 1510, 1454, 1381, 1298, 1238, 1175, 1121, 1026 $cm^{-1}$.

(vi) 1-[4-(4-Benzyloxyphenyl)butyl]-1H-1,2,3-triazole

Benzyl 4-(4-iodobutyl)phenyl ether (1.1 g), 1H-1,2,3-triazole (0.31 g) and potassium carbonate (0.622 g) were dissolved in DMF (7.5 mL) and stirred at 70°C for 26.5 hours. After cooling, the reaction mixture was extracted with ethyl acetate and then washed with water and saturated brine. Under reduced pressure, the solvent was distilled off and the residue was subjected to silica gel column chromatography (eluent; hexane:ethyl acetate = 4:1 → 2:3) to obtain the title compound (0.391 g).
$^1$H-NMR ($CDCl_3$) δ: 1.61 (2H, quintet, J = 7.8Hz), 1.93 (2H, quintet, J = 7.8Hz), 2.59 (2H, t, J = 7.6Hz), 4.39 (2H, t, J = 7.1Hz), 5.04 (2H, s), 6.90 (2H, d, J = 8.8Hz), 7.06 (2H, d, J = 8.8Hz), 7.30-7.48 (5H, m), 7.49 (1H, s), 7.69 (1H, s) .
IR (KBr): 3106, 3034, 2940, 2861, 1611, 1582, 1512, 1454, 1387, 1298, 1244, 1177, 1113, 1080, 1040, 1028 $cm^{-1}$.

(vii) 4-[4-(1H-1,2,3-Triazole-1-yl)butyl]phenol

1-[4-(4-Benzyloxyphenyl)butyl]-1H-1,2,3-triazole (0.38 g) was dissolved in methanol (7.6 mL) and 10% palladium-carbon (0.1 g) was added, followed by vigorous stirring in a hydrogen atmosphere for 14 hours. The catalyst was removed by filtration and the filtrate was concentrated to dryness under reduced pressure to obtain the title compound (0.268 g) as a crystalline powder.
$^1$H-NMR ($CDCl_3$) δ: 1.60 (2H, quintet, J = 7.0Hz), 1.93 (2H, quintet, J = 7.4Hz), 2.57 (2H, t, J = 7.5Hz), 4.40 (2H, t, J = 7.0Hz), 6.79 (2H, d, J = 8.6Hz), 6.99 (2H, d, J = 8.6Hz), 7.51(1H,s), 7.71(1H,s).
IR (KBr): 3148, 3129, 3017, 2946, 2861, 2814, 1615, 1593, 1514, 1462, 1381, 1269, 1242, 1225, 1123, 1078 $cm^{-1}$.

Reference Example A10

4-[3- (1H-1,2,3-Triazole-1-yl)propyl]phenol

Benzyl 4-(3-iodopropyl)phenyl ether (2.47 g), 1H-1,2,3-triazole (629 mg) and potassium carbonate (1.26 g) were dissolved in (17.5 mL) and then stirred at 70°C for 18.5 hours. After returning to room temperature, the mixture was extracted with ethyl acetate and then washed with water and saturated brine. Under reduced pressure, the solvent was distilled off and the residue was purified by subjecting to silica gel column chromatography (eluent; hexane:ethyl acetate = 4:1 → 2:3) to obtain 1-[3-(4-benzyloxyphenyl)propyl]-1H-1,2,3-triazole (856 mg).
$^1$H-NMR ($CDCl_3$) δ: 2.23 (2H, quintet, J = 7.2Hz), 2.60 (2H, t, J = 7.5Hz), 4.38 (2H, t, J = 7.1Hz), 5.05 (2H, s), 6.92 (2H, d, J = 8.8Hz), 7.10 (2H, d, J = 8.8Hz), 7.30-7.48 (5H, m), 7.52 (1H, s), 7.72 (1H, s).
IR (KBr): 3100, 3030, 2960, 2926, 2860, 1613, 1585, 1514, 1454, 1383, 1298, 1250, 1215, 1177, 1115, 1082, 1044, 1028, 1019 $cm^{-1}$.
1-[3-(4-Benzyloxyphenyl)propyl]-1H-1,2,3-triazole (850 mg) was dissolved in methanol(29 mL) and 10% palladium-carbon (0.1 g) was added, followed by vigorous stirring in a hydrogen atmosphere for 13 hours. The catalyst was removed by filtration and the filtrate was concentrated to dryness under reduced pressure to obtain the title compound (600 mg) as a crystalline powder.
$^1$H-NMR ($CDCl_3$) δ: 2.22 (2H, quintet, J = 7.0Hz), 2.56 (2H, t, J = 7.0Hz), 4.38 (2H, t, J = 7.0Hz), 6.87 (2H, d, J = 8.6Hz), 7.04 (2H, d, J = 8.6Hz), 7.55 (1H, s), 7.74 (1H, s). IR (KBr): 3127, 3100, 3015, 2932, 1615, 1595, 1516, 1456, 1373, 1244, 1223, 1175, 1121, 1080, 1038 $cm^{-1}$.

Reference Example A11

3-[3-(1H-1,2,3-Triazole-1-yl)propyl]phenol

(i) 3-[3-(Benzyloxy)phenyl]-1-propanol

In a stream of argon gas, 3-benzyloxybenzaldehyde (21.3 g) and ethyl diethylphosphonoacetate (23.6 g) were

suspended in dry DMF (250 mL). 65% Oily sodium hydride (3.88 g) was added in several portions with ice-cooling and stirring, and, after the completion of the addition, the mixture was stirred at room temperature for 2 hours. After the solvent was distilled off, the residue was dissolved in ethyl acetate, washed with water and saturated brine and then dried over anhydrous sodium sulfate. Under reduced pressure, the solvent was distilled off to obtain 33.15 g of a crude product of ethyl (E)-3-[3-(benzyloxy)phenyl]-2-propenate as an oily product. The resulting product was dissolved in ethanol (406 mL) and 5% palladium-carbon [Pd-C(en), 2.7 g] treated with ethylenediamine was added, followed by vigorous stirring in a hydrogen atmosphere. The addition of hydrogen was terminated by consumption of hydrogen (1.75 L) and the catalyst was removed by filtration. Under reduced pressure, the solvent was distilled off and the residue was dissolved in dehydrated THF (120 mL). The resulting solution was added dropwise in a mixture of lithium aluminum hydride (4.61 g) suspended in dehydrated THF (120 mL) with ice-cooling, followed by stirring for 1.5 hours with ice-cooling and further stirring at the room temperature for an hour. The reaction mixture was added to ice-water and, after acidification, the reaction mixture was extracted with ethyl acetate, washed with water and saturated brine and then dried over anhydrous sodium sulfate. Under reduced pressure, the solvent was distilled off and the residue was purified by subjecting to silica gel column chromatography to obtain the title compound (14.39 g) as a colorless oily product.
$^1$H-NMR ($CDCl_3$ ) δ: 1.80-1.96 (2H, m), 2.69 (2H, t, J = 7.7Hz), 3.66 (2H, t, J = 6.4Hz), 5.05 (2H, s), 6.77-6.87 (3H, m), 7.20 (1H, t, J = 8.0Hz), 7.28-7.48 (5H, m).
IR (neat): 3330, 3063, 3032, 2940, 2867, 1599, 1582, 1487, 1453, 1381, 1314, 1258, 1155, 1026 $cm^{-1}$.

(ii) 3-[3-(Benzyloxy)phenyl]propylmethanesulfonate

Using 3-(3-benzyloxyphenyl)propanol (13.5 g), triethylamine (8.16 mL) and methanesulfonyl chloride (4.53 mL), the same reaction as in Reference Example A9-(iv) was conducted to obtain the title compound (19.7 g) as an oily product.
$^1$H-NMR C$CDCl_3$ ) δ: 2.00-2.15 (2H, m) , 2.73 (2H, t, J = 7.5Hz), 2.98 (3H, s), 4.22 (2H, t, J = 6.3Hz), 5.06 (2H, s), 6.77-6.88 (3H, m), 7.22 (1H, t, J = 7.7Hz), 7.31-7.48 (5H, m).
IR (neat): 3032, 2940, 2870, 1599, 1584, 1487, 1453, 1381, 1354, 1260, 1175, 1026 $cm^{-1}$.

(iii) Benzyl 3-(3-iodopropyl)phenyl ether

Using 3-[3-(benzyloxy)phenyl]propylmethanesulfonate (19.7 g) and sodium iodide (29.25 g), the same reaction as in Reference Example A9-(v) was conducted to obtain the title compound (18.4 g) as an oily product.
$^1$H-NMR ($CDCl_3$) δ: 2.11 (2H, quintet, J = 7.3Hz), 2.70 (2H, t, J = 7.2Hz), 3.16 (2H, t, J = 6.8Hz), 5.06 (2H, s), 6.78-6.87 (3H, m), 7.21 (1H, t, J = 7.2Hz), 7.32-7.48 (5H, m).
IR (neat): 3063, 3031, 2934, 2861, 1599, 1582, 1487, 1451, 1381, 1316, 1258, 1213, 1155, 1080, 1028 $cm^{-1}$.

(iv) 1-[3-(3-Benzyloxyphenyl)propyl]-1H-1,2,3-triazole

In an argon atmosphere, 1H-1,2,3-triazole (0.9 g) was dissolved in DMF (20 mL) and 65% oily sodium hydride (0.48 g) was added. After stirring for 30 minutes, a DMF (5 mL) solution of benzyl 3-(3-iodopropyl)phenyl ether (3.53 g) was added, followed by stirring at room temperature for 19 hours. The reaction mixture was diluted with ethyl acetate and then washed with water and saturated brine. Under reduced pressure, the solvent was distilled off and the residue was subjected to column chromatography to obtain the title compound (1.1 g) as colorless crystals.
mp 74-75°C.
$^1$H-NMR ($CDCl_3$) δ: 2.25 (2H, quintet, J = 7.2Hz), 2.63 (2H, t, J = 7.3Hz), 4.37 (2H, t, J = 7.1Hz), 5.05 (2H, s), 6.75-6.88 (3H, m), 7.23 (1H, t, J = 8.2Hz), 7.31-7.47 (5H, m), 7.49 (1H, d, J = 1.0Hz), 7.71 (1H, d, J = 1.0Hz).
IR (KBr): 3125, 3063, 3032, 2944, 2867, 1599, 1584, 1487, 1453, 1381, 1316, 1260, 1215, 1157, 1113, 1074, 1028 $cm^{-1}$.

(v) 3-[3-(1H-1,2,3-Triazole-1-yl)propyl]phenol

To a methanol solution (32 mL) of 1-[3-(3-benzyloxyphenyl)propyl]-1H-1,2,3-triazole(0.937 g), 10% palladium-carbon (0.1 g) was added, followed by vigorous stirring in a hydrogen atmosphere at room temperature for 8 hours. The catalyst was removed by filtration and the filtrate was concentrated to dryness under reduced pressure to obtain the title compound (0.593 g) as colorless crystals.
mp 85-86°C.
$^1$H-NMR ($CDCl_3$) δ: 2.24 (2H, quintet, J = 7.1Hz), 2.60 (2H, t, J = 7.5Hz), 4.38 (2H, t, J = 7.1Hz), 6.68-6.79 (3H, m), 6.96 (1H, s), 7.16 (1H, t, J = 8.1Hz), 7.54 (1H, d, J = 1.0Hz), 7.73 (1H, d, J = 1.0Hz).
IR (KBr): 3129, 3077, 3054, 2949, 2863, 2722, 2614, 1599, 1588, 1483, 1458, 1362, 1337, 1281, 1221, 1157, 1121, 1080, 1038 $cm^{-1}$.

Reference Example A12

4-{4-[2-(2-Hydroxyethyl)-1H-imidazol-1-yl]butyl}phenol (i) 2-(1-{4-[4-(Benzyloxy)phenyl]butyl}-1H-imidazol-2-yl)-1-ethanol

Benzyl 4-(4-iodobutyl)phenyl ether (14.29 g), 2-(2-hydroxyethyl)imidazole (13.1 g) and potassium carbonate (5.39 g) were mixed in DMF (390 mL) at 60°C for 16 hours. After cooling, insolubles were removed by filtration and the filtrate was concentrated under reduced pressure. The residue was dissolved in ethyl acetate and then washed with water and saturated brine. Under reduced pressure, the solvent was distilled off and the residue was purified by subjecting to column chromatography (eluent; ethyl acetate:methanol = 19:1 → 9:1). The effluent was recrystallized from ethyl acetate-methanol to obtain the title compound (10.99 g) as colorless crystals.
mp 75-77°C.
$^1$H-NMR ($CDCl_3$) δ: 1.53-1.82 (4H, m), 2.58 (2H, t, J = 7.1Hz), 2.78 (2H, t, J = 5.5Hz), 3.81 (2H, t, J = 6.9Hz), 4.03 (2H, t, J = 5.5Hz), 5.04 (2H, s), 6.80 (1H, d, J = 1.2Hz), 6.90 (2H, d, J = 8.6Hz), 6.93 (1H, d, J = 1.2Hz), 7.05 (2H, d, J = 8.6Hz), 7.34-7.47 (5H, m).
IR (KBr): 3144, 3032, 2934, 2859, 1611, 1582, 1514, 1495, 1456, 1431, 1381, 1298, 1273, 1244, 1175, 1150, 1121, 1109, 1051, 1026 $cm^{-1}$.

(ii) 4-{4-[2-(2-Hydroxyethyl)-1H-imidazol-1-yl]butyl}phenol

Using 2-(1-{4-[4-(benzyloxy)phenyl]butyl}-1H-imidazol-2-yl)-1-ethanol(10.67 g) and 10% palladium-carbon (1.6 g), the same reaction as in Reference Example A11-(v) was conducted to obtain the title compound (5.3 g).
mp 118-119°C.
$^1$H-NMR ($CDCl_3$) δ: 1.50-1.80 (4H, m), 2.55 (2H, t, J = 7.0Hz), 2.79 (2H, t, J = 5.8Hz), 3.82 (2H, t, J = 7.0Hz), 3.97 (2H, t, J = 5.8Hz), 3.85-4.40 (1H, br), 6.77 (2H, d, J = 8.4Hz), 6.80 (1H, s), 6.94 (1H, s), 6.96 (2H, d, J = 8.4Hz).
IR (KBr): 3600-2400, 1615, 1593, 1516, 1489, 1456, 1373, 1252, 1171, 1150, 1125, 1103, 1055 $cm^{-1}$.

Reference Example A13

(i) 2-(1-{3-[4-(Benzyloxy)phenyl]propyl}-1H-imidazol-2-yl)-1-ethanol

Using benzyl 4-(3-iodopropyl)phenyl ether (5.28 g), 2-(2-hydroxyethyl)imidazole (5.05 g) and potassium carbonate (2.07 g), the same reaction as in Reference Example A12-(i) was conducted to obtain the title compound (2.78 g) as colorless crystals.
mp 80-82°C.
$^1$H-NMR ($CDCl_3$) δ: 2.03 (2H, quintet, J = 7.4Hz), 2.58 (2H, t, J = 7.4Hz), 2.74 (2H, t, J = 5.6Hz), 3.82 (2H, t, J = 7.4Hz), 4.01 (2H, t, J = 5.6Hz), 5.05 (2H, s), 6.83 (1H, s), 6.92 (2H, d, J = 8.6Hz), 6.94 (1H, s), 7.07 (2H, d, J = 8.6Hz), 7.32-7.47 (5H, m).
IR (KBr): 3500-3100, 3110, 3063, 3032, 2934, 2865, 1611, 1584, 1512, 1495, 1454, 1381, 1298, 1240, 1177, 1152, 1121, 1057, 1024 $cm^{-1}$.

(ii) 4-{3-[2-(2-Hydroxyethyl)-1H-imidazol-1-yl]propyl}phenol

Using 2-(1-{3-[4-(benzyloxy)phenyl]propyl}-1H-imidazol-2-yl)-1-ethanol (2.53 g) and 10% palladium-carbon (0.38 g), the same reaction as in Reference Example A11-(v) was conducted to obtain the title compound (1.85 g) as colorless crystals.
mp 116-117°C.
$^1$H-NMR ($CDCl_3$+$CD_3OD$) δ: 2.03 (2H, quintet, J = 7.3Hz), 2.55 (2H, t, J = 7.3Hz), 2.75 (2H, t, J = 6.2Hz), 3.83 (2H, t, J = 7.3Hz), 3.91 (2H, t, J = 6.2Hz), 6.77 (2H, d, J = 8.6Hz), 6.84 (1H, d, J = 1.2Hz), 6.93 (1H, d, J = 1.2Hz), 6.97 (2H, d, J = 8.6Hz).
IR (KBr): 3500-3100, 3119, 2934, 2861, 1615, 1593, 1516, 1495, 1454, 1373, 1252, 1173, 1152, 1123, 1053 $cm^{-1}$.

Reference Example A14

3-{3-[2-(2-Hydroxyethyl)-1H-imidazol-1-yl]propyl}phenol

(i) 2-(1-{3-[3-(Benzyloxy)phenyl]propyl}-1H-imidazol-2-yl)-1-ethanol

Using benzyl 3-(3-iodopropyl)phenyl ether (3.53 g), 2-(2-hydroxyethyl)imidazole (1.46 g) and 65% oily sodium

hydride (0.48 g), the same reaction as in Reference Example A11-(iv) was conducted to obtain the title compound (2.66 g) as a colorless oily product.
$^1$H-NMR ($CDCl_3$) δ: 2.05 (2H, quintet, J = 7.3Hz), 2.61 (2H, t, J = 7.5Hz), 2.73 (2H, t, J = 5.5Hz), 3.81 (2H, t, J = 7.3Hz), 4.02 (2H, t, J = 5.5Hz), 5.06 (2H, s), 6.73-6.88 (3H, m), 6.82 (1H, d, J = 1.2Hz), 6.95 (1H, d, J = 1.2Hz), 7.23 (1H, t, J = 8.2Hz), 7.31-7.48 (5H, m).
IR (neat): 3500-3100, 3067, 3034, 2938, 2867, 1599, 1584, 1524, 1491, 1453, 1381, 1316, 1260, 1155, 1119, 1053, 1026 $cm^{-1}$.

(ii) 3-{3-[2-(2-Hydroxyethyl)-1H-imidazol-1-yl]propyl}phenol

Using 2-(1-{3-[3-(benzyloxy)phenyl]propyl}-1H-imidazol-2-yl)-1-ethanol (2.42 g) and 10% palladium-carbon (0.24 g), the same reaction as in Reference Example A11-(v) was conducted to obtain the title compound (1.69 g) as colorless crystals.
mp 111-113°C.
$^1$H-NMR ($CDCl_3$) δ: 2.07 (2H, quintet, J = 6.9Hz), 2.55 (2H, t, J = 7.3Hz), 2.73 (2H, t, J = 5.9Hz), 3.80 (2H, t, J = 7.1Hz), 4.00 (2H, t, J = 5.9Hz), 6.55-6.76 (3H, m), 6.86 (1H, d, J = 1.4Hz), 6.96 (1H, d, J = 1.4Hz), 7.15 (1H, t, J = 7.8Hz).
IR (KBr)$cm^{-1}$: 3500-3100, 3046, 2940, 2865, 2712, 2604, 1599, 1588, 1528, 1483, 1456, 1372, 1279, 1250, 1155, 1123, 1057.

Reference Example A15

3-{1-[4-(4-Hydroxyphenyl)butyl]-1H-imidazol-2-yl}-1,2-propanediol

(i) 3-{1-[4-(4-Benzyloxyphenyl}butyl]-1H-imidazol-2-y1}-1,2-propanediol

Using benzyl 4-(4-iodobutyl)phenyl ether (2.05 g), 2-(2,3-dihydroxypropyl)imidazole (1.0 g) and 65% oily sodium hydride (0.259 g), the same reaction as in Reference Example A11-(iv) was conducted to obtain the title compound (1.23 g) as colorless crystals.
$^1$H-NMR ($CDCl_3$) δ: 1.52-1.83 (4H, m), 2.57 (2H, t, J = 7.1Hz), 2.78 (2H, d, J = 5.2Hz), 2.79 (1H, d, J = 6.8Hz), 3.62 (1H, dd, J = 4.8Hz, 11.2Hz), 3.74 (1H, dd, J = 4.8Hz, 11.2Hz), 3.82 (2H, t, J = 7.1Hz), 4.12-4.23 (1H, m), 5.04 (2H, s), 6.79 (1H, d, J = 1.4Hz), 6.90 (2H, d, J = 8.6Hz), 6.91 (1H, d, J = 1.4Hz), 7.05 (2H, d, J = 8.6Hz), 7.30-7.47 (5H, m).
IR (KBr): 3500-3200, 3065, 3030, 2932, 2861, 1611, 1582, 1510, 1495, 1454, 1379, 1296, 1275, 1240, 1177, 1150, 1123, 1080, 1026 $cm^{-1}$.

(ii) 3-{1-[4-(4-Hydroxyphenyl)butyl]-1H-imidazol-2-yl}-1,2-propanediol

Using 3-{1-[4-(4-benzyloxyphenyl)butyl]-1H-imidazol-2-yl}-1,2-propanediol (1.22 g) and 10% palladium-carbon (0.18 g), the same reaction as in Reference Example A11-(v) was conducted to obtain the title compound (0.918 g) as colorless crystals.
$^1$H-NMR ($CDCl_3$+$CD_3OD$) δ: 1.50-1.80 (4H, m), 2.55 (2H, t, J = 7.0Hz), 2.75 (1H, d, J = 7.2Hz), 2.76 (1H, d, J = 5.6Hz), 3.49 (1H, dd, J = 5.4Hz, 11.6Hz), 3.62 (1H, dd, J = 4.2Hz, 11.6Hz), 3.84 (2H, t, J = 7.0Hz), 3.97-4.08 (1H, m), 6.75 (2H, d, J = 8.6Hz), 6.80 (1H, d, J = 1.4Hz), 6.89 (1H, d, J = 1.4Hz), 6.97 (2H, d, J = 8.6Hz).
IR (KBr): 3500-3100, 3011, 2936, 2859, 1613, 1595, 1516, 1489, 1456, 1372, 1360, 1252, 1171, 1150, 1125, 1101, 1030 $cm^{-1}$.

Reference Example A16

(i) 3-{1-[3-(3-Benzyloxyphenyl)propyl]-1H-imidazol-2-yl}-1,2-propanediol

Using benzyl 3-(3-iodopropyl)phenyl ether (1.98 g), 2-(2,3-dihydroxypropyl)imidazole (1.0 g) and 65% oily sodium hydride (0.259 g), the same reaction as in Reference Example A11-(iv) was conducted to obtain the title compound (1.31 g) as a colorless oily product.
$^1$H-NMR ($CDCl_3$) δ: 2.05 (2H, quintet, J = 7.3Hz), 2.60 (2H, t, J = 7.3Hz), 2.73 (1H, d, J = 4.8Hz), 2.74 (1H, d, J = 7.2Hz), 3.61 (1H, dd, J = 4.8Hz, 11.2Hz), 3.74 (1H, dd, J = 4.8Hz, 11.2Hz), 3.82 (2H, t, J = 7.3Hz), 4.12-4.23 (1H, m), 5.06 (2H, s), 6.73-6.88 (3H, m), 6.81 (1H, d, J = 1.2Hz), 6.93 (1H, d, J = 1.2Hz), 7.23 (1H, t, J = 8.4Hz), 7.31-7.48 (5H, m).
IR (neat): 3500-3200, 3063, 3032, 2934, 2865, 1599, 1584, 1526, 1489, 1454, 1381, 1316, 1260, 1155, 1123, 1082, 1028 $cm^{-1}$.

(ii) 3-{1-[3-(3-Hydroxyphenyl}propyl]-1H-imidazol-2-yl}-1,2-propanediol

Using 3-{1-[3-(3-benzyloxyphenyl)propyl]-1H-imidazol-2-yl}-1,2-propanediol(1.30 g) and 10% palladium-carbon (0.195 g), the same reaction as in Reference Example A11-(v) was conducted to obtain the title compound (0.979 g) as a colorless oily product.
$^1$H-NMR ($CDCl_3$+$CD_3OD$) δ: 2.07 (2H, quintet, J = 7.4Hz), 2.58 (2H, t, J = 7.3Hz), 2.72 (1H, d, J = 6.8Hz), 2.72 (1H, d, J = 5.8Hz), 3.50 (1H, dd, J = 5.4Hz, 11.4Hz), 3.61 (1H, d, J = 4.2Hz, 11.4Hz), 3.85 (2H, t, J = 7.3Hz), 3.98-4.10 (1H, m), 6.60-6.74 (3H, m), 6.86 (1H, d, J = 1.4Hz), 6.92 (1H, d, J = 1.4Hz), 7.14 (1H, t, J = 7.8Hz).
IR (neat): 3500-3100, 3040, 2942, 2863, 1599, 1588, 1530, 1483, 1456, 1360, 1279, 1254, 1155, 1125, 1088, 1030 $cm^{-1}$.

Reference Example A17

2-[(E)-2-(2,4-Difluorophenyl)ethenyl]-4-[[4-(4-iodobutyl)phenoxy]methyl]-1,3-oxazole

(i) 4-[4-[2-(E)-[2-(2,4-Difluorophenyl)ethenyl]-1,3-oxazol-4-yl]methoxyphenyl]-1-butanol

To a DMF(20 mL) solution of 4-(4-hydroxyphenyl)-1-butanol(1.99 g), 60% oily sodium hydride (528 mg) was added with ice-cooling, followed by stirring at room temperature for 30 minutes. (E)-4-Chloromethyl-2-[2-(2,4-difluorophenyl)ethenyl]oxazole (3.37 g) was added thereto with ice-cooling, followed by stirring at room temperature overnight. After adding water and 1 N hydrochloric acid, the reaction mixture was extracted with ethyl acetate. The extract was dried over magnesium sulfate and concentrated under reduced pressure, and then the residue was recrystallized from ethyl acetate-diethyl ether-hexane to obtain the title compound (3.71 g) as colorless crystals. mp 75-76°C.
$^1$H-NMR ($CDCl_3$) δ: 1.5-1.7 (4H, m), 2.60 (2H, t, J = 6.8Hz), 3.66 (2H, t, J = 6.0Hz), 5.02 (2H, s), 6.8-6.9 (1H, m), 6.89 (2H, d, J = 8.4Hz), 6.98 (1H, d, J = 17.0Hz), 7.11 (2H, d, J = 8.4Hz), 7.5-7.6 (1H, m), 7.59 (1H, d, J = 17.0Hz), 7.66 (1H, s).
IR (KBr): 1613, 1514, 1493, 1431, 1279, 1246, 1140, 968, 856 $cm^{-1}$.

(ii) 2-[(E)-2-(2,4-Difluorophenyl)ethenyl]-4-[[4-(4-iodobutyl)phenoxy]methyl]-1,3-oxazole

To a THF (50 mL) solution of 4-[4-[2-(E)-[2-(2,4-difluorophenyl) ethenyl]-1,3-oxazol-4-yl]methoxyphenyl]-1-butanol (3.47 g), triethylamine (1.37 mL) was added and methanesulfonyl chloride (0.77 mL) was added thereto with ice-cooling, followed by stirring at room temperature for 30 minutes. After addition of water, the reaction mixture was extracted with ethyl acetate and the extract was washed with brine and dried over magnesium sulfate. The solvent was distilled off and acetone (100 mL) and sodium iodide (6.75 g) were added to the residue, followed by stirring at 40-50°C for 2 hours. The reaction mixture was concentrated and, after addition of water, the mixture was extracted with ethyl acetate. The extract was washed in turn with aqueous sodium thiosulfate and brine, dried over magnesium sulfate and then concentrated under reduced pressure. The deposit was collected by filtration and then washed with diethyl ether-hexane to obtain the title compound (3.55 g) as a pale yellow powder.
$^1$H-NMR ($CDCl_3$) δ: 1.6-1.9 (4H, m), 2.5-2.7 (2H, m), 3.1-3.3 (2H, m), 5.02 (2H, s), 6.8-7.2 (6H, m), 7.5-7.75 (4H, m).
IR (KBr): 1615, 1514, 1493, 1431, 1279, 1246, 1140, 966, 856 $cm^{-1}$.

Reference Example A18

2-[(E)-2-(4-Bromophenyl)ethenyl]-4-[[4-(4-iodobutyl)phenoxy]methyl]-1,3-oxazole

Using 4-(4-hydroxyphenyl)-1-butanol (4.99 g) and (E)-4-chloromethyl-2-[2-(4-bromophenyl)ethenyl]oxazole (7.43 g), the same reaction as in Reference Example A17-(i) was conducted to obtain 4-[4-[2-(E)-[2-(4-bromophenyl) ethenyl]-1,3-oxazol-4-yl]methoxyphenyl]-1-butanol (9.70 g). Using the resulting compound (4.28 g), the same reaction as in Reference Example A17-(ii) was conducted to obtain the title compound (4.47 g) as a white powder.
$^1$H-NMR ($CDCl_3$) δ: 1.65-1.95 (4H, m), 2.58 (2H, t, J = 7.2Hz), 3.20 (2H, t, J = 6.8Hz), 5.02 (2H, s), 6.92 (1H, d, J = 16.4Hz), 6.92 (2H, d, J = 8.6Hz), 7.38 (2H, d, J = 8.4Hz), 7.47 (1H, d, J = 16.4Hz), 7.52 (2H, d, J = 8.4Hz), 7.66 (1H, s).

Reference Example B1

[1-[4-[4- [[2-[(E)-2-(4-Methylphenyl)ethenyl] -1,3-oxazol-4-yl]methoxy]phenyl]butyl]-1H-1,2,3-triazole

To a DMF(4 mL) solution of 4-[4-(1H-1,2,3-triazole-1-yl)butyl]phenol (174 mg), 60% oily sodium hydride (35 mg) was added with ice-cooling, followed by stirring at room temperature for 30 minutes. (E)-4-Chloromethyl-2-[2-(4-methylphenyl)ethenyl]oxazole (206 mg) was added thereto with ice-cooling, followed by stirring at room temperature for 2 hours. Water was added to the reaction mixture and the deposit was collected by filtration and then washed with water. The resulting substance thus obtained was dissolved in a mixture of THF and ethyl acetate, washed with water and brine, dried over magnesium sulfate and then concentrated under reduced pressure. The residue was recrystallized from ethyl acetate-hexane to obtain the title compound (281 mg) as colorless crystals.
mp 154-155°C.
$^1$H-NMR ($CDCl_3$) δ: 1.5-1.7 (2H, m), 1.85-2.05 (2H, m), 2.38 (3H, s), 2.60 (2H, t, J = 7.5Hz), 4.39 (2H, t, J = 7.0Hz), 5.01 (2H, s), 6.87 (2H, d, J = 8.6Hz), 6.9-7.0 (1H, m), 7.19 (2H, d, J = 8.6Hz), 7.19 (2H, d, J = 8.0Hz), 7.42 (2H, d, J = 8.0Hz), 7.5-7.7 (4H, m).
IR (KBr): 1640, 1607, 1530, 1514, 1464, 1339, 1256, 1211, 1053, 974, 810 $cm^{-1}$.
Anal. Calcd for $C_{25}H_{26}N_4O_2$: C, 72.44; H, 6.32; N, 13.52.
Found : C, 72.36; H, 6.49; N, 13.70.

Reference Example B2

1-{4-[4-({2-[(E)-2-(4-Fluorophenyl)ethenyl]-1,3-oxazol-4-yl}methoxy}phenyl]butyl}-1H-1,2,3-triazole

In an argon atmosphere, 4-[4-(1H-1,2,3-triazole-1-yl)butyl]phenol (218 mg) and 65% oily sodium hydride (39 mg) were dissolved in DMF (5 mL). 4-(Chloromethyl)-2-[(E)-2-(4-fluorophenyl)ethenyl]-1,3-oxazole (250 mg) was added thereto with ice-cooling and stirring, followed by stirring at room temperature for 3 hours. After addition of water, the reaction mixture was extracted with ethyl acetate. The extract was washed with water and saturated brine, dried over sodium sulfate and then concentrated under reduced pressure. The residue was purified by subjecting to silica gel column chromatography (eluent; chloroform:ethanol = 24:1) and then recrystallized from ethyl acetate to obtain the title compound (368 mg) as colorless crystals.
mp 124-125°C.
$^1$H-NMR ($CDCl_3$) δ: 1.62 (2H, quintet, J = 7.0Hz), 1.94 (2H, quintet, J = 7.5Hz), 2.61 (2H, t, J = 7.5Hz), 4.40 (2H, t, J = 7.0Hz), 5.01 (2H, s), 6.86 (1H, d, J = 16.0Hz), 6.92 (2H, d, J = 8.6Hz), 7.08 (2H, d, J = 8.6Hz), 7.09 (2H, t, J = 8.7Hz), 7.46-7.57 (4H, m), 7.66 (1H, s), 7.70 (1H, d, J = 1.0Hz).
IR (KBr): 3420, 3160, 3120, 2940, 2924, 2865, 1644, 1599, 1584, 1532, 1512, 1466, 1435, 1400, 1337, 1302, 1248, 1229, 1211, 1177, 1161, 1113, 1076, 1049, 1030 $cm^{-1}$.
Anal Calcd for $C_{24}H_{23}N_4O_2F$: C, 68.88; H, 5.55; N, 13.39.
Found: C, 68.70; H, 5.55; N, 13.49.

Reference Example B3

1-{3-[3-({2-[(E)-2-(4-Fluorophenyl)ethenyl]-1,3-oxazol-4-yl)methoxy)phenyl]propyl}-1H-1,2,3-triazole

Using 3-[3-(1H-1,2,3-triazole-1-yl)propyl]phenol (208 mg), 65% oily sodium hydride (39 mg) and 4-(chloromethyl)-2-[(E)-2-(4-fluorophenyl)ethenyl]-1,3-oxazole (250 mg), the same reaction as in Reference Example B2 was conducted to obtain the title compound (366 mg).
mp 105-106°C.
$^1$H-NMR ($CDCl_3$) δ: 2.26 (2H, quintet, J = 7.2Hz), 2.64 (2H, t, J = 7.5Hz), 4.39 (2H, t, J = 7.0Hz), 5.03 (2H, s), 6.78-6.89 (3H, m), 6.86 (1H, d, J = 16.2Hz), 7.09 (2H, t, J = 8.6Hz), 7.25 (1H, t, J = 7.8Hz), 7.51 (1H, d, J = 16.2Hz), 7.47-7.54 (3H, m), 7.68 (1H, s), 7.72 (1H, s).
IR (KBr): 3110, 3050, 2955, 2870, 1642, 1601, 1586, 1532, 1507, 1489, 1460, 1453, 1337, 1310, 1273, 1240, 1213, 1177, 1159, 1113, 1097, 1080, 1065 $cm^{-1}$.
Anal calcd for $C_{23} H_{21} N_4 O_2$ F: C, 68.30; H, 5.23; N, 13.85.
Found: C, 68.22; H, 5.04; N, 14.00.

Reference Example B4

1-(4-{4-[(2-{ (E)-2-[4-(Trifluoromethyl)phenyl]ethenyl}-1,3-oxazol-4-yl)methoxy]phenyl}butyl)-1H-1,2,3-triazole

Using 4-[4- (1H-1,2,3-triazole-1-yl)butyl]phenol (152 mg), 65% oily sodium hydride (28 mg) and 4-(chloromethyl)-2-{ (E)-2-[4-(trifluoromethyl)phenyl]ethenyl}-1,3-oxazole (212 mg), the same reaction as in Reference Example B2 was conducted to obtain the titled compound (290 mg).
mp 160-161°C.
$^1$H-NMR ($CDCl_3$) δ: 1.62 (2H, quintet, J = 7.0Hz), 1.94 (2H, quintet, J = 7.6Hz), 2.61 (2H, t, J = 7.4Hz), 4.40 (2H, t, J = 7.4Hz), 5.02 (2H, s), 6.92 (2H, d, J = 8.6Hz), 7.02 (1H, d, J = 16.6Hz), 7.08 (2H, d, J = 8.6Hz), 7.50 (1H, s), 7.56

(1H, d, J = 16.6Hz), 7.64 (4H, s), 7.69 (1H, s), 7.71 (1H, s).
IR (KBr): 3120, 2936, 1615, 1584, 1512, 1464, 1414, 1327, 1248, 1159, 1125, 1069 $cm^{-1}$.
Anal calcd for $C_{25}H_{23}N_4O_2F_3$: C, 64.10; H, 4.95; N, 11.96.
Found: C, 64.18; H, 5.12; N, 11.98.

Reference Example B5

1-(3-{4-[(2-{ (E)-2-[4-(Trifluoromethyl)phenyl]ethenyl}-1, 3-oxazol-4-yl)methoxy]phenyl}propyl)-1H-1,2,3-triazole

Using 4-[3-(1H-1,2,3-triazole-1-yl)propyl]phenol (143 mg), 65% oily sodium hydride( ( 28 mg) and 4-(chloromethyl)-2-{ (E)-2-[4-(trifluoromethyl)phenyl]ethenyl}-1, 3-oxazole (212 mg), the same reaction as in Reference Example B2 was conducted to obtain the title compound (232 mg).
mp 157-158°C.
$^1$H-NMR ($CDCl_3$) δ: 2.24 (2H, quintet, J = 7.2Hz), 2.61 (2H, t, J = 7.3Hz), 4.39 (2H, t, J = 7.2Hz), 5.03 (2H, s), 6.94 (2H, d, J = 8.4Hz), 7.02 (1H, d, J = 16.4Hz), 7.11 (2H, d, J = 8.4Hz), 7.52 (1H, s), 7.56 (1H, d, J = 16.4Hz), 7.64 (4H, s), 7.69 (1H, s), 7.72 (1H, s).
IR (KBr): 3129, 3100, 2934, 1613, 1584, 1547, 1510, 1449, 1416, 1337, 1329, 1291, 1238, 1179, 1140, 1109, 1071, 1009 $cm^{-1}$.
Anal calcd for $C_{24}H_{21}N_4O_2F_3$: C, 63.43; H, 4.66; N, 12.33.
Found: C, 63.21; H, 4.73; N, 12.26.

Reference Example B6

1-(3-{3-[(2-{ (E)-2-[4-(Trifluoromethyl)phenyl]ethenyl}-1, 3-oxazol-4-yl)methoxy]phenyl}propyl)-1H-1,2, 3-triazole

Using 3-[3-(1H-1,2,3-triazole-1-yl)propyl]phenol (123 mg), 65% oily sodium hydride (24 mg) and 4-(chloromethyl)-2-{ (E)-2-[4-(trifluoromethyl)phenyl]ethenyl}-1,3-oxazole (183 mg), the same reaction as in Reference Example B2 was conducted to obtain the title compound (248 mg).
mp 115-116°C.
$^1$H-NMR ($CDCl_3$) δ: 2.26 (2H, quintet, J = 7.2Hz), 2.64 (2H, t, J = 7.2Hz), 4.39 (2H, t, J = 7.2Hz), 5.04 (2H, s), 6.77-6.91 (3H, m), 7.01 (1H, d, J = 16.6Hz), 7.25 (1H, t, J = 8.4Hz), 7.52 (1H, s), 7.56 (1H, d, J = 16.6Hz), 7.64 (4H, s), 7.71 (2H, s).
IR (KBr): 3140, 3050, 2940, 2860, 1610, 1599, 1586, 1487, 1451, 1415, 1327, 1262, 1169, 1125, 1113, 1069, 1017 $cm^{-1}$.
Anal calcd for $C_{24}H_{21}N_4O_2F_3$: C, 63.43; H, 4.66; N, 12.33.
Found: C, 63.36; H, 4.73; N, 12.26.

Reference Example B7

1-{4-[4-({2-[(E)-2-(2,4-Difluorophenyl)ethenyl]-1,3-oxazol-4-yl}methoxy)phenyl]butyl}-1H-1,2,3-triazole

Using 4-[4-(1H-1,2,3-triazole-1-yl)butyl]phenol (152 mg), 65% oily sodium hydride (28 mg) and 4-(chloromethyl)-2-[(E)-2-(2,4-difluorophenyl)ethenyl]-1,3-oxazole (188 mg), the same reaction as in Reference Example B2 was conducted to obtain the title compound (254 mg).
mp 115-117°C.
$^1$H-NMR ($CDCl_3$) δ: 1.62 (2H, quintet, J = 7.2Hz), 1.94 (2H, quintet, J = 7.5Hz), 2.60 (2H, t, J = 7.5Hz), 4.39 (2H, t, J = 7.1Hz), 5.01 (2H, s), 6.81-6.98 (2H, m), 6.91 (2H, d, J = 8.6Hz), 6.98 (1H, d , J=16 . 2Hz ) , 7.07 (2H, d, J = 8.6Hz), 7.47-7.53 (1H, m), 7.50 (1H, s) , 7.59 (1H, d, J = 16.2Hz), 7.67 (1H, s), 7.70 (1H, s).
IR (KBr): 3133, 2932, 2863, 1644, 1615, 1590, 1532, 1514, 1493, 1468, 1431, 1345, 1298, 1279, 1246, 1215, 1179, 1140, 1086, 1049, 1032 $cm^{-1}$.
Anal calcd for $C_{24}H_{22}N_4O_2F_2$ : C, 66.05; H, 5.08; N, 12.84.
Found: C, 66.03; H, 5.00; N,13.03.

Reference Example B8

1-(3-[3-({2-[(E)-2-(2,4-Difluorophenyl)ethenyl]-1,3-oxazol-4-yl}methoxy)phenyl]propyl}-1H-1,2,3-triazole

Using 3-[3-(1H-1,2,3-triazole-1-yl)propyl]phenol (143 mg), 65% oily sodium hydride (28 mg) and 4-(chloromethyl)-2-[(E)-2-(2,4-difluorophenyl)ethenyl]-1,3-oxazole (188 mg), the same reaction as in Reference Example B2 was conducted to obtain the title compound (257 mg).

mp 89-90°C.
$^1$H-NMR ($CDCl_3$) δ: 2.26 (2H, quintet, J = 7.3Hz), 2.64 (2H, t, J = 7.4Hz), 4.39 (2H, t, J = 7.1Hz), 5.03 (2H, s), 6.77-6.98 (5H, m), 6.98 (1H, d, J = 16.8Hz), 7.24 (1H, t, J = 7.6Hz), 7.47-7.60 (1H, m), 7.52 (1H, s), 7.59 (1H, d, J = 16.8Hz), 7.68 (1H, s), 7.71 (1H, s).
IR (KBr): 3127, 3071, 2934, 2868, 1644, 1615, 1599, 1534, 1495, 1453, 1433, 1354, 1273, 1215, 1159, 1142, 1090, 1028 $cm^{-1}$.
Anal calcd for $C_{23}H_{20}N_4O_2F_2$: C, 65.39; H, 4.77; N, 13.26.
Found: C, 65.32; H, 4.56; N, 13.34.

Reference Example B9

[1-[4-[4-[[2-[(E)-2-(2,6-Difluorophenyl)ethenyl]-1,3-oxazol-4-yl]methoxy]phenyl]butyl]-1H-1,2,3-triazole

To a DMF (4 mL) solution of 4-[4-(1H-1,2,3-triazole-1-yl)butyl]phenol (217 mg), 65% oily sodium hydride (41 mg) was added with ice-cooling. After stirring at room temperature for 30 minutes, 4-(chloromethyl)-2-[(E)-2-(2,6-difluorophenyl)ethenyl]-1,3-oxazole (281 mg) was added with ice-cooling, followed by stirring at room temperature overnight. Water was added thereto with ice-cooling and the deposit was collected by filtration, washed with water and then dissolved in THF-ethyl acetate. The solution was washed with water and brine, dried over magnesium sulfate and then concentrated under reduced pressure. The residue was recrystallized from ethyl acetate-hexane to obtain the title compound (348 mg) as colorless crystals.
$^1$ H-NMR ($CDCl_3$) δ: 1.5-1.7 (2H, m) , 1.85-2.05 (2H, m), 2.60 (2H, t, J = 7.4Hz), 4.39 (2H, t, J = 7.2Hz), 5.02 (2H, s), 6.92 (2H, d, J = 8.8Hz), 6.94 (1H, d, J = 17.4Hz), 6.85-7.35 (3H, m), 7.07 (2H, d, J = 8.8Hz), 7.61 (1H, d, J = 17.4Hz), 7.45-7.7 (3H, m).
IR (KBr): 1620, 1586, 1514, 1464, 1244, 1024, 999, 968, 783 $cm^{-1}$.
Anal. Calcd for $C_{24}H_{22}F_2N_4O_2$: C, 66.05; H, 5.08; N, 12.84.
Found: C, 65.83; H, 5.06; N, 12.93.

Reference Example B10

2-[1-[4-[4-[[2-[(E)-2-(4-Methylphenyl)ethenyl]-1,3-oxazol-4-yl]methoxy]phenyl]butyl]-1H-imidazol-2-yl]-1-ethanol

Using 4-[4-[2-(2-hydroxyethyl)-1H-imidazol-1-yl]butyl]phenol (260 mg) and (E)-4-chloromethyl-2-[2-(4-methylphenyl)ethenyl]oxazole (257 mg), the same reaction as in Reference Example B1 was conducted to obtain the title compound (331 mg) as colorless crystals.
mp 108-109°C.
$^1$H-NMR ($CDCl_3$) δ: 1.5-1.8 (4H, m), 2.38 (3H, s), 2.58 (2H, t, J = 7.0Hz), 2.79 (2H, t, J = 5.6Hz), 3.82 (2H, t, J = 6.8Hz), 4.03 (2H, t, J = 5.6Hz), 5.01 (2H, s), 6.8-6.85 (2H, m), 6.89 (1H, d, J = 16.6Hz), 6.92 (2H, d, J = 8.6Hz), 7.07 (2H, d, J = 8.6Hz), 7.19 (2H, d, J = 7.8Hz), 7.43 (2H, d, J = 7.8Hz), 7.51 (1H, d, J = 16.6Hz), 7.64 (1H, s).
IR (KBr): 1510, 1240, 1055, 806 $cm^{-1}$.
Anal. Calcd for $C_{28}H_{31}N_3O_3$: C, 73.50; H, 6.83; N, 9.18.
Found: C, 73.36; H, 6.66; N, 9.12.

Reference Example B11

2-[1-[4-[4-[[2-[(E)-2-(3-Methylphenyl)ethenyl] -1,3-oxazol-4-yl]methoxy]phenyl]butyl]-1H-imidazol-2-yl]-1-ethanol

Using 4-[4-[2-(2-hydroxyethyl)-1H-imidazol-1-yl]butyl]phenol (260 mg) and (E)-4-chloromethyl-2-[2-(3-methylphenyl)ethenyl]oxazole (257 mg), the same reaction as in Reference Example B1 was conducted to obtain the title compound (290 mg) as colorless crystals.
mp 109-111°C.
$^1$H-NMR ($CDCl_3$) δ: 1.55-1.8 (4H, m), 2.38 (3H, s), 2.58 (2H, t, J = 7.0Hz), 2.78 (2H, t, J = 5.6Hz), 3.82 (2H, t, J = 7.0Hz), 4.03 (2H, t, J = 5.6Hz), 5.01 (2H, s), 6.80 (1H, d, J = 1.4Hz), 6.92 (1H, d, J = 16.6Hz), 6.92 (2H, d, J = 8.8Hz), 6.93 (1H, d, J = 1.4Hz), 7.07 (2H, d, J = 8.8Hz), 7.1-7.2 (1H, m), 7.2-7.4 (3H, m), 7.51 (1H, d, J = 16.6Hz), 7.65 (1H, s).
IR (KBr): 1514, 1460, 1250, 1051, 976, 828, 789 $cm^{-1}$.
Anal. Calcd for $C_{28}H_{31}N_3O_3 \cdot 0.2H_2O$: C, 72.92; H, 6.86; N, 9.11.
Found: C, 72.71.; H, 6.74; N, 8.97.

Reference Example B12

2-[1-[4-[4-[[2-[(E)-2-(2-Methylphenyl)ethenyl]-1,3-oxazol-4-yl]methoxy]phenyl]butyl]-1H-imidazol-2-yl]-1-ethanol

Using 4-[4-[2-(2-hydroxyethyl)-1H-imidazol-1-yl]butyl]phenol (153 mg) and (E)-4-chloromethyl-2-[2-(2-methylphenyl)ethenyl]oxazole (151 mg), the same reaction as in Reference Example B1 was conducted to obtain the title compound (167 mg) as colorless crystals.
mp 91-93°C (ethyl acetate-hexane).
$^1$H-NMR ($CDCl_3$) δ: 1.5-1.8 (4H, m), 2.46 (3H, s), 2.59 (2H, t, J = 7.0Hz), 2.79 (2H, t, J = 5.6Hz), 3.82 (2H, t, J = 7.0Hz), 4.03 (2H, t, J = 5.6Hz), 5.02 (2H, s) , 6.8-6.9 (3H, m), 6.92 (2H, d, J = 8.6Hz), 7.07 (2H, d, J = 8.6Hz), 7.2-7.3 (3H, m), 7.55-7.65 (1H, m), 7.66 (1H, s), 7.79 (1H, d, J = 16.2Hz).
IR (KBr): 1508, 1464, 1231, 1061, 1009, 862, 752 $cm^{-1}$.
Anal. Calcd for $C_{28}H_{31}N_3O_3 \cdot 0.2H_2O$ : C, 72.92; H, 6.86; N, 9.11.
Found: C, 72.98; H, 6.70; N, 9.23.

Reference Example B13

2-[1-[4-[4-[[2-[(E)-2-(4-Ethylphenyl)ethenyl]-1,3-oxazol-4-yl]methoxy]phenyl]butyl]-1H-imidazol-2-yl]-1-ethanol

To a DMF (4 mL) solution of [4-[2-(2-hydroxyethyl)-1H-imidazol-1-yl]butyl]phenol (260 mg), 60% oily sodium hydride (44 mg) was added with ice-cooling. After stirring at room temperature for 30 minutes, (E)-4-chloromethyl-2-[2-(4-ethylphenyl)ethenyl]oxazole (272 mg) was added thereto with ice-cooling. After stirring at room temperature overnight, water was added thereto with ice-cooling. The deposit was collected by filtration and washed with water. The deposit was dissolved in ethyl acetate and the solution was dried over (magnesium sulfate) and then concentrated under reduced pressure. The residue was recrystallized from ethyl acetate-hexane to obtain the title compound (297 mg) as colorless crystals.
mp 94-95°C.
$^1$H-NMR ($CDCl_3$) δ: 1.25 (3H, t, J = 7.4Hz), 1.5-1.85 (4H, m), 2.59 (2H, t, J = 7.0Hz), 2.67 (2H, q, J = 7.4Hz), 2.79 (2H, t, J = 5.4Hz), 3.82 (2H, t, J = 7.0Hz), 4.04 (2H, t, J = 5.4), 5.01 (2H, s), 6.8-7.0 (3H, m), 6.92 (2H, d, J = 8.4Hz), 7.07 (2H, d, J = 8.4Hz), 7.2-7.3 (2H, m), 7.4-7.5 (2H, m), 7.53 (1H, d, J = 17.2Hz), 7.65 (1H, s).
IR (KBr): 1508, 1462, 1231, 1181, 1061, 1007, 864, 833 $cm^{-1}$. Anal. Calcd for $C_{29}H_{33}N_3O_3$: C, 73.86; H, 7.05; N, 8.91.
Found: C, 73.73; H, 6.79; N, 8.76.

Reference Example B14

2-(1-{4-[4-({2-[(E)-2-(4-Fluorophenyl)ethenyl]-1,3-oxazol-4-yl}methoxy)phenyl]butyl}-1H-imidazol-2-yl)-1-ethanol

Using 4-[4-[2-(2-hydroxyethyl)-1H-imidazol-1-yl]butyl]phenol (391 mg), 65% oily sodium hydride(60 mg) and 4-(chloromethyl)-2-[(E)-2-(4-fluorophenyl)ethenyl]-1,3-oxazole (375 mg), the same reaction as in Reference Example B2 was conducted to obtain the title compound (583 mg).
mp 130-132°C.
$^1$H-NMR ($CDCl_3$) δ: 1.56-1.84 (4H, m), 2.10-2.90 (1H, br), 2.58 (2H, t, J = 7.1Hz), 2.78 (2H, t, J = 5.5Hz), 3.82 (2H, t, J = 7.1Hz), 4.03 (2H, t, J = 5.5Hz), 5.01 (2H, s), 6.80-6.94 (5H, m), 7.04-7.13 (4H, m), 7.46-7.55 (3H, m), 7.65 (1H, s).
IR (KBr): 3150, 3113, 3048, 2936, 2861, 1642, 1599, 1582, 1532, 1512, 1464, 1422, 1399, 1375, 1337, 1302, 1277, 1246, 1229, 1209, 1177, 1159, 1148, 1105, 1051, 1001 $cm^{-1}$.
Anal calcd for $C_{27}H_{28}N_3O_3F$: C, 70.26; H, 6.11; N, 9.10.
Found: C, 70.15; H, 6.06; N, 9.35.

Reference Example B15

2-[1-[4-[4-[[2-[(E)-2-(4-Chlorophenyl)ethenyl]-1,3-oxazol-4-yl]methoxy]phenyl]butyl]-1H-imidazol-2-yl] -1-ethanol

To a DMF (4 mL) solution of 4-[4-[2-(2-hydroxyethyl)-1H-imidazol-1-yl]butyl]phenol (130 mg), 60% oily sodium hydride (22 mg) was added with ice-cooling. After stirring at room temperature for 30 minutes, (E)-4-chloromethyl-2-[2-(4-chlorophenyl)ethenyl]oxazole (140 mg) was added thereto with ice-cooling. After stirring at 0°C for 1 hour and stirring at room temperature overnight, water was added thereto with ice-cooling. The deposit was collected by filtration, washed with water and then dissolved in a mixture of THF and ethyl acetate. The resulting solution was dried over magnesium sulfate and then concentrated under reduced pressure. The residue was recrystallized from methanol-ethyl acetate-diethyl ether to obtain the title compound (168 mg) as colorless crystals.
mp 127-128°C.
$^1$H-NMR ($CDCl_3$) δ: 1.5-1.8 (4H, m), 2.58 (2H, t, J = 7.0Hz), 2.78 (2H, t, J = 5.4Hz), 3.82 (2H, t, J = 7.0Hz), 4.03 (2H, t, J = 5.4Hz), 5.01 (2H, s), 6.8-7.0 (5H, m), 7.07 (2H, d, J = 8.8Hz), 7.35 (2H, d, J = 8.4Hz), 7.46 (2H, d, J = 8.4Hz), 7.4-7.55 (1H, m), 7.66 (1H, s).
IR (KBr): 1514, 1474, 1341, 1264, 1246, 1076, 966, 814 $cm^{-1}$. Anal. Calcd for $C_{27}H_{28}C1N_3O_3$: C, 67.85; H, 5.90; N, 8.79. Found: C, 67.85; H, 5.72; N, 9.09.

Reference Example B16

2-[1-[4-[4-[[2-[(E)-2-(4-Bromophenyl)ethenyl]-1,3-oxazol-4-yl]methoxy]phenyl]butyl]-1H-imidazol-2-yl]-1-ethanol

To DMF(10 mL) solution of 2-(1H-imidazol-2-yl)-ethanol (449 mg), 60% oily-sodium hydride (176 mg) was added with ice-cooling. After stirring at room temperature for 30 minutes, 4-[[4-(4-iodobutyl)phenoxy)methyl]-2-[(E)-2-(4-bromophenyl)ethenyl]-1,3-oxazole (2.15 g) was added with ice-cooling. After stirring at room temperature overnight, water was added with ice-cooling. The reaction mixture was extracted with a mixture of ethyl acetate and THF, dried over magnesium sulfate and then concentrated under reduced pressure. The residue was recrystallized from ethyl acetate-hexane to obtain the title compound (2.09 g) as pale yellow crystals.
mp 149-150°C.
$^1$H-NMR($CDCl_3$) δ: 1.55-1.8 (4H, m), 2.58 (2H, t, J = 7.0Hz), 2.78 (2H, t, J = 5.6Hz), 3.82 (2H, t, J = 7.0Hz), 4.03 (2H, t, J = 5.6Hz), 5.01 (2H, s), 6.91 (2H, d, J = 8.8Hz), 6.92 (1H, d, J = 16.3Hz), 6.8-7.0 (2H, m), 7.07 (2H, d, J = 8.8Hz), 7.38 (2H, d, J = 8.6Hz), 7.47 (1H, d, J = 16.3Hz), 7.52 (2H, d, J = 8.6Hz), 7.66 (1H, s).
IR (KBr): 1514, 1487, 1254, 1055, 972, 826, 814 $cm^{-1}$.
Anal. Calcd for $C_{27}H_{28}BrN_3O_3$: C, 62.07; H, 5.40; N, 8.04.
Found: C, 61.82; H, 5.26; N, 7.90.

Reference Example B17

2-[1-[4-[4-[2-[ (E)-2-(4-Trifluoromethylphenyl)ethenyl]oxazol-4-yl]methoxyphenyl]butyl-1H-imidazol-2-yl]-1-ethanol

In an argon atmosphere, DMF(4 mL) was added to 65% sodium hydride (40.6 mg) and 4-[4-[2-(2-hydroxyethyl)-1H-imidazol-1-yl]butyl]phenol (260 mg) at 0°C. After stirring at room temperature for 30 minutes, [2-[(E)-2-(4-trifluoromethylphenyl)ethenyl]oxazol-4-yl]methylchloride (316 mg) was added thereto at 0°C, followed by stirring at room temperature for 15 hours. Water was added to the reaction mixture and the deposited crystals were collected by filtration, washed with water and isopropyl ether, and then recrystallized from acetone-hexane to obtain the title compound (393 mg) as pale yellow needles.
$^1$H-NMR ($CDCl_3$) 5: 1.56-1.74 (4H, m), 2.59 (2H, t, J = 6.6 Hz), 2.78 (2H, t, J = 5.4 Hz), 3.82 (2H, t, J = 6.8 Hz), 4.03 (2H, t, J = 5.4 Hz), 5.02 (2H, d, J = 1.2 Hz), 6.81 (1H, d, J = 1.6 Hz), 6.90-6.95 (4H, m), 7.02 (2H, d, J = 16.2 Hz), 7.52-7.69 (6H, m).
IR (KBr): 1512, 1323, 1244, 1175, 1132, 1113, 1067, 1055 $cm^{-1}$.

Reference Example B18

2-[1-[3-[4-[2-[(E)-2-(4-Trifluoromethylphenyl)ethenyl]oxazol-4-yl]methoxyphenyl]propyl] -1H-imidazol-2-yl]-1-ethanol

Using 65% sodium hydride (40.6 mg), 4-[3-[2-(hydroxyethyl)-1H-imidazol-1-yl]propyl]phenol (246 mg) and [2-[(E)-2-(4-trifluoromethylphenyl)ethenyl]oxazol-4-yl]methylchloride (316 mg), the same reaction as in Reference Example B17 was conducted to obtain the title compound (330 mg) as colorless needles.
$^1$H-NMR ($CDCl_3$) δ: 2.01-2.08 (2H, m), 2.60 (2H, t, J = 7.8 Hz), 2.74 (2H, t, J = 5.8 Hz), 3.83 (2H, t, J = 7.4 Hz), 4.03 (2H, t, J = 5.8 Hz), 5.03 (2H, s), 6.84 (1H, d, J = 1.2 Hz), 6.96-7.12 (6H, m), 7.52-7.70 (6H, m) .
IR (KBr): 1512, 1327, 1246, 1173, 1125, 1069, 1017, 826 $cm^{-1}$.

Reference Example B19

2-[1-[4-[4-[[2-[(E)-2-(2,4-Difluorophenyl)ethenyl]-1,3-oxazol-4-yl]methoxy]phenyl]butyl]-1H-imidazol-2-yl]-1-ethanol

To a DMF(4 mL) solution of 4-[4-[2-(2-hydroxyethyl)-1H-imidazol-1-yl]butyl]phenol (260 mg), 60% oily sodium hydride (44 mg) was added with ice-cooling. After stirring at room temperature for 30 minutes, (E)-4-chloromethyl-2-[2-(2,4-difluorophenyl)ethenyl]oxazole (281 mg) was added thereto with ice-cooling. After stirring at room temperature for 3 days, water was added thereto with ice-cooling. The deposit was collected by filtration and then washed with water. The deposit was dissolved in a mixture of ethyl acetate and THF and the solution was dried over magnesium sulfate and then concentrated under reduced pressure. The residue was recrystallized from ethyl acetate-hexane to obtain the title compound (275 mg) as pale yellow crystals.
mp 93-95°C.
$^1$H-NMR ($CDCl_3$) δ: 1.55-1.85 (4H, m), 2.58 (2H, t, J = 7.0Hz), 2.78 (2H, t, J = 5.4Hz), 3.82 (2H, t, J = 7.0Hz), 4.03 (2H, t, J = 5.4Hz), 5.01 (2H, s), 6.8-7.0 (6H, m), 6.98 (1H, d, J = 16.3Hz), 7.07 (2H, d, J = 8.8Hz), 7.5-7.6 (1H, m), 7.59 (1H, d, J = 16.3Hz), 7.67 (1H, s).
IR (KBr): 1611, 1508, 1277, 1231, 1140, 1103, 1063, 970, 860 $cm^{-1}$.
Anal. Calcd for $C_{27}H_{27}F_2N_3O_3 \cdot 0.1H_2O$: C, 67.38; H, 5.70; N, 8.73.
Found: C, 67.24; H, 5.74; N, 8.55.

Reference Example B20

2-[1-[3-[4-[[2-[(E)-2-(2,4-Difluorophenyl)ethenyl]-1,3-oxazol-4-yl]methoxy]phenyl]propyl]-1H-imidazol-2-yl]-1-ethanol

To a DMF (4 mL) solution of 4-[4-[2-(2-hydroxyethyl)-1H-imidazol-1-yl]propyl]phenol (246 mg), 60% oily sodium hydride (44 mg) was added with ice-cooling. After stirring at room temperature for 30 minutes, (E)-4-chloromethyl-2-[2-(2,4-difluorophenyl)ethenyl]oxazole (281 mg) was added thereto with ice-cooling. After stirring at room temperature overnight, water was added thereto with ice-cooling. The deposit was collected by filtration and then washed with water. The deposit was dissolved in ethyl acetate and the solution was dried over magnesium sulfate and then concentrated under reduced pressure. The residue was recrystallized from ethyl acetate-diethyl ether-hexane to obtain the title compound (272 mg) as colorless crystals. mp 94-96°C.
$^1$H-NMR ($CDCl_3$ ) δ: 1.95-2.15 (2H, m), 2.5-2.65 (2H, m), 2.65-2.8 (2H, m), 3.75-3.9 (2H,m), 3.95-4.1 (2H, m), 5.02 (2H, s), 6.8-7.15 (9H, m), 7.45-7.7 (3H, m).
IR (KBr): 1609, 1512, 1277, 1231, 1140, 1061, 1020, 974, 860 $cm^{-1}$.
Anal. Calcd for $C_{26}H_{25}F_2N_3O_3 \cdot 0.4H_2O$: C, 66.06; H, 5.50; N, 8.89.
Found: C, 66.13; H, 5.38; N, 8.55.

Reference Example B21

2-[1-[3-[4-[[2-[(E)-2-(2,6-Difluorophenyl)ethenyl]-1,3-oxazol-4-yl]methoxy]phenyl]propyl]-1H-imidazol-2-yl]-1-ethanol

Using 2-(2-hydroxyethyl)-1-[4-(4-hydroxyphenyl)butyl]imidazole (260 mg), 60% oily sodium hydride (41 mg) and (E)-4-chloromethyl-2-[2-(2, 6-difluorophenyl)ethenyl]oxazole (281 mg), the same reaction as in Reference Example B19 was conducted to obtain the title compound (359 mg) as colorless crystals.
mp 106-107°C.
$^1$H-NMR ($CDCl_3$) δ: 1.5-1.8 (4H, m), 2.58 (2H, t, J = 7.0Hz), 2.78 (2H, t, J = 5.6Hz), 3.82 (2H, t, J = 7.0Hz), 4.03 (2H, t, J = 5.6Hz), 5.02 (2H, s), 6.8-7.0 (6H, m), 7.07 (2H, d, J = 8.4Hz), 7.2-7.35 (2H, m), 7.61 (1H, d, J = 16.8Hz), 7.68 (1H, s).
IR (KBr): 1618, 1516, 1472, 1456, 1246, 1065, 1001, 974, 789 $cm^{-1}$.
Anal. Calcd for $C_{27}H_{27}F_2N_3O_3$: C, 67.63; H, 5.68; N, 8.76.
Found: C, 67.78; H, 5.57; N, 9.01.

Reference Example B22

3-(1-{4-[4-({2-[(E)-2-(3-Methylphenyl) ethenyl]-1,3-oxazol-4-yl}methoxy)phenyl]butyl}-1H-imidazol-2-yl)-1,2-propanediol

Using 3-{1-[4-(4-hydroxyphenyl)butyl]-1H-imidazol-2-yl}-1,2-propanediol (154 mg), 65% oily sodium hydride (21 mg) and 4-(chloromethyl)-2-[(E)-2-(3-methylphenyl)ethenyl]-1,3-oxazole (131 mg), the same reaction as in Reference Example B2 was conducted to obtain the title compound (156 mg).
mp 102-104°C.
$^1$H-NMR ($CDCl_3$) δ: 1.52-1.82 (4H, m), 2.39 (3H, s), 2.59 (2H, t, J = 7.0Hz), 2.77 (1H, d, J = 5.0Hz), 2.78 (1H, d, J = 6.8Hz), 3.64 (1H, dd, J = 4.8Hz, 11.2Hz), 3.76 (1H, dd, J = 4.2Hz, 11.2Hz), 3.82 (2H, t, J = 7.0Hz), 4.12-4.24 (1H, m), 5.02 (2H, s), 6.80 (1H, d, J = 1.4Hz), 6.92 (1H, d, J = 1.4Hz), 6.93 (1H, d, J = 16.2Hz), 6.93 (1H, d, J = 8.8Hz), 7.08 (2H, d, J = 8.8Hz), 7.13-7.39 (4H, m), 7.52 (1H, d, J - 16.2Hz), 7.66 (1H, s).
IR (KBr): 3500-3200, 3112, 3029, 2934, 2865, 1645, 1609, 1584, 1510, 1491, 1462, 1379, 1350, 1242, 1177, 1150,

1123, 1100, 1026 $cm^{-1}$.
Anal calcd for $C_{29}H_{33}N_3O_4 \cdot 0.5H_2O$ : C, 70.14; H, 6.90; N, 8.46.
Found: C, 70.39; H, 6.63; N, 8.51.

Reference Example B23

3-(1-{4-[4-({2-[(E)-2-(4-Fluorophenyl) ethenyl]-1,3-oxazol-4-yl}methoxy)phenyl]butyl}-1H-imidazol-2-yl)-1,2-propanediol

Using 3-{1-[4-(4-hydroxyphenyl)butyl]-1H-imidazol-2-yl}-1,2-propanediol (291 mg), 65% oily sodium hydride (39 mg) and 4-(chloromethyl)-2-[(E)-2-(4-fluorophenyl)ethenyl]-1,3-oxazole (250 mg), the same reaction as in Reference Example B2 was conducted to obtain the title compound (347 mg).
mp 114-116°C.
$^1$H-NMR ($CDCl_3$) δ: 1.52-1.83 (4H, m), 2.59 (2H, t, J = 7.2Hz), 2.76 (1H, d, J = 5.2Hz), 2.77 (1H, d, J = 7.0Hz), 3.64 (1H, dd, J = 4.8Hz, 11.4Hz), 3.76 (1H, dd, J = 4.2Hz, 11.4Hz), 3.82 (2H, t, J = 6.8Hz), 4.12-4.24 (1H, m), 5.01 (2H, s), 6.80 (1H, d, J = 1.4Hz), 6.86 (1H, d, J = 16.8Hz), 6. 92 (1H, d, J=1. 4Hz) , 6.93(.2H, d, J = 8.8Hz), 7.07 (2H, d, J = 8.8Hz), 7.09 (2H, d, J = 8.7Hz), 7.46-7.56 (3H, m), 7.66 (1H, s).
IR (KBr): 3500-3200, 3152, 3104, 3044, 2940, 2865, 1644, 1599, 1584, 1532, 1512, 1495, 1462, 1422, 1400, 1339, 1300, 1246, 1177, 1159, 1098, 1047 $cm^{-1}$.
Anal calcd for $C_{28}H_{30}N_3O_4F$: C, 68.42; H, 6.15; N, 8.55.
Found: C, 68.16; H, 5.98; N, 8.46.

Reference Example B24

3-[1-(4-{4-[(2-{ (E)-2-[4-(Trifluoromethyl)phenyl]ethenyl}-1,3-oxazol-4-yl)methoxy]phenyl}butyl)-1H-imidazol-2-yl]-1,2-propanediol

Using 3-{1-[4-(4-hydroxyphenyl)butyl]-1H-imidazol-2-yl}-1,2-propanediol (204 mg), 65% oily sodium hydride (28 mg) and 4-(chloromethyl)-2-{ (E)-2- [4-(trifluoromethyl)phenyl]ethenyl}-1,3-oxazole (212 mg), the same reaction as in Reference Example B2 was conducted to obtain the title compound (285 mg).
mp 142-143°C.
$^1$H-NMR ($CDCl_3$) δ: 1.53-1.82 (4H, m), 2.59 (2H, t, J = 7.1Hz), 2.76 (1H, d, J = 5.0Hz), 2.77 (1H, d, J = 7.0Hz), 3.64 (1H, dd, J = 4.8Hz, 11.4Hz), 3.76 (1H, dd, J = 4.2Hz, 11.4Hz), 3.83 (2H, t, J = 6.8Hz), 4.12-4.24 (1H, m), 5.02 (2H, s), 6.81 (1H, d, J = 1.4Hz), 6.92 (1H, d, J = 1.4Hz), 6.93 (2H, d, J = 8.8Hz), 6.95 (1H, d, J = 16.4Hz), 7.08 (2H, d, J = 8.8Hz), 7.56 (1H, d, J = 16.4Hz), 7.64 (4H, s), 7.70 (1H, s).
IR (KBr): 3500-3200, 3148, 3071, 2936, 2867, 1642, 1615, 1582, 1510, 1491, 1466, 1416, 1397, 1323, 1246, 1173, 1138, 1117, 1067, 1046, 1017 $cm^{-1}$.
Anal calcd for $C_{29}H_{30}N_3O_4F_3$: C, 64.32; H, 5.58; N, 7.76.
Found: C, 64.26; H, 5.70; N, 7.62.

Reference Example B25

3-[1-(3-{3-[(2-{ (E)-2-[4-(Trifluoromethyl)phenyl]ethenyl}-1,3-oxazol-4-y1)methoxy]phenyl}propyl)-1H-imidazol-2-yl]-1,2-propanediol

Using 3-{1-[3- (3-hydroxyphenyl)propyl]-1H-imidazol-2-yl}-1,2-propanediol (194 mg), 65% oily sodium hydride (28 mg) and 4-(chloromethyl)-2-{(E)-2-[4-(trifluoromethyl)phenyl]ethenyl}-1,3-oxazole (212 mg), the same reaction as in Reference Example B2 was conducted to obtain the title compound (255 mg).
mp 102-104°C.
$^1$H-NMR ($CDCl_3$) δ: 2.08 (2H, quintet, J = 7.0Hz), 2.62 (2H, t, J = 7.4Hz), 2.72 (1H, d, J = 4.8Hz), 2.73 (1H, d, J = 7.6Hz), 3.63 (1H, dd, J = 4.8Hz, 11.4Hz), 3.74 (1H, dd, J = 4.2Hz, 11.4Hz), 3.83 (2H, t, J = 7.2Hz), 4.13-4.24 (1H, m), 5.03 (2H, s), 6.77-6.91 (3H, m), 6.84 (1H, d, J = 1.4Hz), 6.94 (1H, d, J = 1.4Hz), 7.02 (1H, d, J = 16.4Hz), 7.25 (1H, t, J = 7.8Hz), 7.57 (1H, d, J = 16.4Hz), 7.64 (4H, s), 7.71 (1H, s).
IR (KBr): 3500-3200, 3108, 3056, 2932, 2867, 1613, 1599, 1586, 1534, 1489, 1451, 1416, 1325, 1260, 1167, 1125, 1069, 1030, 1017 $cm^{-1}$ .
Anal calcd for $C_{28}H_{28}N_3O_4F_3$: C, 63.75; H, 5.35; N, 7.97.
Found: C, 63.60; H, 5.32; N, 7.88.

Reference Example B26

3-(1-{4-[4-({2-[(E) -2-(2,4-Difluorophenyl)ethenyl]-1,3-oxazol-4-yl}methoxy)phenyl]butyl}-1H-imidazol-2-yl)-1,2-propanediol

Using 3-{1-[4-(4-hydroxyphenyl)butyl]-1H-imidazol-2-yl}-1,2-propanediol (204 mg), 65% oily sodium hydride (28 mg) and 4-(chloromethyl)-2-[(E)-2-(2,4-difluorophenyl)ethenyl]-1,3-oxazole (188 mg), the same reaction as in Reference Example B2 was conducted to obtain the title compound (223 mg).
mp 126-128°C.
$^1$H-NMR ($CDCl_3$) δ: 1.52-1.81 (4H, m), 2.58 (2H, t, J = 6.9Hz), 2.77 (2H, d, J = 5.4Hz), 3.63 (1H, dd, J = 4.8Hz, 11.4Hz), 3.75 (1H, dd, J = 4.2Hz, 11.4Hz), 3.82 (2H, t, J = 7.0Hz), 4.10-4.24 (1H, m), 5.01 (2H, s), 6.76-7.02 (7H, m), 7.07 (2H, d, J = 8.6Hz), 7.48-7.51 (1H, m), 7.59 (1H, d, J = 16.6Hz), 7.67 (1H, s).
IR (KBr): 3500-3200, 3106, 3073, 3032, 2934, 2865, 1644, 1613, 1593, 1532, 1512, 1495, 1462, 1431, 1354, 1298, 1275, 1244, 1177, 1142, 1090, 1028 $cm^{-1}$.
Anal calcd for $C_{28}H_{29}N_3O_4F_2$: C, 66.00; H, 5.74; N, 8.25.
Found: C, 65.89; H, 5.94; N, 8.37.

Reference Example B27

3-(1-{3-[3-({2-[(E)-2-(2,4-Difluorophenyl)ethenyl]-1,3-oxazol-4-yl}methoxy)phenyl]propyl}-1H-imidazol-2-yl)-1,2-propanediol

Using 3-{1-[3-(3-hydroxyphenyl)propyl]-1H-imidazol-2-yl}-1,2-propanediol (203 mg), 65% oily sodium hydride (29 mg) and 4-(chloromethyl)-2-[(E)-2-(2,4-difluorophenyl)ethenyl]-1,3-oxazole (197 mg), the same reaction as in Reference Example B2 was conducted to obtain the title compound (220 mg).
mp 92-94°C.
$^1$H-NMR ($CDCl_3$) δ: 2.08 (2H, quintet, J = 7.2Hz), 2.62 (2H, t, J = 7.3Hz), 2.73 (1H, d, J = 5.0Hz), 2.74 (1H, d, J = 7.0Hz), 3.63 (1H, dd, J = 4.8Hz, 11.2Hz), 3.74 (1H, dd, J = 4.2Hz, 11.2Hz), 3.83 (2H, t, J = 7.4Hz), 4.14-4.24 (1H, m), 5.02 (2H, s), 6.76-6.98 (5H, m), 6.84 (1H, d, J = 1.4Hz), 6.93 (1H, d, J = 1.4Hz), 6.98 (1H, d, J = 16.4Hz), 7.25 (1H, t, J = 7.9Hz), 7.48-7.61 (1H, m), 7.60 (1H, d, J = 16.4Hz), 7.69 (1H, s).
IR (KBr): 3500-3200, 3106, 3067, 3042, 2938, 2872, 1644, 1613, 1599, 1534, 1495, 1453, 1431, 1379, 1354, 1275, 1155, 1142, 1123, 1090, 1028 $cm^{-1}$.
Anal calcd for $C_{27}H_{27}N_3O_4F_2$: C, 65.44; H, 5.49; N, 8.48.
Found: C, 65.39; H, 5.32; N, 8.62.

Reference Example B28

3-[1-[4-[4-[[2-[(E)-2-(2,6-Difluorophenyl)ethenyl]-1,3-oxazol-4-yl]methoxy]phenyl]butyl]-1H-imidazol-2-yl]-1,2-propanediol

Using 3-{1-[3-(3-hydroxyphenyl)propyl]-1H-imidazol-2-yl}-1,2-propanediol (142 mg), 60% oily sodium hydride (40 mg) and 4-(chloromethyl)-2-[(E)-2-(2,6-difluorophenyl)ethenyl]-1,3-oxazole (495 mg), the same reaction as in Reference Example B2 was conducted to obtain the title compound (395 mg) as colorless crystals.
mp 123-125°C.
$^1$H-NMR ($CDCl_3$) δ: 1.5-1.8 (4H, m), 2.59 (2H, t, J = 7.0), 2.7-2.8 (2H, m), 3.6-3.75 (2H, m), 3.83 (2H, t, J = 7.0Hz), 4.1-4.25 (1H, m), 5.03 (2H, s), 6.8-7.0 (4H, m), 6.92 (2H, d, J = 8.6Hz), 7.07 (2H, d, J = 8.6Hz), 7.2-7.3 (1H, m), 7.29 (1H, d, J = 16.8Hz), 7.61 (1H, d, J = 16.8Hz), 7.69 (1H, s).
IR (KBr) : 1620, 1508, 1458, 1236, 1051, 1001, 789 $cm^{-1}$.
Anal. Calcd for $C_{28}H_{29}F_2N_3O_4$: C, 66.00; H, 5.74; N, 8.25.
Found: C, 65.71; H, 5.78; N, 8.09.

Reference Example B29

(2R)-3-[[1-[4-[4-[[2-[(E)-2-(2,4-Difluorophenyl)ethenyl]-1,3-oxazol-4-yl]methoxy]phenyl]butyl]-1H-imidazol-2-yl]-1,2-propanediol

To a DMF (4 mL) solution of (2R)-3-(1H-imidazol-2-yl)-1,2-propanediol (127 mg), 60% oily sodium hydride (37 mg) was added with ice-cooling. After stirring at room temperature for 30 minutes, 4-[[4-(4-iodobutyl)phenoxy]

methyl]-2-[(E)-2-(2,4-difluorophenyl)ethenyl]-1,3-oxazole (485 mg) was added thereto with ice-cooling. After stirring at room temperature for 3 hours, water was added thereto with ice-cooling. The reaction mixture was extracted with a mixture of THF and ethyl acetate, washed with water and brine, dried over magnesium sulfate and then concentrated under reduced pressure. The residue was purified by subjecting to silica gel column chromatography (eluent; ethyl acetate:methanol = 10:1) and recrystallized from ethyl acetate-hexane to obtain the title compound (262 mg) as colorless crystals.
mp 104-106°C.
$^1$ H-NMR (CDCl3) δ: 1.5-1.8 (4H, m), 2.59 (2H, t, J = 7.0Hz), 2.7-2.8 (2H, m), 3.55-3.75 (2H, m), 3.79 (2H, t, J = 7.0Hz), 4.1-4.2 (1H, m), 5.01 (2H, s), 6.8-7.1 (5H, m), 6.92 (2H, d, J = 8.4Hz), 7.07 (2H, d, J = 8.4Hz), 7.5-7.6 (1H, m) , 7.59 (1H, d, J = 16.2Hz), 7.67 (1H, s).
IR (KBr): 1507, 1472, 1273, 1235, 1140, 1092, 966, 858 cm$^{-1}$. Anal. Calcd for $C_{28}H_{29}F_2N_3O_4$: C, 66.00; H, 5.74; N, 8.25.
Found: C, 65.69; H, 5.82; N, 8.06.
$[\alpha]^{22}$ D = + 4.2° (c = 1.0, methanol).

Reference Example B30

(2S)-3-[[1-[4-[4-[[2-[(E)-2-(2,4-Difluorophenyl)ethenyl]-1,3-oxazol-4-yl]methoxy]phenyl]butyl]-1H-imidazol-2-yl]-1,2-propanediol

Using (2S) -3-(1H-imidazol-2-yl)-1,2-propanediol, 60% oily sodium hydride (50 mg) and 4-[[4-(4-iodobutyl) phenoxy]methyl]-2-[(E)-2-(2,4-difluorophenyl)ethenyl]-1,3-oxazole (415 mg), the same reaction as in Reference Example B29 was conducted to obtain the title compound (219 mg) as colorless crystals. mp 106-108°C.
$^1$H-NMR ($CDCl_3$) δ: 1.5-1.8 (4H, m), 2.58 (2H, t, J = 6.8Hz), 2.7-2.8 (2H, m), 3.6-3.75 (2H, m), 3.82 (2H, t, J = 7.0Hz), 4.1-4.2 (1H, m), 5.01 (2H, s), 6.8-7.1 (5H, m), 6.89 (2H, d, J = 8.4Hz), 7.07 (2H, d, J = 8.4Hz), 7.5-7.6 (1H, m), 7.59 (1H, d, J = 16.4Hz), 7.67 (1H, s).
IR (KBr): 1615, 1512, 1497, 1273, 1246, 1229, 1140, 1094, 1046, 966, 847 cm$^{-1}$.
Anal. Calcd for $C_{28}H_{29}F_2N_3O_4$: C, 66.00; H, 5.74; N, 8.25.
Found: C, 65.75; H, 5.60; N, 8.12.
$[\alpha]^{22}$ D = - 3.5° (c = 1.0, methanol).

Reference Example C1

Inhibition of Phosphorylation of Receptor Tyrosine in Human Breast Cancer Cells

In each well of a 24-well plate, 500 μl of the cell suspension (500 cells) of human breast cancer cell line MCF-7 was seeded and incubated at 37°C in a 5% carbon dioxide gas incubator. On the next day, 250 μl of a 4-fold serial dilution of a solution of a test compound was added to each well and, 2 hours later, 250 μl of a heregulin solution, which was adjusted so as to be the final concentration of 0.8 μg/mL, was added thereto. After 5 minutes, an extracting solution was added thereto to terminate the reaction as well as to extract a protein. The protein was fractionated by protein electrophoresis and the protein in the electrophoretic gel was transferred to a nylon filter. The filter was reacted with a phosphorylated tyrosine-specific antibody. The reaction product was fluorescence-labeled and was exposed to a photo-film. The intensity of the exposure on the photo-film was quantitated by an image-analyzing apparatus. The proportion of an amount of phosphorylation of HER2 tyrosine of the cells to which the solution of the test compound at each concentration was added was determined by taking the amount of phosphorylation of HER2 tyrosine of the heregulin-added cells as 100% to calculate the concentration of the test compound requiring for inhibiting the phosphorylation of tyrosine by 50% of the phosphorylation of tyrosine of the control ($IC_{50}$ value).
The results are shown in Table 1.
As seen from the results, it has been shown that the test compound strongly inhibits the phosphorylation reaction of the tyrosine residue in the receptor protein which is caused by activation of the receptor tyrosine kinase accompanied with the stimulation of the growth factor heregulin upon stimulation of human breast cancer cells with the growth factor.

Table 1

| Reference Example No. (Compound No.) | Inhibition of phosphorylation of HER2 in cells MCF-7 ($IC_{50}$: μM) |
|---|---|
| B2 | 1.9 |

Table 1 (continued)

| Reference Example No. (Compound No.) | Inhibition of phosphorylation of HER2 in cells MCF-7 ($IC_{50}$: μM) |
|---|---|
| B3 | 0.18 |
| B4 | 0.10 |
| B6 | 1.2 |
| B11 | 1.1 |
| B20 | 1.5 |
| B22 | 1.9 |
| B26 | 0.92 |

Reference Example C2

In vitro Inhibition of Growth of Breast Cancer Cell BT-474

In each well of a 96-well microtiter plate, 100 μl (containing 1,000 cells) of a cell suspension of human breast cancer cell line BT-474 was seeded and incubated at 37°C in a 5% carbon dioxide gas incubator. On the next day, 100 μl of the 2-fold serial dilution of a solution of each test compound prepared by using 250 μl of a heregulin solution, which was adjusted so as to be the final concentration of 0.8 μg/mL, was added to each well and cultured for 5 days. The solution containing the test compound was removed and the cells were washed and fixed with a 50% trichloroacetic acid solution. To the cells was added a 0.4% (W/V) solution of pigment SRB (dissolved in 1% acetic acid) to fix as well as stain the cell protein (Skehan P. et al., Journal of the National Cancer Institute Vol. 82, pp. 1107-1112, 1990). The pigment solution was removed and the remainder was washed with a 1% acetic acid solution. Then, 100 ul of an extracting solution (10 mM Tris buffer) was added thereto to extract the pigment. The optical density of the extract at the wavelength of 550 nm was measured to determine the amount of the cells in terms of the amount of the protein. The proportion of the amount of remaining protein of each treated group was determined by taking the amount of protein of the control to which no solution of a test compound was added as 100% to calculate the value of the concentration of the compound required for inhibiting the remaining cells by 50% of that of the control ($IC_{50}$).

The results are shown in Table 2.

As seen from the results, it has been shown that the test compound of the present invention strongly inhibits the growth of human breast cancer cell line BT-474.

Table 2

| Reference Example No. (Compound No.) | Inhibition of cell growth BT-474 ($IC_{50}$: μM) |
|---|---|
| B2 | < 0.05 |
| B3 | < 0.05 |
| B4 | < 0.05 |
| B5 | < 0.05 |
| B6 | < 0.05 |
| B11 | < 0.05 |
| B19 | 0.017 |
| B20 | < 0.05 |
| B22 | < 0.05 |
| B26 | < 0.05 |

Reference Example C3

In vivo Breast cancer Inhibiting Effect

Human breast cancer cell line BT-474 (5,000,000 cells) were suspended in a gel-matrix solution. The suspension was subcutaneously implanted in the breast of 6-week-old female Balb/C strain nude mice (Friedman R. et al., Proceedings of the National Academy of Sciences of the U.S.A. Vol. 87, pp. 6698-6702, 1990). At the time of the implantation and seven days after the implantation, an estradiol dipropionate solution (5 mg/mL) was intramuscularly administered to the hind leg for the purpose of enhancing a take ratio of tumors. Fourteen days after the implantation,

the diameters of tumors were determined. For the experiment were used 5 mice per group with similar tumor size. Each of compounds (4, 6, 14, 17, 19, 20, 23, 24 and 26) of the present invention was suspended in a 5% gum arabic solution (physiological saline) and administered orally twice daily in dose of 30 mg/kg body weight for 10 days. On the day when the administration was terminated, the diameters of tumors were measured. Tumor volume was calculated by the following equation.

$$\text{Volume of tumor} = \text{longest diameter} \times \text{shortest diameter} \times \text{shortest diameter} \times 1/2$$

The growth rate of tumors was calculated as the proportion of the value obtained by subtracting the initial volume from the final volume in the group to which the drug was administered, to the value obtained by subtracting the initial volume from the final volume in the control group to which a gum arabic solution was administered.
The results are shown in Table 3.
The test compounds inhibited the growth of human breast cancer implanted to nude mice. The body weights of mice were measured during the experiment, while no decrease in body weights due to administration of the test compounds was observed.

Table 3

| Reference Example No. (Compound No.) | Inhibition ratio (%) |
|---|---|
| B4 | 5 |
| B6 | 28 |
| B23 | 27 |
| B24 | 28 |
| B26 | 15 |

Example 1a

A mixture of 1000 mg of the compound of Reference Example B4, 1000 mg of sodium deoxycholate (NaDC) and 1000 mg of hydroxypropyl cellulose (HPC-L) was comminuted (at room temperature for 2 hours) using a ball mill. After addition of 80 ml of distilled water, the comminuted mixture was subjected to an ultrasonic treatment (for 30 minutes, TITEC VP-60) with cooling by ice-water to obtain a uniform drug dispersion. The resulting dispersion was further treated eight times at 1500 bar using a high-pressure homogenizer Micronlab 40 (AVP Gaulin GmbH) to obtain a suspension of crystalline particulate of the compound of Reference Example B4.

Example 1b

A mixture of 1000 mg of the compound of Reference Example B6, 1000 mg of sodium deoxycholate (NaDC) and 1000 mg of hydroxypropyl cellulose (HPC-L) is comminuted (at room temperature for 2 hours) using a ball mill and, after adding 80 ml of distilled water, the comminuted mixture is subjected to an ultrasonic treatment (for 30 minutes, TITEC VP-60) with cooling by ice-water to obtain a uniform drug dispersion. The resulting dispersion is further treated eight times at 1500 bar using a high-pressure homogenizer Micronlab 40 (AVP Gaulin GmbH) to obtain a suspension of crystalline particulate of the compound of Reference Example B6.

Example 1c

A mixture of 1000 mg of the compound of Reference Example B26, 1000 mg of sodium deoxycholate (NaDC) and 1000 mg of hydroxypropyl cellulose (HPC-L) is comminuted (at room temperature for 2 hours) using a ball mill and, after adding 80 ml of distilled water, the comminuted mixture is subjected to an ultrasonic treatment (for 30 minutes, TITEC VP-60) with cooling by ice-water to obtain a uniform drug dispersion. The resulting dispersion is further treated eight times at 1500 bar using a high-pressure homogenizer Micronlab40 (AVP Gaulin GmbH) to obtain a suspension of crystalline particulate of the compound of Reference Example B26.

Example 2a

A mixture of 1000 mg of the compound of Reference Example B4, 1000 mg of sodium deoxycholate and 1000 mg of hydroxypropyl cellulose (HPC-L) was comminuted (at room temperature for 2 hours) using a ball mill and, after adding 40 ml of distilled water, the comminuted mixture was subjected to an ultrasonic treatment (for 30 minutes, TITEC VP-60) with cooling by ice-water to obtain a uniform drug dispersion. The resulting dispersion was further treated ten times at 1500 bar using a high-pressure homogenizer Micronlab 40 (AVP Gaulin- GmbH) to obtain a suspension of crystalline particulate of the compound of Reference Example B4.

Example 2b

A mixture of 1000 mg of the compound of Reference Example B6, 1000 mg of sodium deoxycholate and 1000 mg of hydroxypropyl cellulose (HPC-L) is comminuted (at room temperature for 2 hours) using a ball mill and, after adding 40 ml of distilled water, the comminuted mixture is subjected to an ultrasonic treatment (for 30 minutes, TITEC VP-60) with cooling by ice-water to obtain a uniform drug dispersion. The resulting dispersion is further treated ten times at 1500 bar using a high-pressure homogenizer Micronlab 40 (AVP Gaulin GmbH) to obtain a suspension of crystalline particulate of the compound of Reference Example B6.

Example 2c

A mixture of 1000 mg of the compound of Reference Example B26, 1000 mg of sodium deoxycholate and 1000 mg of hydroxypropyl cellulose (HPC-L) is comminuted (at room temperature for 2 hours) using a ball mill and, after adding 40 ml of distilled water, the comminuted mixture is subjected to an ultrasonic treatment (for 30 minutes, TITEC VP-60) with cooling by ice-water to obtain a uniform drug dispersion. The resulting dispersion is further treated ten times at 1500 bar using a high-pressure homogenizer Micronlab 40 (AVP Gaulin GmbH) to obtain a suspension of crystalline particulate of the compound of Reference Example B26.

Example 3a

A mixture of 37 mg of the compound of Reference Example B4, 37 mg of sodium deoxycholate and 37 mg of hydroxypropyl cellulose (HPC-L) was dispersed in 7 ml of distilled water and then uniformly dispersed by subjecting to a ultrasonic treatment (for 30 minutes, Branson 1200). Stainless steel balls (1/16 inch, 7 ml) as a grinding medium were added to the resulting solution, followed by horizontally shaking in a grind container at room temperature for 4 days to obtain a suspension of crystalline particulate of the compound of Reference Example B4.

Example 3b

A mixture of 37 mg of the compound of Reference Example B4, 37 mg of sodium deoxycholate and 37 mg of hydroxypropyl cellulose (HPC-L) is dispersed in 7 ml of distilled water and then uniformly dispersed by subjecting to a ultrasonic treatment (for 30 minutes, Branson 1200). Stainless steel balls (1/16 inch, 7 ml) as a grinding medium are added to the resulting solution, followed by horizontally shaking in a grind container at room temperature for 4 days to obtain a suspension of crystalline particulate of the compound of Reference Example B6.

Example 3c

A mixture of 37 mg of the compound of Reference Example B26, 37 mg of sodium deoxycholate and 37 mg of hydroxypropyl cellulose (HPC-L) is dispersed in 7 ml of distilled water and then uniformly dispersed by subjecting to a ultrasonic treatment (for 30 minutes, Branson 1200). Stainless steel balls (1/16 inch, 7 ml) as a grinding medium are added to the resulting solution, followed by horizontally shaking in a grind container at room temperature for 4 days to obtain a suspension of crystalline particulate of the compound of Reference Example B26.

Example 4a

A mixture of 1000 mg of the compound of Reference Example B4, 200 mg of sodium deoxycholate and 1000 mg of hydroxypropyl cellulose (HPC-L) was dispersed in 20 ml of distilled water. Stainless steel balls (1/16 inch, 7 ml) as a grinding medium were added to the resulting solution, followed by horizontally shaking in a grind container at room temperature for 4 days to obtain a suspension of crystalline particulate of the compound of Reference Example B4.

Example 4b

A mixture of 1000 mg of the compound of Reference Example B6, 200 mg of sodium deoxycholate and 1000 mg of hydroxypropyl cellulose (HPC-L) is dispersed in 20 ml of distilled water. Stainless steel balls (1/16 inch, 7 ml) as a grinding medium are added to the resulting solution, followed by horizontally shaking in a grind container at room temperature for 4 days to obtain a suspension of crystalline particulate of the compound of Reference Example B6.

Example 4c

A mixture of 1000 mg of the compound of Reference Example B26, 200 mg of sodium deoxycholate and 1000 mg of hydroxypropyl cellulose (HPC-L) is dispersed in 20 ml of distilled water. Stainless steel balls (1/16 inch, 7 ml) as a grinding medium are added to the resulting solution, followed by horizontally shaking in a grind container at room temperature for 4 days to obtain a suspension of crystalline particulate of the compound of Reference Example B26.

Example 5a

A mixture of 50 mg of the compound of Reference Example B4, 10 mg of sodium deoxycholate and 10 mg of polyvinyl pyrrolidone (90K) was dispersed in 10 ml of distilled water. Stainless steel balls (1/16 inch, 7 ml) as a grinding medium were added to the resulting solution, followed by horizontally shaking in a grind container at room temperature for 24 hours to obtain a suspension of crystalline particulate of the compound of Reference Example B4.

Example 5b

A mixture of 50 mg of the compound of Reference Example B6, 10 mg of sodium deoxycholate and 10 mg of polyvinyl pyrrolidone (90K) is dispersed in 10 ml of distilled water. Stainless steel balls (1/16 inch, 7 ml) as a grinding medium are added to the resulting solution, followed by horizontally shaking in a grind container at room temperature for 24 hours to obtain a suspension of crystalline particulate of the compound of Reference Example B6.

Example 5c

A mixture of 50 mg of the compound of Reference Example B26, 10 mg of sodium deoxycholate and 10 mg of polyvinyl pyrrolidone (90K) is dispersed in 10 ml of distilled water. Stainless steel balls (1/16 inch, 7 ml) as a grinding medium are added to the resulting solution, followed by horizontally shaking in a grind container at room temperature for 24 hours to obtain a suspension of crystalline particulate of the compound of Reference Example B26.

Example 6a

To a mixture of 1000 mg of the compound of Reference Example B4, 200 mg of sodium deoxycholate and 200 mg of polyvinyl pyrrolidone (90K), 20 ml of distilled water was added, and then the solution was subjected to an ultrasonic treatment (for 5 minutes, TITEC VP-60) to obtain a uniform dispersion. Stainless steel balls (1/16 inch, 20 ml) as a grinding medium were added to the resulting solution, followed by horizontally shaking in a grind container at room temperature for 24 hours, and further treating with a high-pressure homogenizer Micronlab 40 (AVP Gaulin GmbH) to obtain a suspension of crystalline particulate of the compound of Reference Example B26.

Example 6b

To a mixture of 1000 mg of the compound of Reference Example B6, 200 mg of sodium deoxycholate and 200 mg of polyvinyl pyrrolidone (90K), 20 ml of distilled water is added, and then the solution is subjected to an ultrasonic treatment (for 5 minutes, TITEC VP-60) to obtain a uniform dispersion. Stainless steel balls (1/16 inch, 20 ml) as grinding medium are added to the resulting solution, followed by horizontally shaking in a grind container at room temperature for 24 hours and further treating with a high-pressure homogenizer Micronlab 40 (AVP Gaulin GmbH) to obtain a suspension of crystalline particulate of the compound of Reference Example B6.

Example 6c

To a mixture of 1000 mg of the compound of Reference Example B26, 200 mg of sodium deoxycholate and 200 mg of polyvinyl pyrrolidone (90K), 20 ml of distilled water is added, and then the solution is subjected to an ultrasonic treatment (for 5 minutes, TITEC VP-60) to obtain a uniform dispersion. Stainless steel balls (1/16 inch, 20 ml) as a

grinding medium are added to the resulting solution, followed by horizontally shaking in a grind container at room temperature for 24 hours and further treating with a high-pressure homogenizer Micronlab 40 (AVP Gaulin GmbH) to obtain a suspension of crystalline particulate of the compound of Reference Example B26.

Example 7a

The suspension of crystalline particulate prepared in Example 2 was dried by a spray drier GS-31R (manufactured by Yamato Scientific Co. Ltd.) to obtain a powder. The resulting powder was further subjected to secondary drying by vacuum drying to obtain a powder of crystalline particulate.

Example 7b

The suspension of crystalline particulate prepared in Example 2b is dried by a spray drier GS-31R (manufactured by Yamato Scientific Co. Ltd.) to obtain a powder. The resulting powder is further subjected to secondary drying by vacuum drying to obtain a powder of crystalline particulate.

Example 7c

The suspension of crystalline particulate prepared in Example 2c is dried by a spray drier GS-31R (manufactured by Yamato Scientific Co. Ltd.) to obtain a powder. The resulting powder is further subjected to secondary drying by vacuum drying to obtain a powder of crystalline particulate.

Example 8a

The suspension of crystalline particulate prepared in Example 4a was dried by a spray drier GS-31R (manufactured by Yamato Scientific Co. Ltd.) to obtain a powder. The resulting powder was further subjected to secondary drying by vacuum drying to obtain a powder of crystalline particulate.

Example 8b

The suspension of crystalline particulate prepared in Example 4b is dried by a spray drier GS-31R (manufactured by Yamato Scientific Co. Ltd.) to obtain a powder. The resulting powder is further subjected to secondary drying by vacuum drying to obtain a powder of crystalline particulate.

Example 8c

The suspension of crystalline particulate prepared in Example 4c is dried by a spray drier GS-31R (manufactured by Yamato Scientific Co. Ltd.) to obtain a powder. The resulting powder is further subjected to secondary drying by vacuum drying to obtain a powder of crystalline particulate.

Example 9a

Four g of hydroxypropyl cellulose (grade: SL) and 40 g of the compound of Reference Example B4 were added to 160 mL of distilled water and mixed with a propeller stirrer. To the resulting mixture, 40 mL of a 4% sucrose stearate ester solution was further added, followed by stirring using a propeller stirrer to prepare a uniform suspension. The solution thus obtained was comminuted (revolution speed: 2000 rpm, treating time: 120 minutes) using a compaction shearing type mill Micros (Model MIC-0, manufactured by Nara Machinery Co., Ltd.) to obtain a suspension of crystalline particulate of the compound of Reference Example B4.

Example 9b

Four g of hydroxypropyl cellulose (grade: SL) and 40 g of the compound of Reference Example B6 are added to 160 mL of distilled water and mixed with a propeller stirrer. To the resulting mixture, 40 mL of a 4% sucrose stearate ester solution is further added, followed by stirring using a propeller stirrer to prepare a uniform suspension. The solution thus obtained is comminuted (revolution speed: 2000 rpm, treating time: 120 minutes) using a compaction shearing type mill Micros (Model MIC-0, manufactured by Nara Machinery Co., Ltd.) to obtain a suspension of crystalline particulate of the compound of Reference Example B6.

Example 9c

Four g of hydroxypropyl cellulose (grade: SL) and 40 g of the compound of Reference Example B26 are added to 160 mL of distilled water and mixed with a propeller stirrer. To the resulting mixture, 40 mL of a 4% sucrose stearate ester solution is further added, followed by stirring using a propeller stirrer to prepare a uniform suspension. The solution thus obtained is comminuted (revolution speed: 2000 rpm, treating time: 120 minutes) using a compaction shearing type mill Micros (Model MIC-0, manufactured by Nara Machinery Co., Ltd.) to obtain a suspension of crystalline particulate of the compound of Reference Example B26.

Example 10a

Four g of hydroxypropyl cellulose (grade: SL) and 40 g of the compound of Reference Example B4 were added to 160 mL of distilled water and mixed with a propeller stirrer. To the resulting mixture, 40 mL of a 4% sodium lauryl sulfate stearate ester solution was further added, followed by stirring using a propeller stirrer to prepare a uniform suspension. The solution thus obtained was comminuted (revolution speed: 2000 rpm, treating time: 90 minutes) using a compaction shearing type mill Micros (Model MIC-0, manufactured by Nara Machinery Co., Ltd.) to obtain a suspension of crystalline particulate of the compound of Reference Example B4.

Example 10b

Four g of hydroxypropyl cellulose (grade: SL) and 40 g of the compound of Reference Example B6 are added to 160 mL of distilled water and mixed with a propeller stirrer. To the resulting mixture, 40 mL of a 4% sodium lauryl sulfate stearate ester solution is further added, followed by stirring using a propeller stirrer to prepare a uniform suspension. The solution thus obtained is comminuted (revolution speed: 2000 rpm, treating time: 90 minutes) using a compaction shearing type mill Micros (Model MIC-0, manufactured by Nara Machinery Co., Ltd.) to obtain a suspension of crystalline particulate of the compound of Reference Example B6.

Example 10c

Four g of hydroxypropyl cellulose (grade: SL) and 40 g of the compound of Reference Example B26 are added to 160 mL of distilled water and mixed with a propeller stirrer. To the resulting mixture, 40 mL of a 4% sodium lauryl sulfate stearate ester solution is further added, followed by stirring using a propeller stirrer to prepare a uniform suspension. The solution thus obtained is comminuted (revolution speed: 2000 rpm, treating time: 90 minutes) using a compaction shearing type mill Micros (Model MIC-0, manufactured by Nara Machinery Co., Ltd.) to obtain a suspension of crystalline particulate of the compound of Reference Example B26.

Example 11a

To 164 g of an aqueous 2.5 (w/v) hydroxypropyl cellulose (grade: SSL) solution, 40 g of the compound of Reference Example B4 and 41.6 g of an aqueous 4% sodium lauryl sulfate solution were added, followed by stirring with a propeller stirrer to prepare a uniform suspension. The solution thus obtained was comminuted (revolution speed: 2000 rpm, treating time: 180 minutes) using a compaction shearing type mill Micros (Model MIC-0, manufactured by Nara Machinery Co., Ltd.) to obtain a suspension of crystalline particulate of the compound of Reference Example B4.

Example 11b

To 164 g of an aqueous 2.5 (w/v) hydroxypropyl cellulose (grade: SSL) solution, 40 g of the compound of Reference Example B6 and 41.6 g of an aqueous 4% sodium lauryl sulfate solution are added, followed by stirring with a propeller stirrer to prepare a uniform suspension. The solution thus obtained is comminuted (revolution speed: 2000 rpm, treating time: 180 minutes) using a compaction shearing type mill Micros (Model MIC-0, manufactured by Nara Machinery Co., Ltd.) to obtain a suspension of crystalline particulate of the compound of Reference Example B6.

Example 11c

To 164 g of an aqueous 2.5 (w/v) hydroxypropyl cellulose (grade: SSL) solution, 40 g of the compound of Reference Example B26 and 41.6 g of an aqueous 4% sodium lauryl sulfate solution are added, followed by stirring with a propeller stirrer to prepare a uniform suspension. The solution thus obtained is comminuted (revolution speed: 2000 rpm, treating time: 180 minutes) using a compaction shearing type mill Micros (Model MIC-0, manufactured by

Nara Machinery Co., Ltd.) to obtain a suspension of crystalline particulate of the compound of Reference Example B26.

Test Example 1

(1) Measurement of size of crystalline particulate

The size of fine particles in the suspension of the drug of crystalline particulate obtained in Example 1 was measured. In the measurement of the particle size, a laser diffraction/scattering particle size distribution measuring apparatus (SALD-2000A manufactured by Shimadzu Corporation) was used. Using distilled water as a dispersion medium, the measurement was conducted after sufficiently dispersing a sample having a concentration at which a scattering intensity requited for the measurement can be obtained. Further, the size of particles obtained by comminuting bulk of the compound of Reference Example B4, which was used in Example 1, with a jet mill was measured in the same manner. The measurement results are shown in Table 4.

Table 4

| Substances | Average particle size (μm) |
|---|---|
| Reference Example 4B | 3.85 |
| Example 1a | 0.88 |

As seen from the results shown in Table 4, the particle size of the powder obtained by comminuting bulk of the compound of Reference Example B4 with a jet mill was 4 μm or less, while the particle size of fine particles of the drug in the suspension of fine particles obtained in Example 1a was less than 1 μm.

(2) Drug absorption test

The suspension of the drug of crystalline particulate obtained in Example 1a was administered to IGS male rats and the plasma level of the drug was measured by a HPLC method. Further, a mixture containing the same components was administered to rats and the absorbability of the drug was compared. The test results are shown in Fig. 1.

As seen from Fig. 1, a remarkable improvement in absorption of the drug was recognized when the drug was administered in the form of crystalline particulate and AUC was 1.7 μg/hr/ml upon administration of the mixture, while AUC increased to 5.3 μg/hr/ml by three or more times when administered in the form of crystalline particulate.

Test Example 2

(1) Measurement of size of crystalline particulate

The size of fine particles in the suspension of the drug of crystalline particulate obtained in Example 4a was measured. In the measurement of the particle size, a laser diffraction/scattering particle size distribution measuring apparatus (SALD-2000A manufactured by Shimadzu Corporation) was used. Using distilled water as a dispersion medium, the measurement was conducted after fully dispersing a sample at a concentration sufficient for obtaining a scattering intensity required for the measurement. Further, crystalline particulate obtained in Example 8a were dispersed again in distilled water and the size of fine particles of the drug was measured in the same manner.

The measurement results are shown in Table 5.

Table 5

| Example No. | Average particle size (μm) |
|---|---|
| 4a | 0.92 |
| 8a | 0.99 |

As seen from the results shown in Table 5, crystalline particulate of the drug having an average particle size of 0.92 μm existed in the suspension of Example 4a. Further, when the powder obtained from this suspension by a spray drying method was dispersed again in distilled water (Example 8a), any large change in particle size of fine particles of the drug was not recognized and it was possible to disperse again as fine particles of the drug having an average particle size of 0.99 μm.

(2) Observation of crystalline state of crystalline particulate of drug

The crystalline state of the drug in fine particles of the drug obtained in Example 8a was measured by using a powder X-ray diffraction apparatus RINT-uitima/PC (RIGAKU). The resulting X-ray diffraction pattern was the same as that of the bulk drug, which confirmed that the drug in the form of a powder was not in an amorphous state, but in a crystalline state.

(3) Drug absorption test

The suspension of the drug of crystalline particulate obtained in Example 4a was administered to IGS male rats and the plasma level of the drug was measured by a HPLC method. Further, the composition of the powdered drug obtained in Example 8a was dispersed again in distilled water and the absorbability of the drug after administering to rats was examined.

The results are shown in Fig. 2.

As seen from Fig. 2, AUC was 4.43 μg/hr/ml up to 24 hours after administering the pharmaceutical composition of Example 4a and, in comparison with the administration of the bulk drug, a remarkable improvement in absorption of the drug was recognized. Also the change in the plasma level upon administration of the composition of the drug obtained in Example 8a is almost the same as the change in the plasma level upon administration of the composition of the drug obtain in Example 4a and lowering of the absorption due to conversion into dry powder was not recognized.

INDUSTRIAL APPLICABILITY

The HER2 inhibitor having an average particle size of about 3 μm or less or the composition thereof of the present invention has remarkably improved absorbability of the HER2 inhibitor.

## Claims

1. A HER2 inhibitor having an average particle size of about 3 μm or less.

2. A HER2 inhibitor which has an average particle size of about 3 μm or less when dispersed in water or an aqueous solution.

3. The HER2 inhibitor according to claim 1 or 2, which is in the form of a crystalline particulate.

4. The HER2 inhibitor according to claim 1 or 2, which is a compound represented by the formula:

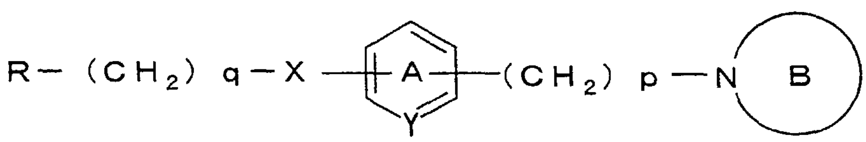

wherein R represents an optionally substituted aromatic heterocyclic group, X represents an oxygen atom, an optionally oxidized sulfur atom, -C(=O)- or -CH(OH)-, Y represents CH or N, p represents an integer of 0 to 10, q represents an integer of 1 to 5, a group represented by the formula:

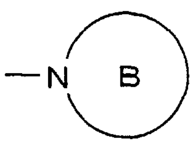

represents an optionally substituted aromatic azole group, and ring A may be further substituted, or a salt thereof or a prodrug thereof.

5. The HER2 inhibitor according to claim 1 or 2, which is a compound represented by the formula:

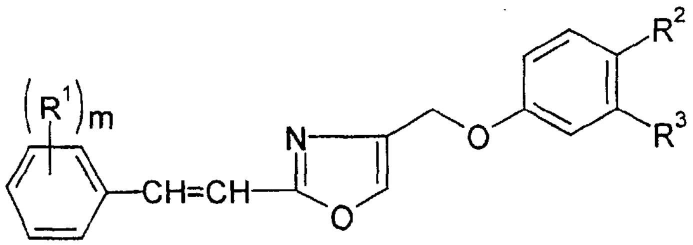

wherein m represents 1 or 2, $R^1$ represents halogen or an optionally halogenated $C_{1-2}$ alkyl, and one of $R^2$ and $R^3$ represents a hydrogen atom and the other represents a group represented by the formula:

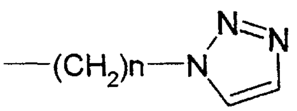

or

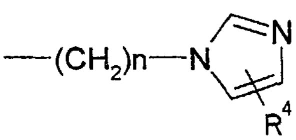

wherein n represents 3 or 4, and $R^4$ represents a $C_{1-4}$ alkyl group substituted with 1 to 2 hydroxy groups, or a salt thereof or a prodrug thereof.

6. The HER2 inhibitor according to claim 1 or 2, which is (i) 1-(4-{4-[(2-{(E)-2-[4-(trifluoromethyl)phenyl]ethenyl}-1,3-oxazol-4-yl)methoxy]phenyl}butyl)-1H-1,2,3-triazole, (ii) 1-(3-{3-[(2-{(E)-2-[4-(trifluoromethyl)phenyl]ethenyl}-1,3-oxazol-4-yl)methoxy]phenyl}propyl)-1H-1,2,3-triazole or (iii) 3-(1-{4-[4-({2-[(E)-2-(2,4-difluorophenyl) ethenyl]-1,3-oxazol-4-yl}methoxy)phenyl]butyl}-1H-imidazol-2-yl)-1,2-propanediol, or a salt thereof or a prodrug thereof.

7. A composition comprising the HER2 inhibitor of claim 1 or 2.

8. The composition according to claim 7, which comprises a stabilizer.

9. The composition according to claim 7, wherein the stabilizer is at least one member selected from (1) a surfactant, (2) a hydrophilic polymer and (3) an easily water-soluble cyclodextrin derivative.

10. The composition according to claim 7, wherein the stabilizer is at least one member selected from sodium deoxycholate, hydroxypropylcellulose and polyvinyl pyrrolidone.

11. The composition according to claim 8, which comprises both a surfactant and a hydrophilic polymer as the stabilizer.

12. The composition according to claim 11, wherein the surfactant is an anionic surfactant or a nonionic surfactant.

13. The composition according to claim 11, wherein the surfactant is an alkyl sulfate salt or a sucrose fatty acid ester.

14. The composition according to claim 11, wherein the surfactant is sodium lauryl sulfate or sucrose stearate ester.

15. The composition according to claim 11, wherein the hydrophilic polymer is hydroxypropylcellulose.

16. The composition according to claim 11, wherein the surfactant is sodium lauryl sulfate or sucrose fatty acid ester, and the hydrophilic polymer is hydroxypropylcellulose.

17. The composition according to claim 7, which is used for oral administration.

18. The composition according to claim 7, which is an anticancer agent.

19. The composition according to claim 7, which is an agent for preventing or treating breast cancer or prostatic cancer.

**20.** A process for producing the HER2 inhibitor according to claim 1 or 2, which comprises comminuting the HER2 inhibitor in water or an aqueous solution.

**21.** A process for producing the composition according to claim 7, which comprises comminuting a HER2 inhibitor in water or an aqueous solution.

**22.** A process for producing the composition according to claim 8, which comprises comminuting a HER2 inhibitor in an aqueous solution containing a stabilizer.

**23.** The process according to claim 20, 21 or 22, wherein the comminution is carried out by using a compaction shearing mill.

**24.** A composition obtainable by comminuting a HER2 inhibitor in an aqueous solution containing a stabilizer and removing a solvent.

**25.** A method for preventing or treating cancer, which comprises orally administering a HER2 inhibitor having an average particle size of about 3 μm or less to a mammal.

**26.** Use of a HER2 inhibitor having an average particle size of about 3 μm or less for the manufacture of an agent for oral administration for preventing or treating cancer.

Fig. 1

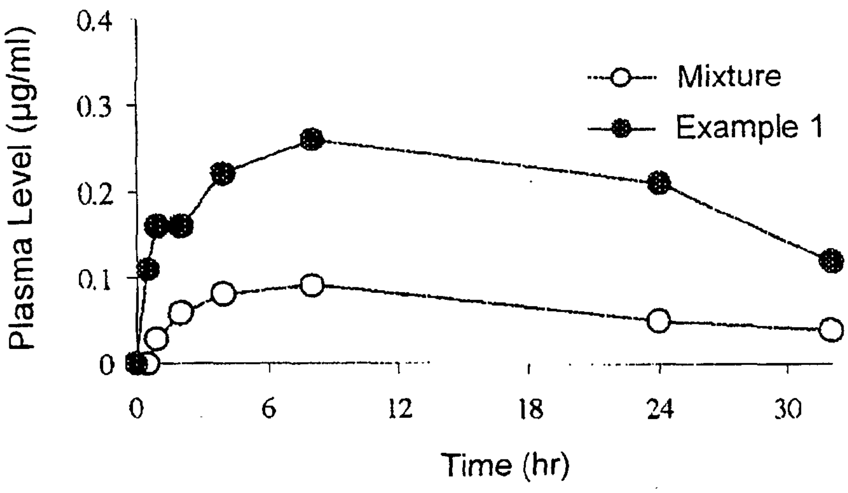
0.4
0.3
0.2
0.1
0
Plasma Level (µg/ml)
Mixture
Example 1
0
6
12
18
24
30
Time (hr)

Fig. 2

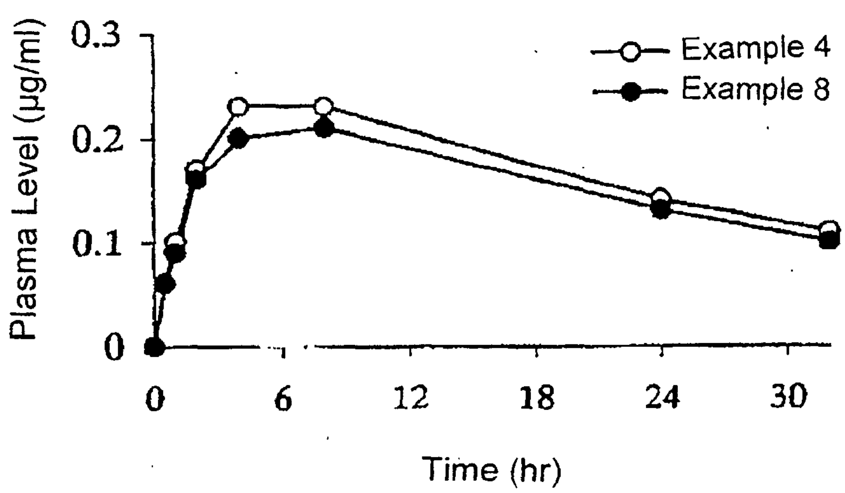
0.3
0.2
0.1
0
Plasma Level (µg/ml)
Example 4
Example 8
0
6
12
18
24
30
Time (hr)

## INTERNATIONAL SEARCH REPORT

International application No.
PCT/JP01/10827

A. CLASSIFICATION OF SUBJECT MATTER
Int.Cl[7] C07D413/12, A61K31/422, 9/10, 9/14, 47/20, 47/26, 47/28, 47/32, 47/38, 47/40, A61P35/00

According to International Patent Classification (IPC) or to both national classification and IPC

B. FIELDS SEARCHED

Minimum documentation searched (classification system followed by classification symbols)
Int.Cl[7] C07D413/12, A61K31/422, 9/10, 9/14, 47/20, 47/26, 47/28, 47/32, 47/38, 47/40, A61P35/00

Documentation searched other than minimum documentation to the extent that such documents are included in the fields searched

Electronic data base consulted during the international search (name of data base and, where practicable, search terms used)
CAPLUS, REGISTRY (STN))

C. DOCUMENTS CONSIDERED TO BE RELEVANT

| Category* | Citation of document, with indication, where appropriate, of the relevant passages | Relevant to claim No. |
|---|---|---|
| X | Cancer Chemother. Pharmacol., (2000), 46 Suppl. S77-82 | 1,2 |
| X | Romanian Journal of Biophysics, (2000), 10(1-2), pages 33 to 47 | 1,2 |
| X | WO 98/3505 A2 (Takeda Chemical Industries, Ltd.), 29 January, 1998 (29.01.1998), & AU 9734616 A & EP 912562 A1 & CN 1223653 A & ZA 9706378 A & JP 11-60571 A & US 6211215 A | 1-24,26 |
| Y | WO 96/19239 A1 (Yamanouchi Pharm. Co., Ltd.), 27 June, 1996 (27.06.1996), & AU 9643141 A | 1-24,26 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 February, 2002 (05.02.02) | 19 February, 2002 (19.02.02) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/10827

**Box I Observations where certain claims were found unsearchable (Continuation of item 1 of first sheet)**

This international search report has not been established in respect of certain claims under Article 17(2)(a) for the following reasons:

1. ☒ Claims Nos.: 25
because they relate to subject matter not required to be searched by this Authority, namely:

The invention as set forth in claim 25 pertains to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The inventions as set forth in claims 1 to 23 and 26 relate to an HER2 inhibitor having an average particle size of about 3 μm or less, while the invention as set forth in claim 24 involves no specification concerning the average particle size. Thus, there is no technical relevancy involving any special technical feature between these groups of inventions. Such being the case, the present application involves two groups of inventions, i.e., the group of the inventions as set forth in claims 1 to 23 and 26 and the group of the invention as set forth in claim 24 and, therefore, fails to satisfy the requirement of unity of invention.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP01/10827

The invention as set forth in claim 1 pertains to an HER2 inhibitor. Although its chemical structure is not specified, the specific substance as set forth in claim 5 is exclusively disclosed in the detailed description of the invention. Namely, no other chemical is substantially cited therein. Therefore, this international search has been practiced mainly on the compound as set forth in claim 5.

Form PCT/ISA/210 (extra sheet) (July 1992)